# EUROPEAN PATENT SPECIFICATION

(11) **EP 4 484 439 B1**
(45) Date of publication and mention of the grant of the patent: **17.09.2025**
(21) Application number: 24736298.1
(22) Date of filing: 26.01.2024
(51) Int. Cl.: C07H 19/067, C07H 19/167, C07H 21/04, C07H 1/00

(54) **6'-CYANO-MODIFIED LOCKED NUCLEOSIDE, NUCLEOTIDE, AND NUCLEIC ACID POLYMER**
6'-CYANO-MODIFIZIERTE BLOCKIERTE NUKLEOSIDE, NUKLEOTIDE UND NEEPESINSÄUREPOLYMERE
NUCLÉOSIDE, NUCLÉOTIDE ET POLYMÈRE D'ACIDE NUCLÉIQUE VERROUILLÉS À MODIFICATION 6'-CYANO

(30) Priority: 18.05.2023 CN 202310565102
(43) Date of publication of application: 01.01.2025
(73) Proprietor: Academy of Military Medical Sciences, Beijing 100850 (CN)
(72) Inventor: HE, Xiaoyang, Beijing 100850 (CN); WANG, Shengqi, Beijing 100850 (CN); DENG, Xinxiu, Beijing 100850 (CN); SHI, Anzhe, Beijing 100850 (CN)
(74) Representative: Eisenführ Speiser
(86) International application number: PCT/CN2024/074208
(87) International publication number: WO 2024/234728

(56) References cited:
- CN-A- 106 459 135
- CN-A- 110 204 583
- CN-A- 110 590 886
- CN-A- 116 606 338
- CN-A- 116 789 725
- LIU TIANTIAN, WANG AIPING, HONGTAO JIN: "Development of Chiral Drugs and their Regulatory Guidelines", YAOWU PINGJIA YANJIU [=DRUG EVALUATION RESEARCH], YAOWU PINGJIA YANJIU ZAZHI BIANJIBU, CN, vol. 41, no. 12, 8 December 2018 (2018-12-08), CN , pages 2362 - 2368, XP093234507, ISSN: 1674-6376, DOI: 10.7501/j.issn.1674-6376.2018.12.045

## Description

The present application claims priority to the Chinese Patent Application No. CN202310565102.7, filed with the China National Intellectual Property Administration (CNIPA) on May 18, 2023, and entitled "6'-CYANO -MODIFIED LOCKED NUCLEOSIDES, NUCLEOTIDES, AND NUCLEIC ACID POLYMERS'.

### TECHNICAL FIELD

The present disclosure relates to the field of biotechnology, and in particular to 6'-cyano (CN)-modified locked nucleosides, nucleotides, and nucleic acid polymers (NAPs).

### BACKGROUND

Nucleic acid drugs are at the forefront of the development of biomedicine and represent the third major type of drug following small molecule drugs and protein drugs. Nucleic acid drugs mainly include antisense oligonucleotides (ASOs) and small interfering RNAs (siRNAs). Compared with traditional small molecule and protein drugs, they have the advantages of rapid design, universal targets, high specificity, the ability to act inside cells, and relatively fast synthesis. They hold significant value in the treatment of chronic, intractable, and major diseases, as well as rare diseases, where it is difficult to develop drugs targeting protein targets. As of 2022, there have been 15 nucleic acid drugs approved worldwide (10 ASOs and 5 siRNAs), with more than 400 nucleic acid drugs in clinical trials.

However, on one hand, unmodified oligonucleotide drugs typically have unsatisfactory druggability. They exhibit poor drug-like properties, such as poor stability and susceptibility to degradation by nucleases; high polarity, making it difficult to enter cells and leading to poor distribution characteristics; and poor binding affinity to target mRNA. To achieve clinical utility, oligonucleotides must undergo chemical modification, and all marketed nucleic acid drugs have employed corresponding chemical modifications of nucleic acids. On the other hand, chemically modified antisense nucleic acid drugs can cause toxicity to highly exposed organs (liver and kidneys) to a certain extent. Especially, chemically modified nucleic acid drugs with high affinity on the phosphorothioate (PS) backbone, such as locked nucleic acids (LNAs) and constrained ethyl locked nucleic acids (cEt-LNAs), may lead to hepatotoxicity or nephrotoxicity, including significant increases in liver ALT and AST, or necrosis, degeneration/regeneration, and other injuries to the renal tubules. Therefore, the development of efficient and safe new nucleic acid chemical modification technologies remains a key and bottleneck technology in the advancement of nucleic acid drugs.

In recent years, ASO drugs have made significant progress in the mechanism of toxicity (Nucleic Acids Res. 2016, 44, 3892; Nature Biotech., 2017, 35, 230; Nucleic Acids Res. 2018, 46, 2204*;* Nature Biotech., 2019, 37, 640; Nucleic Acids Res. 2019, 47, 10865; J. Am. Chem. Soc., 2020, 142, 14754; J. Am. Chem. Soc. 2020, 142, 7456). Studies have shown that the main toxicity mechanism of the ASO drugs lies in the fact that chemically modified phosphorothioate-antisense oligonucleotide drugs (PS-ASOs) may bind to and interfere with the subcellular distribution of intracellular proteins (such as P54nrb), thereby inducing cell apoptosis and producing toxicity. Meanwhile, toxicity is positively correlated with the binding capacity of ASO-protein. The stronger the binding capacity, the greater the potential toxicity. Moreover, this binding capacity is closely related to the hydrophilicity of the chemical modification structure (LogS). The stronger the hydrophobic effect, the greater the binding affinity. Experimental results show that the affinity of 2'-methoxyethyl (2'-MOE) for intracellular proteins is 30 times lower than that of 2'-fluoro (2'-F). Accordingly, the interaction between ASO and protein has become a determinant factor for the efficacy of ASO drugs, that is, structure determines the properties. In order to develop efficient and safe novel types of nucleic acid chemical modification structures, the inventor previously disclosed a novel cyano (CN)-modified locked nucleic acid (CN-LNA) structure (ZL 20190914759.3), which shows a high nuclease resistance as well as desirable target gene affinity and selectivity. Moreover, through further chemical property calculations (calculated by Chemdraw), it is shown that the CN-LNA exhibits desirable hydrophilicity with a LogS value of -0.032, which is about 2 times, 4 times, and 20 times that of commonly used 2'-methoxyethyl (MOE, with a LogS value of -0.073), locked nucleic acid (LNA, with a LogS value of -0.122), and 2'-fluoro (with a LogS value of -0.661), respectively. The CN-LNA has the potential to reduce the binding affinity of PS-ASO to intracellular proteins, thereby reducing the impact on the distribution of intracellular protein, reducing toxicity, and improving therapeutic effects. CN110590886A (ACADEMY OF MILITARY MEDICAL SCIENCES, December 20, 2019) discloses modified nucleosides, nucleotides, nucleic acid polymers, and their preparation method and use. CN 106459135 A (MIRAGEN THERAPEUTICS, INC., February 22, 2017) discloses methods for synthesizing modified nucleosides, nucleotides, and oligonucleotides comprising at least one 2'-C-Bridged Bicyclic Nucleotide, and to intermediates used in the process. CN 110204583 A (ACADEMY OF MILITARY MEDICAL SCIENCES, September 6, 2019) discloses modified nucleosides, and by modifying the nucleoside, azido group, amino group or amine group is introduced at the 4' position, obtaining a novel modified nucleoside structure. Tiantian et al. (Development of Chirall Drugs and their Regulatory Guidelines, Drug Evaluation Research, December 8, 2018) investigated the development of chiral drugs and outline the pharmacological and toxic characteristics of them; and discussed different statements by drug regulatory authorities in the development and approval of stereoisomeric drugs along with the current status for the safety evaluation of stereoisomeric drugs in China.

However, the previously disclosed CN-LNA synthesis method cannot stereoselectively synthesize the R and S configurations of the C6'-epimers, and it has not further explained the differences in the druggability properties of C6'-epimers for modifying nucleic acids, which cannot meet the needs of establishing a new generation of highly efficient and low-toxicity nucleic acid chemical modification technology.

In view of this, the present disclosure is specifically proposed.

The invention is defined in the appended claims. Any disclosure going beyond the scope of said claims is only intended for illustrative purposes and should not be construed as being part of the invention.

### SUMMARY

A first objective of the present disclosure is to provide 6'-CN-modified locked nucleosides in R configuration or S configuration, nucleotides, and NAPs thereof.

A second objective of the present disclosure is to provide preparation methods of the 6'-CN-modified locked nucleosides in R configuration or S configuration, the nucleotides, and the NAPs thereof. With simple preparation method and high yield, at least one of the above problems can be solved.

A third objective of the present disclosure is to provide use of the NAPs in the preparation of a nucleic acid diagnostic agent or a nucleic acid therapeutic agent.

To achieve the above objective, the following technical solutions are adopted:

In a first aspect, the present disclosure provides a 6'-CN-modified locked nucleoside, where the 6'-CN-modified locked nucleoside is selected from the group consisting of a compound with a structure shown in Formula 1, salts and isomers thereof: 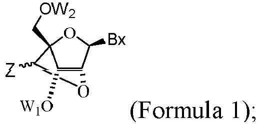
in Formula 1, Bx is selected from the group consisting of substituted or unsubstituted adenine, guanine, thymine, cytosine, and uracil, and salts thereof;
Z is CN;
W₁ and W₂ are independently selected from the group consisting of H or a hydroxyl protecting group; the hydroxyl protecting group is selected from but not limited to the group consisting of acetyl, tert-butyl, tert-butoxymethyl, methoxymethyl, tetrahydropyranyl, 1-ethoxyethyl, 1-(2-chloroethoxy)ethyl, *p*-chlorophenyl, 2,4-dinitrophenyl, benzyl, 2,6-dichlorobenzyl, diphenylmethyl, *p*-nitrobenzyl, bis(2-acetoxyethoxy)methyl, 2-trimethylsilylethyl, trimethylsilyl, triethylsilyl, tert-butyldimethylsilyl, tert-butyldiphenylsilyl, triphenylsilyl, [(triisopropylsilyl)oxy]methyl, benzoylformate, chloroacetyl, trichloroacetyl, trifluoroacetyl, pivaloyl, benzoyl, *p*-phenylbenzoyl, 9-fluorenylmethyl carbonate, methanesulfonate, p-toluenesulfonate, trityl, monomethoxytrityl, dimethoxytrityl, trimethoxytrityl, 1-(2-fluorophenyl)-4-methoxypiperidin-4-yl, 9-phenylxanthin-9-yl, 9-(*p*-methoxyphenyl)xanthin-9-yl, and 2-naphthylmethyl; and
the isomer is selected from the group consisting of a 6'-CN-modified locked nucleoside with Z in R configuration shown in 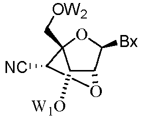 and a 6'-CN-modified locked nucleoside with Z in S configuration shown in

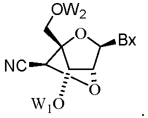

In a second aspect, the present disclosure provides nucleotides, including a 3'-active phosphorus group derivative of the 6'-CN-modified locked nucleosides; where
the active phosphorus group is selected from the group consisting of phosphoramidite, phosphoramidite derivative, H-phosphate, H-phosphate derivative, phosphate triester, and phosphate triester derivative.

Further, the nucleotide is selected from the group consisting of a compound with a structure shown in Formula 2, salts and isomers thereof: 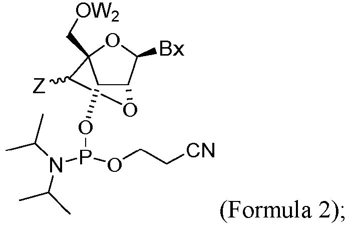
in Formula 2, Bx, W₂, and Z are consistent with those in Formula 1; and
the isomer is selected from the group consisting of a nucleoside with Z in R configuration shown in 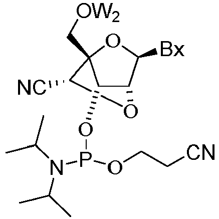 and a nucleoside with Z in S configuration shown in

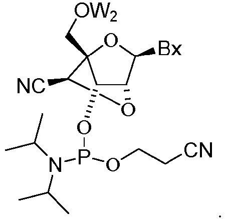

In a third aspect, the present disclosure provides use of the nucleotides in reducing an interaction between a NAP and an intracellular protein or in the preparation of a drug for reducing the interaction between the NAP and the intracellular protein.

In a fourth aspect, the present disclosure provides NAPs, including a monomer with a structure shown in Formula 3: 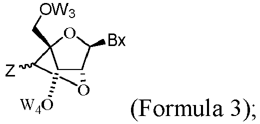 where
in Formula 3, Bx is selected from the group consisting of substituted or unsubstituted adenine, guanine, thymine, cytosine, and uracil, and salts thereof;
Z is CN;
W₃ and W₄ are independently selected from the group consisting of H, hydroxyl protecting group, and an internucleoside linking group linking the monomer to other portions of the NAP; and at least one of W₃ and W₄ is the internucleoside linking group linking the monomer to the other portions of the NAP; and
the hydroxyl protecting group is selected from the group consisting of acetyl, tert-butyl, tert-butoxymethyl, methoxymethyl, tetrahydropyranyl, 1-ethoxyethyl, 1-(2-chloroethoxy)ethyl, *p*-chlorophenyl, 2,4-dinitrophenyl, benzyl, 2,6-dichlorobenzyl, diphenylmethyl, *p*-nitrobenzyl, bis(2-acetoxyethoxy)methyl, 2-trimethylsilylethyl, trimethylsilyl, triethylsilyl, tert-butyldimethylsilyl, tert-butyldiphenylsilyl, triphenylsilyl, [(triisopropylsilyl)oxy]methyl, benzoylformate, chloroacetyl, trichloroacetyl, trifluoroacetyl, pivaloyl, benzoyl, *p*-phenylbenzoyl, 9-fluorenylmethyl carbonate, methanesulfonate, *p*-toluenesulfonate, trityl, monomethoxytrityl, dimethoxytrityl, trimethoxytrityl, 1-(2-fluorophenyl)-4-methoxypiperidin-4-yl, 9-phenylxanthin-9-yl, 9-(*p*-methoxyphenyl)xanthin-9-yl, and 2-naphthylmethyl.

Further, the monomer is selected from the group consisting of a monomer with Z in R configuration shown in 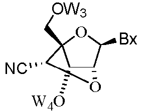 and a monomer with Z in S configuration shown in

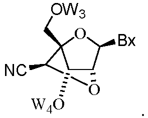

Further, the NAP is selected from the group consisting of a ribonucleic acid (RNA), a deoxyribonucleic acid (DNA), or a copolymer of the RNA and the DNA.

In a fifth aspect, the present disclosure provides use of the NAPs in preparation of a nucleic acid diagnostic agent or a nucleic acid therapeutic agent.

In a sixth aspect, the present disclosure provides a synthesis method of a C6'-CN-modified nucleoside in R configuration or S configuration, where the C6'-CN-modified nucleoside in R configuration is 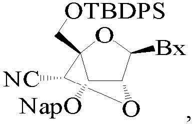 and a synthesis process thereof includes: isomerizing a terminal olefin of 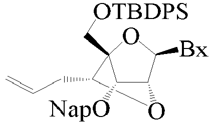 to obtain 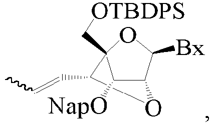 conducting dihydroxylation to obtain 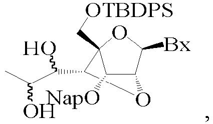 conducting oxidative cleavage to obtain an aldehyde compound 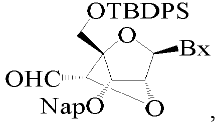 and then converting an aldehyde group of 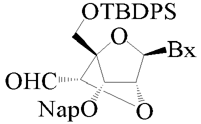 into a CN group to obtain the C6'-CN-modified nucleoside in R configuration shown in. 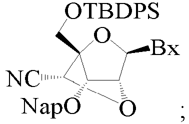
the C6'-CN-modified nucleoside in S configuration is 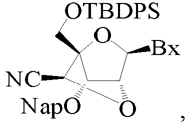 and a synthesis process thereof includes: isomerizing a terminal olefin of 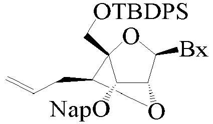 to obtain 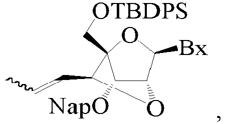 conducting dihydroxylation to obtain 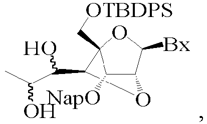 conducting oxidative cleavage to obtain an aldehyde compound 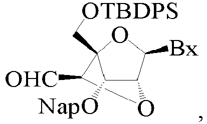 and then converting an aldehyde group of 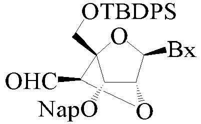 into a CN group to obtain the C6'-CN-modified nucleoside in S configuration shown in 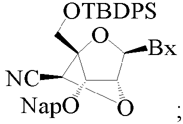 and
Bx is selected from the group consisting of substituted or unsubstituted adenine, guanine, thymine, cytosine, and uracil, and salts thereof.

Further, a synthesis method of 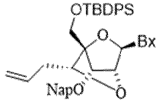 includes: methylsulfonating 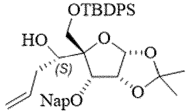 in S configuration, removing methylene of the methylsulfonated product, and acetylating to obtain 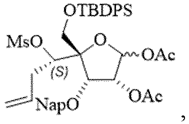 and then conducting glycosylation and nucleophilic substitution in sequence to obtain 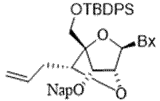 in R configuration;
a synthesis process of the 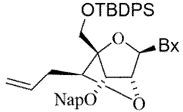 includes: methylsulfonating 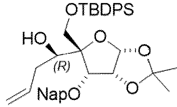 in R configuration, removing methylene of the methylsulfonated product, and acetylatiing to obtain 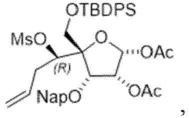 and then conducting glycosylation and nucleophilic substitution in sequence to obtain the 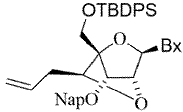 in S configuration; and
the glycosylation includes: subjecting 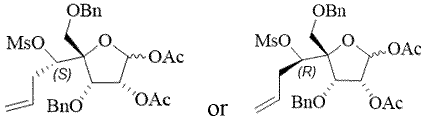 to a reaction with thymine, N6-benzoyladenine, or 6-chloroguanine at 50°C to 100°C in the presence of an activator; the activator includes *N*,*O*-bis(trimethylsilyl)acetamide (BSA) and trimethylsilyl trifluoromethanesulfonate (TMSOTf), and a reaction medium is selected from the group consisting of acetonitrile, 1,2-dichloroethane, and toluene.

Further, a synthesis process of the 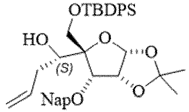 in S configuration includes: oxidizing a primary alcohol group of 5-*O*-(tert-butyldiphenylsilyl)-4-*C*-hydroxymethyl-1,2-*O*-isopropylidene-3-*O*-(2-naphthylmethyl)-α-*D*-ribofuranose 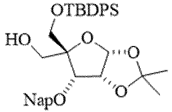 into an aldehyde group, and then allylating the aldehyde group to obtain 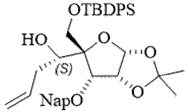 in S configuration;
in some embodiments, a synthesis process of 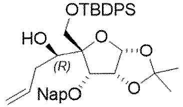 in R configuration includes: oxidizing a secondary alcohol group of 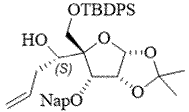 into a ketone group, and then reducing the ketone group into a secondary alcohol group to obtain 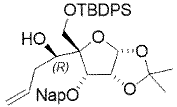 in R configuration; and
in the process of reducing the ketone group into the secondary alcohol group, a reducing agent used is at least one selected from the group consisting of lithium aluminum hydride, lithium borohydride, lithium chloride, and sodium borohydride; a reaction medium is at least one selected from the group consisting of dichloromethane (DCM), tetrahydrofuran (THF), methanol, and ethanol, and the reducing is conducted at -78°C to 0°C.

Further, a process of reducing the ketone group into the secondary alcohol group includes: conducting the reducing at -40°C to 0°C using a mixture of sodium borohydride and lithium chloride as the reducing agent and using a mixture of the THF and the methanol as the solvent.

Further, the catalyst for the isomerization of the terminal olefin is selected from the group consisting of a ruthenium catalyst, a palladium catalyst, a rhodium catalyst, and an iridium catalyst, in some embodiments, the catalyst is carbonylchlorohydridotris(triphenylphosphine)ruthenium(II);
a reaction medium for the isomerization of the terminal olefin is selected from the group consisting of methanol, ethanol, n-butanol, and toluene, in some embodiments, the reaction medium is ethanol;
the isomerization of the terminal olefin is conducted at 60°C to 100°C, preferably 60°C to 80°C; and
the isomerization of the terminal olefin is conducted for 12 h to 72 h.

In a seventh aspect, the present disclosure provides a synthesis method of a C6'-CN-modified LNA T phosphoramidite monomer in R configuration or S configuration, where a synthesis process of the C6'-CN-modified LNA T phosphoramidite monomer in R configuration includes: subjecting 3'-hydroxyl and 5'-hydroxyl groups in 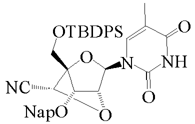 in R configuration to deprotection to obtain 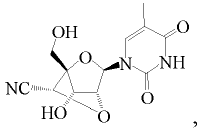 and then subjecting the 5'-hydroxyl group to 4,4'-dimethoxytrityl (DMTr) protection while subjecting the 3'-hydroxyl group to phosphoramidation to obtain 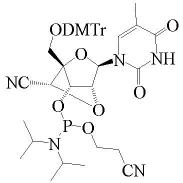 in R configuration; and
a synthesis process of the C6'-CN-modified LNA T phosphoramidite monomer in S configuration includes: subjecting 3'-hydroxyl and 5'-hydroxyl groups in 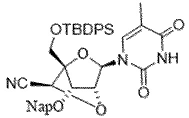 in S configuration to deprotection to obtain 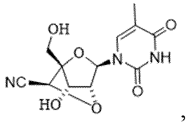 and then subjecting the 5'-hydroxyl group to DMTr protection while subjecting the 3'-hydroxyl group to phosphoramidation to obtain 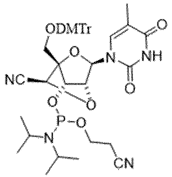 in S configuration.

In an eighth aspect, the present disclosure provides a synthesis method of a C6'-CN-modified LNA A phosphoramidite monomer in R configuration or S configuration, where a synthesis process of the C6'-CN-modified LNA A phosphoramidite monomer in R configuration includes: subjecting a base in 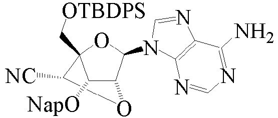 in R configuration to protection to obtain 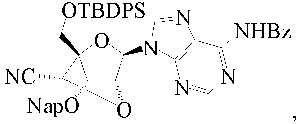 subjecting 3'-hydroxyl and 5'-hydroxyl groups to deprotection to obtain 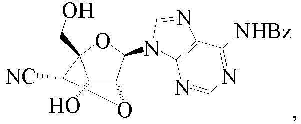 and then subjecting the 5'-hydroxyl group to DMTr protection while subjecting the 3'-hydroxyl group to phosphoramidation to obtain 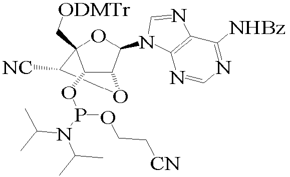 in R configuration; and
a synthesis process of the C6'-CN-modified LNA A phosphoramidite monomer in S configuration includes: subjecting a base in 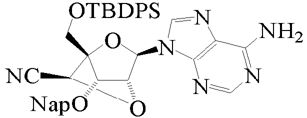 in S configuration to protection to obtain 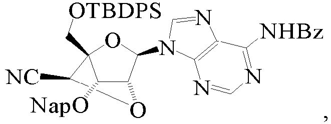 subjecting 3'-hydroxyl and 5'-hydroxyl groups to deprotection to obtain 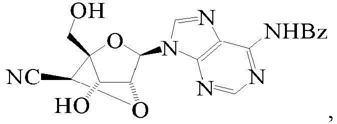 and then subjecting the 5'-hydroxyl group to DMTr protection while subjecting the 3'-hydroxyl group to phosphoramidation to obtain 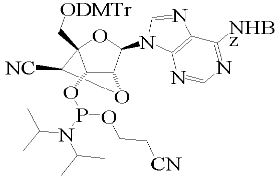 in S configuration.

In a ninth aspect, the present disclosure provides a synthesis method of a C6'-CN-modified LNA G phosphoramidite monomer in R configuration or S configuration, where the synthesis process of the C6'-CN-modified LNA G phosphoramidite monomer in R configuration includes: demethylating a methoxy group in 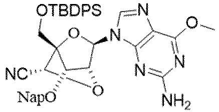 in R configuration to obtain 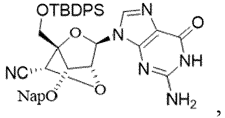 conducting base protection to obtain 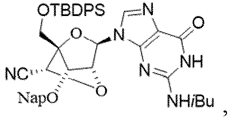 subjecting 3'-hydroxyl and 5'-hydroxyl groups to deprotection to obtain 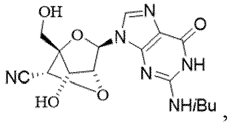 and then subjecting the 5'-hydroxyl group to DMTr protection while subjecting the 3'-hydroxyl group to phosphoramidation to obtain 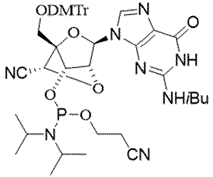 in R configuration; and
a synthesis method of the C6'-CN-modified LNA G phosphoramidite monomer in S configuration includes: demethylating a methoxy group in 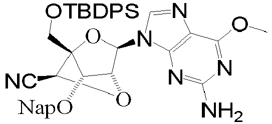 in S configuration to obtain 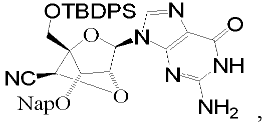 conducting base protection to obtain 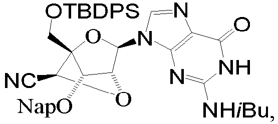 subjecting 3'-hydroxyl and 5'-hydroxyl groups to deprotection to obtain 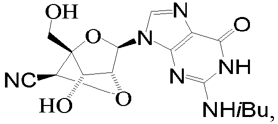 and then subjecting the 5'-hydroxyl group to DMTr protection while subjecting the 3'-hydroxyl group to phosphoramidation to obtain 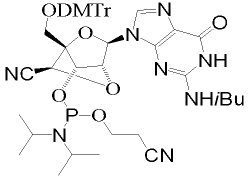 in S configuration.

In a tenth aspect, the present disclosure provides a synthesis method of a C6'-CN-modified LNA C phosphoramidite monomer in R configuration or S configuration, where a synthesis process of the C6'-CN-modified LNA C phosphoramidite monomer in R configuration includes: subjecting 3'-hydroxyl and 5'-hydroxyl groups in 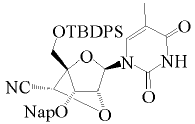 in R configuration to deprotection to obtain 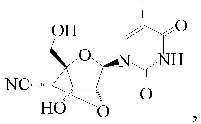 subjecting the 5'-hydroxyl group to DMTr protection to obtain 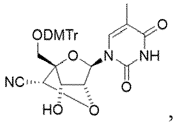 silylation modifying the 3'-hydroxyl group, converting a carbonyl group into an amino group to obtain 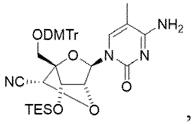 conducting base protection, subjecting the 3'-hydroxyl group to deprotection to obtain 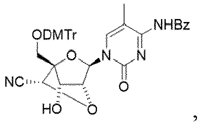 and then subjecting the 3'-hydroxyl group to phosphoramidation to obtain 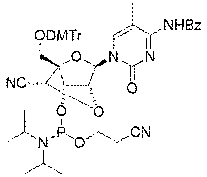 in R configuration; and
a synthesis process of the C6'-CN-modified LNA C phosphoramidite monomer in S configuration includes: subjecting 3'-hydroxyl and 5'-hydroxyl groups in 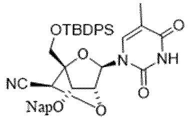 in S configuration to deprotection to obtain 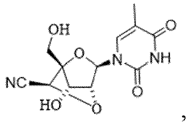 subjecting the 5'-hydroxyl group to DMTr protection to obtain 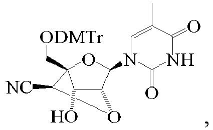 silylation modifying the 3'-hydroxyl group, converting a carbonyl group into an amino group to obtain 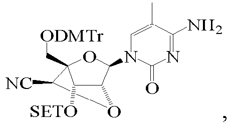 conducting base protection, subjecting the 3'-hydroxyl group to deprotection to obtain 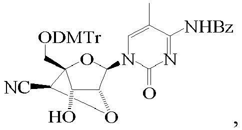 and then subjecting the 3'-hydroxyl group to phosphoramidation to obtain 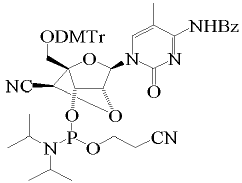 in S configuration.

In an eleventh aspect, the present disclosure provides a synthesis method of a NAP, including: polymerizing monomers to obtain the NAP; where
the monomer is selected from the group consisting of the 6'-CN-modified locked nucleoside and the nucleotide.

Compared with the prior art, the technical scheme of the present disclosure has the following beneficial effects:
In the present disclosure, the 6'-CN-modified locked nucleoside has a structure of R configuration or S configuration, and is further modified to obtain the nucleotide and the NAP. the inventor has found that a 6'-CN-modified NAP shows better nuclease resistance than unmodified or other modified NAPs, and they also reduce or regulate the interaction between the NAP and intracellular proteins. Moreover, in terms of improving the nuclease resistance of NAP and reducing the interaction between NAP and intracellular proteins, the 6'-CN-modified NAP in S configuration is more significant than that in R configuration. Therefore, the 6'-CN-modified locked nucleoside, the nucleotide, and the NAP exhibit a significant application value in the field of nucleic acid drugs.

The present disclosure also provides a general preparation method for the aforementioned R or S configuration 6'-cyano-modified locked nucleosides, nucleotides, and nucleic acid polymers, which is robust, efficient, and has a high yield.

Furthermore, the CN-LNA modified nucleic acid sequences obtained through the synthetic method of the present disclosure can significantly reduce the disturbance to the distribution of intracellular proteins compared to LNA modification, and reduce the induction of apoptosis effects, thus providing a significant advantage in the development of efficient and safe nucleic acid drugs.

### BRIEF DESCRIPTION OF THE DRAWINGS

In order to more clearly illustrate the technical solutions in the specific embodiments of the present disclosure or the prior art, the accompanying drawings required for describing the specific embodiments or the prior art are briefly described below. It is evident that the drawings described below are some embodiments of the present disclosure, and for ordinary technical personnel in this field, without the need for creative effort, other drawings can also be obtained based on these drawings.
FIG. 1 shows a mass spectrum of the nucleic acid ON1;
FIG. 2 shows a mass spectrum of the nucleic acid ON2;
FIG. 3 shows a mass spectrum of the nucleic acid ON3;
FIG. 4 is a 60× magnification showing the aggregation of P54nrb induced by ON1, ON2, and ON3;
FIG. 5 shows the activity results of Caspase 3/7;
FIG. 6 shows a mass spectrum of the nucleic acid ON4;
FIG. 7 shows the enzymatic stability of 5'-d(TTTTTTTTT)-3' to snake venom phosphodiesterase (SVPDE); and
FIG. 8 is a crystal structure diagram showing the S-6'-CN-LNA-T monomer (compound 36).

### DETAILED DESCRIPTION OF THE EMBODIMENTS

The technical solutions of the present disclosure will be described in detail in conjunction with the embodiments and accompanying drawings. However, those skilled in the art will understand that the following embodiments and examples are merely intended to illustrate the present disclosure, but should not be construed as limiting the scope of the present disclosure. All other embodiments obtained by those skilled in the art based on the embodiments of the present disclosure without creative efforts shall fall within the protection scope of the present disclosure. If no specific conditions are specified, the examples will be carried out according to conventional conditions or the conditions recommended by the manufacturer. All of the used reagents or instruments which are not specified with manufacturers are conventional commercially-available products.

The term "nucleic acid polymer (NAP)" may refer to any nucleic acid molecule having at least one sugar, nucleobase or internucleoside linkage that is modified relative to naturally occurring linked nucleosides, including but not limited to DNA, RNA, and hybrids thereof including but not limited to single-stranded and double-stranded hybrids. The reminder of the sugars, nucleobases and internucleoside linkages can be independently modified or unmodified wherein each nucleoside and linkage can be the same or different. It can be a therapeutic oligonucleotide typically ~20 nucleotides (nt) or a chimeric oligonucleotide with more nucleotides.

In a first aspect, the present disclosure provides 6'-CN-modified locked nucleosides, where the 6'-CN-modified locked nucleoside is selected from the group consisting of a compound with a structure shown in Formula 1, salts and isomers thereof: 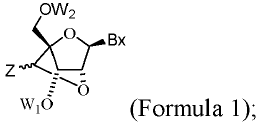
in Formula 1, Bx is selected from the group consisting of substituted or unsubstituted adenine, guanine, thymine, cytosine, and uracil, and salts thereof;
Z is CN;
W₁ and W₂ are independently selected from the group consisting of H or hydroxyl protecting group; the hydroxyl protecting group is selected from but not limited to the group consisting of acetyl, tert-butyl, tert-butoxymethyl, methoxymethyl, tetrahydropyranyl, 1-ethoxyethyl, 1-(2-chloroethoxy)ethyl, *p*-chlorophenyl, 2,4-dinitrophenyl, benzyl, 2,6-dichlorobenzyl, diphenylmethyl, *p*-nitrobenzyl, bis(2-acetoxyethoxy)methyl, 2-trimethylsilylethyl, trimethylsilyl, triethylsilyl, tert-butyldimethylsilyl, tert-butyldiphenylsilyl, triphenylsilyl, [(triisopropylsilyl)oxy]methyl, benzoylformate, chloroacetyl, trichloroacetyl, trifluoroacetyl, pivaloyl, benzoyl, *p*-phenylbenzoyl, 9-fluorenylmethyl carbonate, methanesulfonate, p-toluenesulfonate, trityl, monomethoxytrityl, dimethoxytrityl, trimethoxytrityl, 1-(2-fluorophenyl)-4-methoxypiperidin-4-yl, 9-phenylxanthin-9-yl, 9-(*p*-methoxyphenyl)xanthin-9-yl, and 2-naphthylmethyl; and
the isomer is selected from the group consisting of a 6'-CN-modified locked nucleoside with Z in R configuration shown in 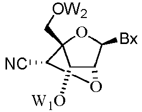 and a 6'-CN-modified locked nucleoside with Z group in S configuration shown in

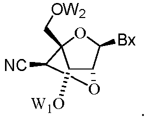

In the present disclosure, the 6'-CN-modified locked nucleosides may improve the nuclease resistance of NAP prepared from the same as a raw material and reduce the interaction between the NAP and intracellular proteins.

In a second aspect, the present disclosure provides nucleotides, including a 3'-active phosphorus group derivative of the 6'-CN-modified locked nucleoside; where
the active phosphorus group is selected from the group consisting of phosphoramidite, phosphoramidite derivative, H-phosphate, H-phosphate derivative, phosphate triester, and phosphate triester derivative.

In the present disclosure, the nucleotides may improve the nuclease resistance of NAP prepared from the same as a monomer and reduce the interaction between the NAP and intracellular proteins.

In some embodiments, the nucleotide is selected from the group consisting of a compound with a structure shown in Formula 2, salts and isomers thereof: 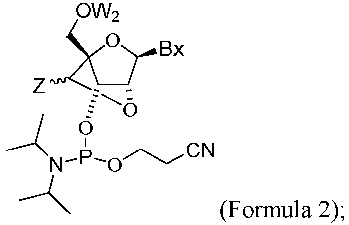 the isomer is selected from the group consisting of a nucleoside with Z in R configuration shown in 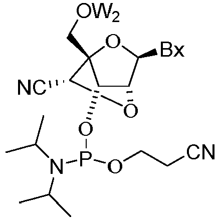 and a nucleoside with Z in S configuration shown in

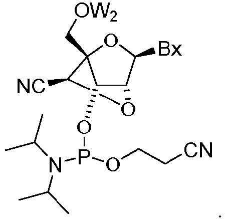

In a third aspect, the present disclosure provides use of the nucleotides in reducing an interaction between the NAP with an intracellular protein.

It is found by the inventor that the NAPs prepared with the nucleotides provided by the present disclosure have little interaction with the intracellular protein, thus helping to reduce *in vivo* toxicity of the NAP.

In a fourth aspect, the present disclosure provides NAPs, including a monomer with a structure shown in Formula 3: 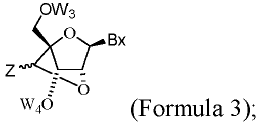 where
in Formula 3, Bx is selected from the group consisting of substituted or unsubstituted adenine, guanine, thymine, cytosine, and uracil, and salts thereof;
Z is CN;
W₃ and W₄ are independently selected from the group consisting of H, hydroxyl protecting group, and internucleoside linking group linking the monomer to other portions of the NAP; and at least one of W₃ and W₄ is the internucleoside linking group linking the monomer to the other portions of the NAP; and
the hydroxyl protecting group is selected from but not limited to the group consisting of acetyl, tert-butyl, tert-butoxymethyl, methoxymethyl, tetrahydropyranyl, 1-ethoxyethyl, 1-(2-chloroethoxy)ethyl, *p*-chlorophenyl, 2,4-dinitrophenyl, benzyl, 2,6-dichlorobenzyl, diphenylmethyl, *p*-nitrobenzyl, bis(2-acetoxyethoxy)methyl, 2-trimethylsilylethyl, trimethylsilyl, triethylsilyl, tert-butyldimethylsilyl, tert-butyldiphenylsilyl, triphenylsilyl, [(triisopropylsilyl)oxy]methyl, benzoylformate, chloroacetyl, trichloroacetyl, trifluoroacetyl, pivaloyl, benzoyl, *p*-phenylbenzoyl, 9-fluorenylmethyl carbonate, methanesulfonate, *p*-toluenesulfonate, trityl, monomethoxytrityl, dimethoxytrityl, trimethoxytrityl, 1-(2-fluorophenyl)-4-methoxypiperidin-4-yl, 9-phenylxanthin-9-yl, 9-(*p*-methoxyphenyl)xanthin-9-yl, and 2-naphthylmethyl.

In the present disclosure, the NAPs show better nuclease resistance than unmodified or other modified NAPs. The NAPs can reduce the interaction with intracellular proteins, reduce the impact on the subcellular distribution of intracellular proteins, and reduce cell apoptosis.

In some embodiments, the monomer is selected from the group consisting of a monomer with Z in R configuration shown in 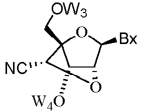 and a monomer with Z in S configuration shown in

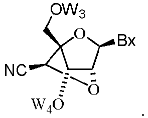

In some embodiments, the NAP is selected from the group consisting of an RNA, a DNA, or a copolymer of the RNA and the DNA.

In a fifth aspect, the present disclosure provides use of the NAPs in preparation of a nucleic acid diagnostic agent or a nucleic acid therapeutic agent.

In the present disclosure, the NAP shows better nuclease resistance than unmodified or other modified NAPs. Moreover, the NAPs have low interaction with intracellular proteins, low apoptosis-inducing effect, and small toxic and side effects, and may be used to prepare a nucleic acid diagnostic agent or nucleic acid therapeutic agent.

In a sixth aspect, the present disclosure provides a synthesis method of a C6'-CN-modified nucleoside in R configuration or S configuration, where the C6'-CN-modified nucleoside in R configuration is 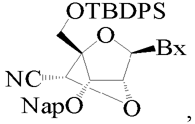 and the synthesis method includes: isomerizing a terminal olefin of 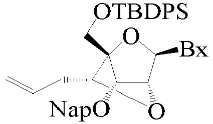 to obtain 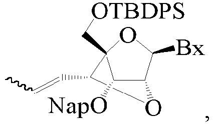 conducting dihydroxylation to obtain 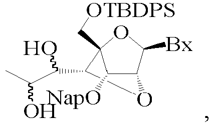 followed by oxidative cleavage to obtain an aldehyde compound 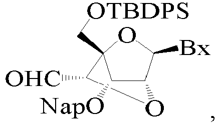 and then converting an aldehyde group of 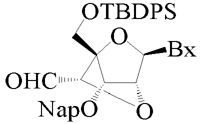 into a CN group to obtain the C6'-CN-modified nucleoside in R configuration 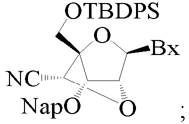
the C6'-CN-modified nucleoside in S configuration is 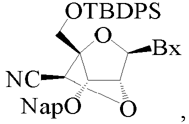 and the synthesis method includes: isomerizing a terminal olefin of 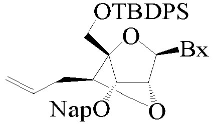 to obtain 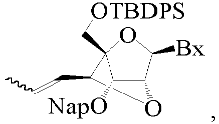 conducting dihydroxylation to obtain 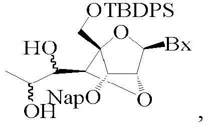 followed by oxidative cleavage to obtain an aldehyde compound 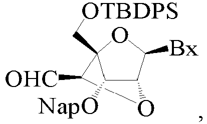 and then converting an aldehyde group of 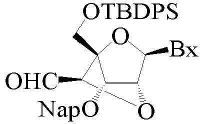 into a CN group to obtain the C6'-CN-modified nucleoside in S configuration, *i.e*., 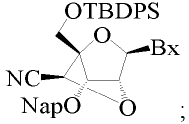 and
Bx is selected from the group consisting of substituted or unsubstituted adenine, guanine, thymine, cytosine, and uracil, and salts thereof.

In the present disclosure, there are no specific restrictions on the reaction processes of isomerization of terminal olefin, dihydroxylation, oxidative cleavage, and conversion of aldehyde group into CN group, and reaction methods well known to those skilled in the art can be used. The C6'-CN-modified nucleoside in R configuration or S configuration synthesized in the present disclosure can be used as a raw material for the synthesis of four kinds of CN-modified LNAs in R configuration or S configuration.

It should be noted that the aldehyde group of the aldehyde compound, an intermediate product of the above reaction, can be converted into other groups including alkanes, alcohols, carboxylic acids, alkynyl groups, alkenyl groups, and amines through conventional chemical methods, thereby achieving a variety of C6'-modified LNAs.

In some examples, a synthesis method of 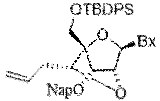 includes: methylsulfonating 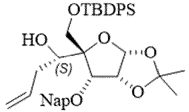 in S configuration, removing methylene of the methylsulfonated product, and acetylatiing to obtain 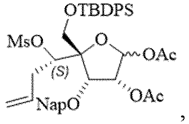 and then conducting glycosylation and nucleophilic substitution in sequence to obtain 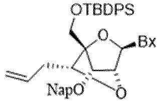 in R configuration;
a synthesis method of 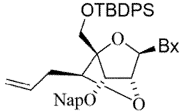 includes: methylsulfonating 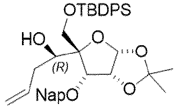 in R configuration, removing methylene of the methylsulfonated product, and acetylatiing to obtain 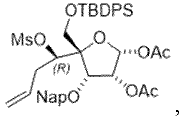 and then conducting glycosylation and nucleophilic substitution in sequence to obtain the 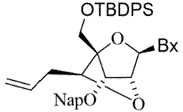 in S configuration; and
the glycosylation includes: subjecting 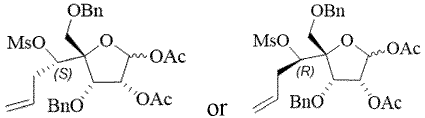 to a reaction with thymine, N6-benzoyladenine, or 6-chloroguanine at 50°C to 100°C in the presence of an activator; the activator includes *N*,*O*-bis(trimethylsilyl)acetamide (BSA) and trimethylsilyl trifluoromethanesulfonate (TMSOTf), and a reaction medium is selected from the group consisting of acetonitrile, 1,2-dichloroethane, and toluene.

In the present disclosure, the synthesis method is robust and practical. There are no specific restrictions on the mesylation, methylene removal, acetylation, and nucleophilic substitution, and reaction methods well known to those skilled in the art can be used.

In some embodiments, a synthesis method of 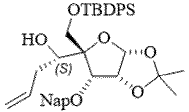 in S configuration includes: oxidizing a primary alcohol group of 5-*O*-(tert-butyldiphenylsilyl)-4-*C*-hydroxymethyl-1,2-*O*-isopropylidene-3-*O*-(2-naphthylmethyl)-α-*D*-ribofuranose 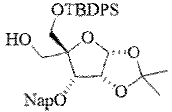 into an aldehyde group, and then allylating the aldehyde group to obtain 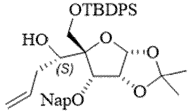 in S configuration;
in some embodiments, a synthesis method of 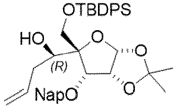 in R configuration includes: oxidizing a secondary alcohol group of 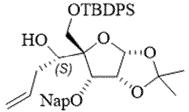 into a ketone group, and then reducing the ketone group into a secondary alcohol group to obtain 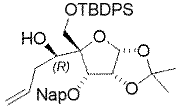 in R configuration; and
in the process of reducing the ketone group into the secondary alcohol group, a reducing agent used is a metal hydrogen compound, such as lithium aluminum hydride, lithium borohydride, lithium chloride, and sodium borohydride; a reaction medium is a solvent, such as DCM, THF, methanol, and ethanol, and the reducing is conducted at -78°C to 0°C.

In some embodiments, a step of reducing the ketone group into the secondary alcohol group includes: conducting the reducing at -40°C to 0°C using a mixture of the sodium borohydride and the lithium chloride as the reducing agent and using a mixture of the THF and the methanol as the reaction medium.

In some preferred embodiments, the catalyst for the isomerization of the terminal olefin is a transition metal catalyst selected from the group consisting of a ruthenium catalyst, a palladium catalyst, a rhodium catalyst, and an iridium catalyst, preferably carbonylchlorohydridotris(triphenylphosphine)ruthenium (II);
a reaction medium for the isomerization of the terminal olefin is selected from the group consisting of methanol, ethanol, n-butanol, and toluene, preferably the ethanol;
the isomerization of the terminal olefin is conducted at 60°C to 100°C, preferably 60°C to 80°C; and
the isomerization of the terminal olefin is conducted for 12 h to 72 h.

It is found by the inventor that the isomerization of the terminal olefin can be stably realized using 2.5% mol to 5% mol of the carbonylchlorohydridotris(triphenylphosphine)ruthenium (II) as the catalyst and ethanol as the reaction solvent without anhydrous and oxygen-free operations.

In a seventh aspect, the present disclosure provides a synthesis method of a C6'-CN-modified LNA T phosphoramidite monomer in R configuration or S configuration, where a preparation method of the C6'-CN-modified LNA T phosphoramidite monomer in R configuration includes: subjecting 3'-hydroxyl and 5'-hydroxyl groups in the 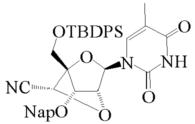 in R configuration to deprotection to obtain 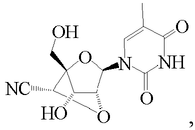 and then subjecting the 5'-hydroxyl group to DMTr protection while subjecting the 3'-hydroxyl group to phosphoramidation to obtain 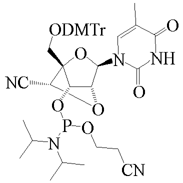 in R configuration; and
a preparation method of the C6'-CN-modified LNA T phosphoramidite monomer in S configuration includes: subjecting 3'-hydroxyl and 5'-hydroxyl groups in 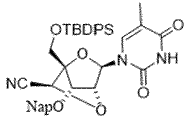 in S configuration to deprotection to obtain 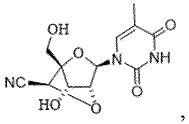 and then subjecting the 5'-hydroxyl group to DMTr protection while subjecting the 3'-hydroxyl group to phosphoramidation to obtain 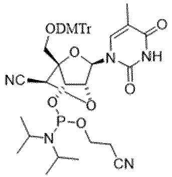 in S configuration.

This synthesis method is robust and efficient, and can be universally used for the synthesis of C6'-CN-modified LNA T phosphoramidite monomer in R configuration or S configuration.

In an eighth aspect, the present disclosure provides a synthesis method of a C6'-CN-modified LNA A phosphoramidite monomer in R configuration or S configuration, where a preparation method of the C6'-CN-modified LNA A phosphoramidite monomer in R configuration includes: subjecting a base in 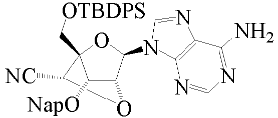 in R configuration to protection to obtain 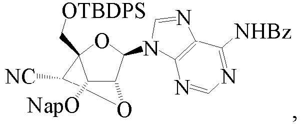 subjecting 3'-hydroxyl and 5'-hydroxyl groups to deprotection to obtain 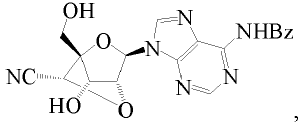 and then subjecting the 5'-hydroxyl group to DMTr protection while subjecting the 3'-hydroxyl group to phosphoramidation to obtain 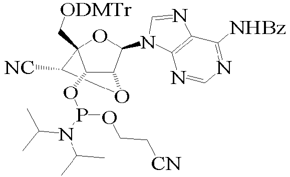 in R configuration; and
a preparation method of the C6'-CN-modified LNA A phosphoramidite monomer in S configuration includes: subjecting a base in 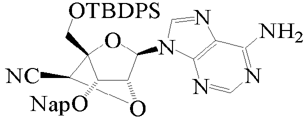 in S configuration to protection to obtain 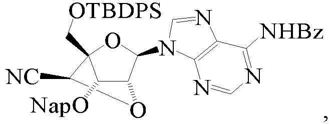 subjecting 3'-hydroxyl and 5'-hydroxyl groups to deprotection to obtain 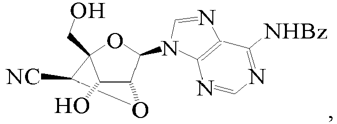 and then subjecting the 5'-hydroxyl group to DMTr protection while subjecting the 3'-hydroxyl group to phosphoramidation to obtain 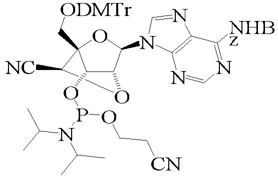 in S configuration.

This synthesis method is robust and efficient, and can be universally used for the synthesis of C6'-CN-modified LNA A phosphoramidite monomer in R configuration or S configuration.

In a ninth aspect, the present disclosure provides a synthesis method of a C6'-CN-modified LNA G phosphoramidite monomer in R configuration or S configuration, where the preparation method of the C6'-CN-modified LNA G phosphoramidite monomer in R configuration includes: demethylating a methoxy group in 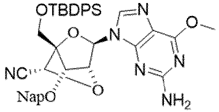 in R configuration to obtain 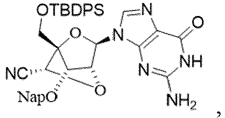 conducting base protection to obtain 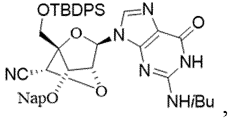 subjecting 3'-hydroxyl and 5'-hydroxyl groups to deprotection to obtain 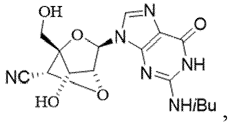 and then subjecting the 5'-hydroxyl group to DMTr protection while subjecting the 3'-hydroxyl group to phosphoramidation to obtain 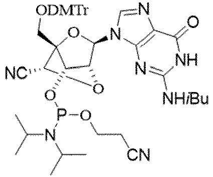 in R configuration; and
a preparation method of the C6'-CN-modified LNA G phosphoramidite monomer in S configuration includes: demethylating a methoxy group in 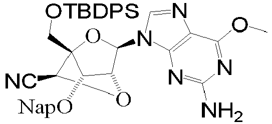 in S configuration to obtain 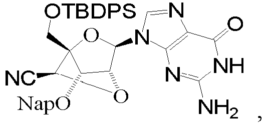 conducting base protection to obtain 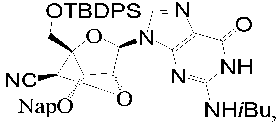 subjecting 3'-hydroxyl and 5'-hydroxyl groups to deprotection to obtain 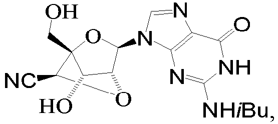 and then subjecting the 5'-hydroxyl group to DMTr protection while subjecting the 3'-hydroxyl group to phosphoramidation to obtain 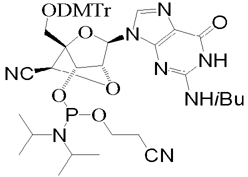 in S configuration.

This synthesis method is robust and efficient, and can be universally used for the synthesis of C6'-CN-modified LNA G phosphoramidite monomer in R configuration or S configuration.

In a tenth aspect, the present disclosure provides a synthesis method of a C6'-CN-modified LNA C phosphoramidite monomer in R configuration or S configuration, where a synthesis method of the C6'-CN-modified LNA C phosphoramidite monomer in R configuration includes: subjecting 3'-hydroxyl and 5'-hydroxyl groups in 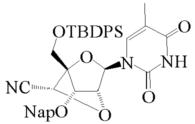 in R configuration to deprotection to obtain 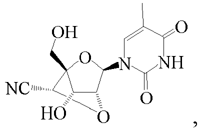 subjecting the 5'-hydroxyl group to DMTr protection to obtain 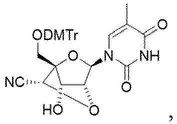 silylation modifying the 3'-hydroxyl group, converting a carbonyl group into an amino group to obtain 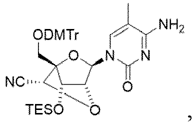 conducting base protection, subjecting the 3'-hydroxyl group to deprotection to obtain 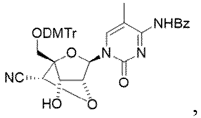 and then subjecting the 3'-hydroxyl group to phosphoramidation to obtain 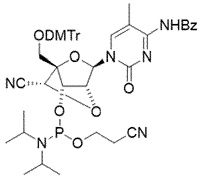 in R configuration; and
a synthesis method of the C6'-CN-modified LNA C phosphoramidite monomer in S configuration includes: subjecting 3'-hydroxyl and 5'-hydroxyl groups in 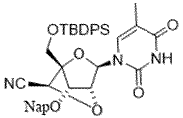 in S configuration to deprotection to obtain 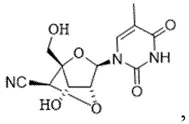 subjecting the 5'-hydroxyl group to DMTr protection to obtain 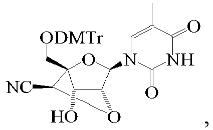 silylation modifying the 3'-hydroxyl group, converting a carbonyl group into an amino group to obtain 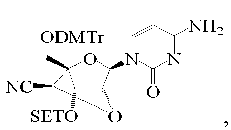 conducting base protection, subjecting the 3'-hydroxyl group to deprotection to obtain 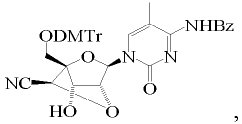 and then subjecting the 3'-hydroxyl group to phosphoramidation to obtain 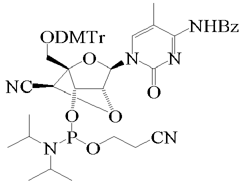 in S configuration.

This synthesis method is robust and efficient, and can be universally used for the synthesis of C6'-CN-modified LNA C phosphoramidite monomer in R configuration or S configuration.

In an eleventh aspect, the present disclosure provides a synthesis method of a NAP, including: polymerizing monomers to obtain the NAP; where
the monomer is selected from the group consisting of the 6'-CN-modified locked nucleoside and the nucleotide.

In some preferred embodiments, the polymerization includes: (i) Chemical synthesis. Nucleic acids are synthesized entirely by organic chemical methods using nucleosides or single nucleotides as raw materials, including: phosphate diester method, phosphate triester method, phosphite triester method, and solid-phase synthesis method. (ii) Enzymatic synthesis. Through enzymatic synthesis, nucleic acid monomers or chemically-synthesized small fragments may be linked into large fragments.

The present disclosure will be further illustrated below through specific examples. However, it should be understood that these examples are for the purpose of more detailed illustration only, and should not be understood as being intended to limit the present disclosure in any way.

### Example 1 Stereoselective synthesis of cyano-modified locked nucleic acid (CN-LNA)

### Part I: Synthesis of R-6'-CN-LNA

### Synthesis of R-6'-allyl-LNA

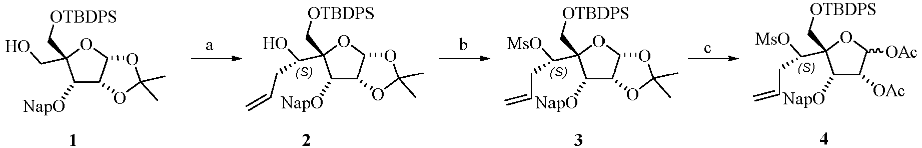

The reaction conditions were: (a) (1) 2-iodoacylbenzoic acid, acetonitrile, refluxing, 5 h; (2) allyltrimethylsilane, boron trifluoride ether solution, DCM, -40°C, 4 h; (b) methanesulfonyl chloride, DMAP, pyridine, room temperature, 16 h; (c) (1) FeCl₃·6H₂O, DCM, 0°C to room temperature, 9 h; (2) Ac₂O, DMAP, pyridine, DCM, room temperature, 2 h.

2
The compound 1 (100.0 g, 0.167 mol) was dissolved in 500 mL of acetonitrile, 2-iodoacylbenzoic acid (56 g, 0.20 mol) was added, the mixture was heated to allow refluxing for 5 h, and the reaction was monitored by TLC (petroleum ether/ethyl acetate=5/1). After the reaction was terminated, the reaction solution was cooled to room temperature and filtered through diatomaceous earth. The resulting filter cake was washed twice with acetonitrile, the solvent was spin-dried, and the remaining substance was dried under vacuum to obtain 101 g of a light yellow liquid, which was directly used for the next reaction without purification.

The obtained aldehyde compound was dissolved in 500 mL of DCM, cooled to -40°C, and added with boron trifluoride ether solution (22 mL, 0.217 mol) to allow reaction by stirring for 5 min; the allyltrimethylsilane (42.7 mL, 0.217 mol) was added dropwise, the temperature was maintained and the resulting mixture was reacted by stirring for 4 h, and the reaction was monitored by TLC (petroleum ether/ethyl acetate=5/1). The resulting reaction solution was quickly poured into 1 L of saturated sodium bicarbonate aqueous solution to allow quenching; a resulting product was extracted with DCM, washed with saturated saline solution, dried over anhydrous NaSO₄, filtered, and subjected to vacuum concentration to obtain 107 g of light yellow thick compound 2, with a yield of 100%. The above product was directly used in the next reaction without purification.

¹H NMR (CDCl₃, 400 MHz) *δ* 7.86-7.78 (m, 4H), 7.60-7.57 (m, 2H), 7.53-7.46 (m, 5H), 7.41-7.27 (m, 6H), 5.87-5.80 (m, 2H), 5.05-4.97 (m, 3H), 4.76 (t, J = 5.2 Hz, 1H), 4.70-4.66 (m, 2H), 4.47 (dd, J = 10.7, 1.7 Hz, 1H), 3.96 (d, J = 11.2 Hz, 1H), 3.80 (d, J = 11.2 Hz, 1H), 3.44 (br s, 1H), 2.54-2.49 (m, 1H), 1.93-1.85 (m, 1H), 1.63 (s, 3H), 1.39 (s, 3H), 0.90 (s, 9H); ¹³C NMR (CDCl₃, 100 MHz) *δ* 136.41, 135.58, 135.50, 134.29, 133.30, 133.22, 133.13, 132.99, 129.76, 129.62, 128.76, 127.99, 127.78, 127.75, 127.70, 127.31, 126.38, 126.32, 125.71, 116.26, 113.78, 104.70, 88.22, 79.18, 78.11, 73.09, 72.56, 62.46, 34.65, 27.09, 26.73, 26.54, 19.15; ESI-MS (m/z) 637.37 [M - H]⁻.

3
The compound 2 (100.0 g, 0.157 mol) and DMAP (1.9 g, 15.7 mmol) were dissolved in 500 mL of pyridine at room temperature, methanesulfonyl chloride (18 mL, 0.235 mol) was added dropwise, and the mixture was reacted at room temperature for 16 h, the reaction was monitored by TLC (petroleum ether/ethyl acetate=5/1). the reaction solution was added with 100 mL of methanol, stirred for 10 min to quench the reaction, the reaction solution was subjected to vacuum concentration to remove excess pyridine, diluted with ethyl acetate, washed with water, 1 N HCl solution, saturated saline solution in sequence, and dried over anhydrous sodium sulfate, followed by filtering and concentrating, then the residue was purified by flash column (gradient elution: petroleum ether/ethyl acetate=0%-25%) to obtain 108.0 g of colorless thick compound 3, with a yield of 96%.

¹H NMR (DMSO-*d*₆, 400 MHz) *δ* 7.95-7.85 (m, 4H), 7.56-7.36 (m, 13H), 5.80-5.69 (m, 2H), 5.40 (dd, *J =* 7.6, 3.2 Hz, 1H), 5.03-4.93 (m, 4H), 4.69 (d, *J =* 12.4 Hz, 1H), 4.40 (d, *J =* 5.3 Hz, 1H), 3.75 (d, *J* = 11.0 Hz, 1H), 3.60 (d, *J* = 11.0 Hz, 1H), 2.97 (s, 3H), 2.85-2.81 (m, 1H), 2.58-2.52 (m, 1H), 1.53 (s, 1H), 1.33 (s, 1H), 0.78 (s, 9H); ¹³C NMR (CDCl₃, 100 MHz) *δ* 135.59, 135.52, 135.29, 134.59, 133.25, 133.19, 132.88, 132.84, 130.39, 130.36, 128.59, 128.34, 128.29, 128.24, 128.09, 127.33, 126.70, 126.55, 126.34, 118.60, 113.27, 105.00, 87.47, 81.43, 78.15, 76.97, 71.93, 64.30, 36.19, 26.83, 26.57, 26.33, 19.04; ESI-MS (m/z) 734.39 [M + NH₄]⁺, 739.34 [M + Na]⁺.

4
The 600 mL of DCM solution containing the compound 3 (108 g, 0.15 mol) was cooled to 0°C, added with ferric chloride hexahydrate (16.3 g, 0.06 mol), and a reaction was conducted at room temperature for 9 h, the reaction was monitored by TLC (petroleum ether/ethyl acetate=4/1). A reaction solution was poured into 1.5 L of water, and extracted with DCM, a resulting organic layer was washed with water, saturated sodium bicarbonate solution, and saturated saline solution in sequence, dried over anhydrous magnesium sulfate, filtered, the obtained filtrate was subjected to vacuum concentration, and dried under vacuum. An obtained thick substance was dissolved in 500 mL of DCM, pyridine (72 mL, 0.9 mol), DMAP (2.75 g, 22.5 mol) were added in sequence, and acetic anhydride (85 mL, 0.9 mol) was added dropwise, and the resulting mixture was subjected to reaction at room temperature for 2 h, the reaction was monitored by TLC (petroleum ether/ethyl acetate=4/1). The reaction solution was poured into 2 L of water and extracted with DCM, a resulting organic layer was washed successively with water, 1 N HCl solution, saturated sodium bicarbonate solution, and saturated saline solution, dried over anhydrous magnesium sulfate, filtered, the obtained filtrate was concentrated, and flash column-purified (gradient elution: petroleum ether/ethyl acetate=0%-60%) to obtain a 68 g colorless thick substance, i.e. the epimer mixture of a diacetyl compound 4, with a yield of 60%. ESI-MS (m/z) 778.39 [M + NH₄]⁺, 783.44 [M + Na]⁺.

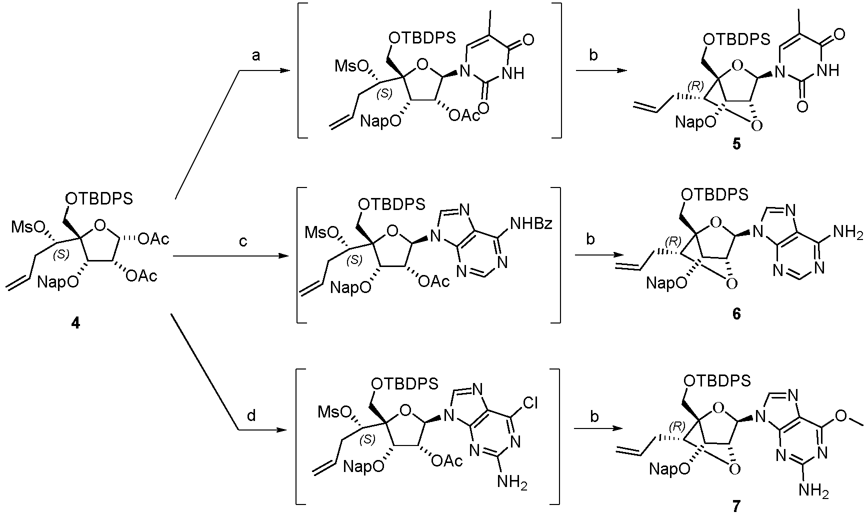

The reaction conditions were: (a) thymine, BSA, TMSOTf, acetonitrile, 80°C, 3 h; (b) K₂CO₃, methanol, room temperature, 16 h; (c) N6-benzoyladenine, BSA, TMSOTf, toluene, 100°C, 3 h; (d) 6-chloroguanine, BSA, TMSOTf, toluene, 100°C, 3 h.

5
BSA (29 mL, 120 mmol) was added to a 50 mL suspension of thymine (7.6 g, 60 mmol) in acetonitrile, and the resulting mixture was stirred at room temperature for 20 min until the thymine was dissolved. After the system became clear, 200 mL of acetonitrile solution of diacetylated sugar 4 (23 g, 30 mmol) was added, and then TMSOTf (8.3 mL, 45 mmol) were quickly added dropwise, the obtained mixture was heated to 80°C to allow reaction by stirring for 3 h, the reaction was monitored by TLC (petroleum ether/ethyl acetate=5/1). After stopping heating and allowing the reaction solution to cool to room temperature, the reaction solution was poured into ethyl acetate, added with a semisaturated NaHCO₃ solution (400 mL), and stirred for 20 min. The insoluble solid was filtered off with diatomaceous earth, while the organic layer was separated and then washed with water and saturated saline solution in sequence, dried over anhydrous NaSO₄, filtered, the obtained filtrate was concentrated, and dried under vacuum. An obtained residue was dissolved in 250 mL of methanol, added with K₂CO₃ (12.4 g, 90 mmol), and reacted at room temperature for 16 h, the reaction was monitored by TLC (DCM/ethyl acetate=10/3). After the reaction solution was concentrated, a residue was extracted with ethyl acetate, washed with water and saturated saline solution in sequence, dried over anhydrous NaSO₄, filtered, the obtained filtrate was concentrated, and purified by flash column (gradient elution: ethyl acetate/DCM=0%-40%), such that 8 g of an LNA derivative 5 was obtained as a white foamy solid, with a yield of 39%. ¹H NMR (CDCl₃, 400 MHz) *δ* 8.72 (br s, 1H), 7.82-7.74 (m, 3H), 7.72 (s, 1H), 7.68-7.63 (m, 4H), 7.59 (s, 1H), 7.75-7.28 (m, 9H), 5.80-5.69 (m, 1H), 5.66 (s, 1H), 5.11-5.00 (m, 2H), 4.87 (d, *J =* 11.4 Hz, 1H), 4.74-4.71 (d, *J =* 11.4 Hz, 1H), 4.57 (s, 1H), 4.24-4.20 (m, 2H), 4.03 (d, *J =* 12.0 Hz, 1H), 3.91 (d, *J =* 12.0 Hz, 1H), 2.49-2.42 (m, 1H), 2.26-2.22 (m, 1H), 1.56 (s, 3H), 1.09 (s, 9H); ¹³C NMR (CDCl₃, 100 MHz) *δ* 163.60, 149.72, 135.44, 135.27, 134.23, 134.16, 133.64, 133.11, 132.70, 132.25, 130.10, 130.06, 128.42, 127.98, 127.85, 127.73, 126.88, 126.39, 126.25, 125.74, 117.88, 110.42, 89.20, 86.82, 79.97, 77.05, 76,47, 72.53, 58.80, 33.76, 26.97, 19.44, 12.04; ESI-MS (m/z) 689.41 [M + H]⁺, 711.38 [M + Na]⁺.

6
The 50 mL toluene suspension containing N6-benzoyladenine (14 g, 60 mmol) was added with BSA (29 mL, 120 mmol), and the resulting mixture was heated to 60°C for reaction until the N6-benzoyladenine was dissolved. After the system became clear, the reaction solution was cooled to room temperature, 170 mL of toluene solution containing diacetylated sugar 4 (23 g, 30 mmol) was added, and then TMSOTf (8.3 mL, 45 mmol) were quickly added dropwise, the obtained mixture was heated to 100°C to allow reaction by stirring for 3 h, the reaction was monitored by TLC (petroleum ether/ethyl acetate=5/1). After stopping heating and allowing a reaction solution to cool to room temperature, the reaction solution was poured into ethyl acetate, added with a semisaturated NaHCO₃ solution (400 mL), and stirred for 20 min. The insoluble solid was filtered off with diatomaceous earth, while the organic layer was separated and then washed by water and saturated saline solution in sequence, dried over anhydrous NaSO₄, filtered, the obtained filtrate was concentrated, and dried under vacuum. The obtained residue was dissolved in 250 mL of methanol, added with K₂CO₃ (20.7 g, 150 mmol), and reacted at room temperature for 16 h, the reaction was monitored by TLC (DCM/ethyl acetate=10/3). After the reaction solution was concentrated, a residue was extracted with ethyl acetate, washed with water and saturated saline solution in sequence, dried over anhydrous NaSO₄, filtered, the obtained filtrate was concentrated, and purified by flash column (gradient elution: ethyl acetate/DCM=0%-100%), such that 7.4 g of an LNA derivative 6 was obtained as a white powder, with a yield of 35%. ¹H NMR (CDCl₃, 400 MHz) *δ* 8.28 (s, 1H), 8.15 (s, 1H), 7.80-7.64 (m, 8H), 7.47-7.28 (m, 9H), 6.06 (s, 1H), 5.93-5.91 (m, 2H), 5.82-5.75 (m, 1H), 5.13 (d, *J* = 17.1 Hz, 1H), 5.04 (d, *J* = 10.2 Hz, 1H), 4.81-4.72 (m, 3H), 4.52 (s, 1H), 4.32-4.29 (m, 1H), 4.02 (d, *J =* 12.0 Hz, 1H), 3.92 (d, *J =* 12.0 Hz, 1H), 2.55-2.49 (m, 1H), 2.34-2.27 (m, 1H), 1.07 (s, 9H); ¹³C NMR (CDCl₃, 100 MHz) *δ* 155.46, 153.16, 148.82, 135.59, 135.49, 130.02, 129.99, 128.36, 127.96, 127.92, 127.83, 127.70, 126.78, 126.29, 120.09, 117.83, 88.57, 86.16, 80.45, 78.74, 76.64, 72.75, 58.82, 33.90, 26.79, 19.22; ESI-MS (m/z) 698.36 [M + H]⁺, 720.35 [M + Na]⁺.

7
50 mL toluene suspension containing 6-chloroguanine (10.2 g, 60 mmol) was added with BSA (29 mL, 120 mmol), and heated to 60°C for reaction until the 6-chloroguanine was dissolved. After the system became clear, the reaction solution was cooled to room temperature, 170 mL of toluene solution containing diacetylated sugar 4 (23 g, 30 mmol) was added, and then TMSOTf (8.3 mL, 45 mmol) were quickly added dropwise, the resulting mixture was heated to 100°C to allow reaction by stirring for 3 h, the reaction was monitored by TLC (petroleum ether/ethyl acetate=5/1). After stopping heating and allowing a reaction solution to cool to room temperature, the reaction solution was poured into ethyl acetate, added with a semisaturated NaHCO₃ solution (400 mL), and stirred for 20 min. The insoluble solid was filtered off with diatomaceous earth, while the organic layer was separated and then washed by water and saturated saline solution in sequence, dried over anhydrous NaSO₄, filtered, the obtained filtrate was concentrated, and dried under vacuum. An obtained residue was dissolved in 250 mL of methanol, added with K₂CO₃ (20.7 g, 150 mmol), and reacted at room temperature for 16 h, the reaction was monitored by TLC (DCM/ethyl acetate=10/3). After the reaction solution was concentrated, a residue was extracted with ethyl acetate, washed with water and saturated saline solution in sequence, dried over anhydrous NaSO₄, filtered, the obtained filtrate was concentrated, and purified by flash column (gradient elution: ethyl acetate/DCM=0%-30%), such that 12.2 g of an LNA derivative 7 was obtained as a white foamy solid, with a yield of 56%. ¹H NMR (CDCl₃, 400 MHz) *δ* 7.98 (s, 1H), 7.82-7.67 (m, 8H), 7.49-7.36 (m, 7H), 7.31-7.27 (m, 2H), 5.92 (s, 1H), 5.84-5.74 (m, 1H), 5.14-5.01 (m, 2H), 4.95 (br s, 2H), 4.79 (dd, *J* = 17.7, 11.5 Hz, 2H), 4.58 (s, 1H), 4.53 (s, 1H), 4.31 (dd, *J* = 8.6, 5.0 Hz, 1H), 4.08 (s, 3H), 4.00 (d, *J =* 12.0 Hz, 1H), 3.90 (d, *J =* 12.0 Hz, 1H), 2.56-2.48 (m, 1H), 2.34-2.27 (m, 1H), 1.07 (s, 9H); ¹³C NMR (CDCl₃, 100 MHz) *δ* 161.49, 159.36, 142.45, 136.44, 135.62, 134.47, 133.94, 133.14, 133.08, 132.53, 132.47, 129.97, 128.34, 127.94, 127.90, 127.69, 126.67, 126.25, 126.12, 125.65, 117.75, 116.09, 88.31, 85.93, 80.46, 78.93, 72.80, 58.86, 53.91, 33.93, 26.78, 19.17; ESI-MS (m/z) 728.39 [M + H]⁺.

### Synthesis of R-6'-CN-LNA

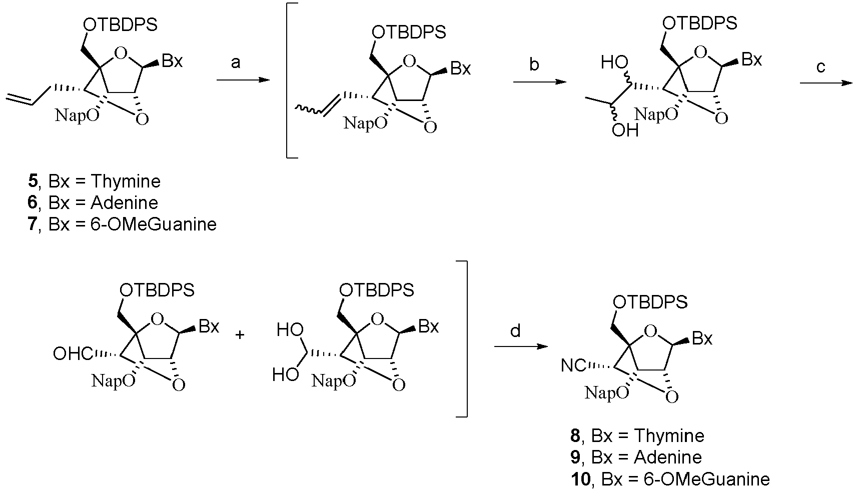

The reaction conditions were: (a) (1) carbonylchlorohydridotris(triphenylphosphine)ruthenium (II) (Wilkinson's catalyst), EtOH, 80°C, 48 h; (b) potassium osmate dihydrate, 50% NMO, t-BuOH, H₂O, 60°C, 8 h; (c) NaIO₄, EtOH-THF (2/1 v/v), H₂O, room temperature, 12 h; (d) NH₄OH, I₂, room temperature, 24 h.

8
The carbonylchlorohydridotris(triphenylphosphine)ruthenium (II) (345 mg, 0.36 mmol, 5% mol) was added into 50 mL of absolute ethanol solution containing allyl nucleoside derivative 5 (5.0 g, 7.26 mmol), and heated to 80°C for reaction for 48 h. The reaction solution was concentrated directly, the residue was dissolved in 40 mL of THF, and added with 5 mL of tert-butanol, 5 mL of water, potassium osmate dihydrate (26.7 mg, 72.6 µmol, 1% mol), and 50% *N*-methylmorpholine-*N*-oxide (2.3 mL, 10.9 mmol) in sequence. The resulting mixture was reacted at 60°C for 8 h, the reaction was monitored by TLC (DCM/methanol=10/1). After the reaction was quenched with saturated sodium sulfite solution, a product was extracted with ethyl acetate, washed with water, saturated saline solution in sequence, dried over anhydrous sodium sulfate and filtered, the obtained filtrate was concentrated, and purified by flash column (gradient elution: ethyl acetate/DCM containing 5% methanol=0%-80%) to obtain 5 g of a dihydroxylated isomeric mixed product.

The above dihydroxylated product was dissolved in 60 mL of ethanol-THF solution, 10 mL aqueous solution containing NaIO₄ (2.33 g, 10.9 mmol) was added dropwise, and stirred at room temperature for 12 h. After filtering out the insoluble solid, 25% ammonia water (5.5 mL, 72.6 mmol) was added into the obtained filtrate, and then I₂ (1.84 g, 7.26 mmol) was added in batches and reacted at room temperature for 24 h. After quenching the reaction with saturated sodium sulfite solution, the product was extracted with ethyl acetate, washed with water, saturated saline solution in sequence, dried over anhydrous sodium sulfate and filtered, the obtained filtrate was concentrated, and purified by flash column (gradient elution: ethyl acetate/DCM containing 5% methanol=0%-60%), thus obtaining 3.3 g of slightly gray foamy solid product 8, with a yield of 48%. ¹H NMR (CDCl₃, 400 MHz) *δ* 8.99, 7.85-7.76 (m, 3H), 7.68-7.64 (m, 5H), 7.52-7.42 (m, 5H), 7.38-7.33 (m, 5H), 5.84 (s, 1H), 4.87 (s, 1H), 4.83 (s, 1H), 4.82 (d, *J =* 6.6 Hz, 1H), 4.71 (d, *J =* 6.6 Hz, 1H), 4.24 (s, 1H), 4.17 (d, *J =* 6.9 Hz, 1H), 4.01 (d, *J =* 6.9 Hz, 1H), 1.63 (s, 3H), 1.10 (s, 9H); ¹³C NMR (CDCl₃, 100 MHz) *δ* 163.50, 149.61, 135.53, 135.32, 133.63, 133.25, 133.21, 133.05, 132.10, 131.80, 130.36, 130.32, 128.68, 128.15, 128.10, 127.86, 127.80, 127.15, 126.63, 126.56, 125.58, 115.47, 111.18, 89.24, 87.30, 78.06, 76.26, 72.81, 70.55, 58.15, 26.86, 19.43, 12.24; ESI-MS (m/z) 674.28 [M + H]⁺, 796.25 [M + Na]⁺.

9
The carbonylchlorohydridotris(triphenylphosphine)ruthenium (II) (475 mg, 0.5 mmol, 5% mol) was added into 70 mL of absolute ethanol solution containing allyl nucleoside 6 (7.0 g, 10 mmol), and heated to 80°C for reaction for 48 h. The reaction solution was concentrated directly, and the residue was dissolved in 50 mL of THF, and added with 10 mL of tert-butanol, 10 mL of water, potassium osmate dihydrate (37 mg, 0.1 mmol, 1% mol) and 50% N-methylmorpholine-N-oxide (3.13 mL, 15 mmol) in sequence. The resulting mixture was reacted at 60°C for 8 h, the reaction was monitored by TLC (DCM/methanol=10/1). After the reaction was quenched with saturated sodium sulfite solution, a product was extracted with ethyl acetate, washed with water, saturated saline solution in sequence, dried over anhydrous sodium sulfate and filtered, the obtained filtrate was concentrated, and purified by flash column (gradient elution: ethyl acetate/DCM=0%-10%) to obtain 7.5 g of a dihydroxylated isomeric mixed product.

The above dihydroxylated product was dissolved in 60 mL of ethanol-THF solution, 15 mL aqueous solution containing NaIO₄ (3.2 g, 10.9 mmol) was added dropwise, and stirred at room temperature for 12 h. After filtering out the insoluble solid, 25% ammonia water (7.6 mL, 100 mmol) was added into the obtained filtrate, and then I₂ (2.54 g, 10 mmol) was added in batches and reacted at room temperature for 24 h. After quenching the reaction with saturated sodium sulfite solution, the product was extracted with ethyl acetate, washed with water, saturated saline solution in sequence, dried over anhydrous sodium sulfate and filtered, the obtained filtrate was concentrated, and purified by flash column (gradient elution: ethyl acetate/DCM=0%-40%), thus obtaining 1.2 g of slightly yellow foamy solid product 9, with a yield of 26%. ¹H NMR (DMSO-*d*₆, 400 MHz) *δ* 10.71 (s, 1H), 7.92-7.81 (m, 5H), 7.66-7.61 (m, 4H), 7.54-7.36 (m, 9H), 6.66 (br s, 2H), 6.04 (s, 1H), 5.30 (s, 1H), 5.13 (s, 1H), 4.92 (d, *J =* 12.0 Hz, 1H), 4.84 (d, *J =* 12.0 Hz, 1H), 4.79 (s, 1H), 4.21 (d, *J =* 12.2 Hz, 1H), 4.00 (d, *J =* 12.2 Hz, 1H), 0.94 (s, 9H); ¹³C NMR (DMSO-*d*₆, 100 MHz) *δ* 157.14, 154.49, 151.10, 135.62, 135.28, 134.48, 132.15, 130.53, 128.44, 128.15, 128.07, 128.76, 126.64, 126.57, 126.17, 117.74, 116.98, 88.23, 84.86, 78.84, 78.44, 71.98, 71.22, 59.76, 26.77, 19.19; ESI-MS (m/z) 683.30 [M + H]⁺, 705.27 [M + Na]⁺.

10
The carbonylchlorohydridotris(triphenylphosphine)ruthenium (II) (718 mg, 0.76 mmol, 5% mol) was added into 120 mL of absolute ethanol solution containing allyl nucleoside derivative 7 (11.0 g, 15.1 mmol), and heated to 80°C for reaction for 48 h. The reaction solution was concentrated directly, and the residue was dissolved in 80 mL of THF, and added with 10 mL of tert-butanol, 10 mL of water in sequence, potassium osmate dihydrate (56 mg, 0.15 mmol, 1% mol) and 50% N-methylmorpholine-N-oxide (4.7 mL, 22.7 mmol) in sequence. The resulting mixture was reacted at 60°C for 8 h, the reaction was monitored by TLC (DCM/methanol=10/1). After the reaction was quenched with saturated sodium sulfite solution, a product was extracted with ethyl acetate, washed with water, saturated saline solution in sequence, dried over anhydrous sodium sulfate and filtered, the obtained filtrate was concentrated, and purified by flash column (gradient elution: ethyl acetate/DCM=0%-60%) to obtain 8 g of a dihydroxylated isomeric mixed product.

The above dihydroxylated product was dissolved in 70 mL of ethanol-THF solution, 15 mL aqueous solution containing NaIO₄ (3.4 g, 10.9 mmol) was added dropwise, and stirred at room temperature for 12 h. After filtering out the insoluble solid, 25% ammonia water (7.9 mL, 105 mmol) was added into the obtained filtrate, and then I₂ (2.67 g, 10.5 mmol) was added in batches and reacted at room temperature for 24 h. After quenching the reaction with saturated sodium sulfite solution, the product was extracted with ethyl acetate, washed with water, saturated saline solution in sequence, dried over anhydrous sodium sulfate and filtered, the obtained filtrate was concentrated, and purified by flash column (gradient elution: ethyl acetate/DCM=0%-40%), thus obtaining 4 g of slightly yellow foamy solid product 10, with a yield of 37%. ¹H NMR (CDCl₃, 400 MHz) *δ* 7.82-7.76 (m, 3H), 7.71-7.16 (m, 6H), 7.51-7.30 (m, 9H), 6.10 (s, 1H), 4.95 (s, 1H), 4.92 (s, 1H), 4.77 (br s, 2H), 4.73 (d, *J =* 2.7 Hz, 2H), 4.50 (s, 1H), 4.14 (d, *J =* 12.0 Hz, 1H), 4.07 (s, 3H), 4.02 (d, *J =* 12.0 Hz, 1H), 1.10 (s, 9H); ¹³C NMR (CDCl₃, 100 MHz) *δ* 161.60, 159.29, 152.25, 136.27, 135.67, 135.54, 133.60, 133.16, 133.05, 132.09, 132.07, 130.16, 128.60, 128.03, 127.97, 127.86, 127.73, 126.88, 126.49, 126.42, 125.43, 116.04, 115.82, 88.54, 86.26, 78.19, 77.97, 72.96, 71.05, 58.73, 54.02, 26.69, 19.20; ESI-MS (m/z) 713.40 [M + H]⁺

### Synthesis of R-6'-CN-LNA phosphoramidite monomers

### Synthesis of R-6'-CN-LNA-T phosphoramidite monomer

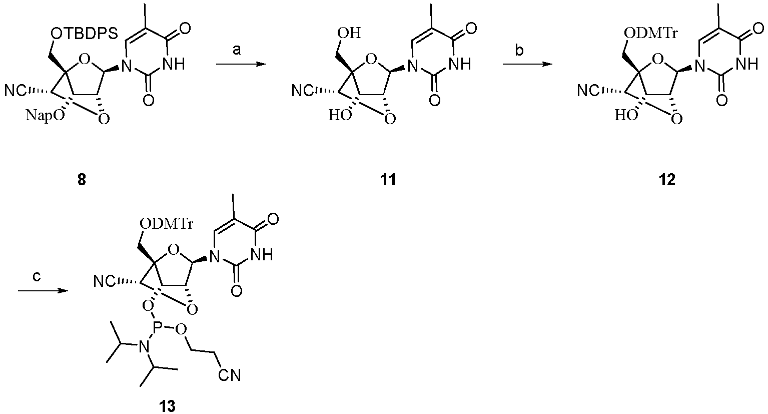

The reaction conditions were: (a) (1) DDQ, DCM-H₂O (20/1), room temperature, 24 h; (2) Et₃N, Et₃N·3HF, THF, room temperature, 9 h; (b) DMTrCl, pyridine, room temperature, 12 h; (c) 2-cyanoethyl *N*,*N*,*N*',*N*'-tetraisopropylphosphorodiamidite, 1*H*-tetrazole, room temperature, 5 h.

11
DDQ (420 mg, 1.86 mmol) was added to the nucleoside derivative 8 (500 mg, 0.74 mmol) in 10.5 mL of DCM-water (20:1) mixture at room temperature, and reacted at room temperature for 24 h, the reaction was monitored by TLC (DCM/methanol=10/1). The reaction solution was subjected to vacuum concentration, extracted with ethyl acetate, The extract was washed with saturated sodium sulfite solution, sodium bicarbonate solution, and saturated saline solution in sequence, dried over anhydrous sodium sulfate and filtered, and the obtained filtrate was concentrated. The obtained residue was dissolved in 10 mL of THF, to which triethylamine (260 µL, 1.86 mmol) and triethylamine trihydrofuric acid (363 µL, 2.23 mmol) were added successively, and the reaction was conducted at room temperature for 9 h, and the reaction was monitored by TLC (DCM/methanol=10/1). 600 mg of sodium bicarbonate solid was added into the reaction solution and stirred until no bubbles were generated, the reaction solution was subjected to vacuum concentration, and directly purified by flash column (gradient elution: methanol/DCM=0%-20%) to obtain 310 mg of colorless amorphous solid 11, with a yield of 46%. ¹H NMR (CD₃OD, 400 MHz) *δ* 7.70 (s, 1H), 5.69 (s, 1H), 4.93 (s, 1H), 4.46 (s, 1H), 4.27 (s, 1H), 3.96 (s, 2H), 1.89 (s, 3H); ¹³C NMR (CD₃OD, 100 MHz) *δ* 164.99, 150.36, 134.88, 116.13, 109.72, 89.51, 86.69, 80.73, 69.97, 69.38, 55.23, 11.26; ESI-MS (m/z) 296.13 [M + H]⁺.

12
4,4'-dimethoxytrityl chloride (13.8 g, 40.6 mmol) was added into 50 mL of pyridine solution containing the CN-modified locked nucleoside derivative 11 (8.0 g, 27.1 mmol), stirred to allow reaction at room temperature for 12 h, and the reaction was monitored by TLC (DCM/methanol=10/1). The reaction was quenched with 50 mL of methanol, the obtained solution was vacuum concentrated, the residue was diluted with ethyl acetate, washed with water and saturated saline solution in sequence, dried over Na₂SO₄, the obtained filtrate was concentrated, and purified by flash column (gradient elution: methanol/DCM=0%-10%) to obtain 13.53 g of 5'-*O*-DMTr-protected nucleoside 12 as a light yellow powder, with a yield of 84%. ¹H NMR (400 MHz, DMSO-*d*₆) *δ* 11.48 (s, 1H), 7.47-6.91 (m, 14H), 6.27 (d, J = 4.0 Hz, 1H), 5.63 (s, 1H), 5.01 (s, 1H), 4.52 (s, 1H), 3.42 (d, J = 4.4 Hz, 1H), 3.74 (s, 6H), 3.65 (d, J = 11.2 Hz, 1H), 3.36 (d, J = 11.2 Hz, 1H), 1.60 (d, J = 0.8 Hz, 3H); ¹³C NMR (100 MHz, DMSO-*d*₆) *δ* 163.81, 158.27, 149.91, 144.50, 135.07, 134.84, 134.00, 129.91, 129.84, 128.00, 127.71, 126.94, 117.41, 113.34, 109.01, 87.99, 86.38, 86.35, 80.37, 70.52, 70.33, 58.47, 55.09, 12.35; ESI-MS (m/z) 620.20 [M + Na]⁺.

13
The nucleoside 12 (4.0 g, 6.7 mmol) and 2-cyanoethyl *N*,*N*,*N'*,*N'*-tetraisopropylphosphorodiamidite (3.0 g, 10.0 mmol) in 40 mL of DCM solution was added with 1*H*-tetrazole (376 mg, 5.36 mmol), the mixture was reacted at room temperature for 5 h, and the reaction was monitored by TLC (ethyl acetate/DCM=5/1). The saturated NaHCO₃ solution was added into the obtained reaction solution, extracted with DCM. The extract was washed with saturated saline solution, dried with MgSO₄ and concentrated, then the concentrated solution was purified by flash column (gradient elution: ethyl acetate/DCM=0%-20%) to obtain 4.4 g of phosphoramidite 13 as a white foamy solid, with a yield of 82%. ³¹P NMR (152 MHz, DMSO-*d*₆) *δ* 148.80, 148.75; ESI-MS (m/z) 798.32 [M + H]⁺.

### Synthesis of R-6'-CN-LNA-A phosphoramidite monomer

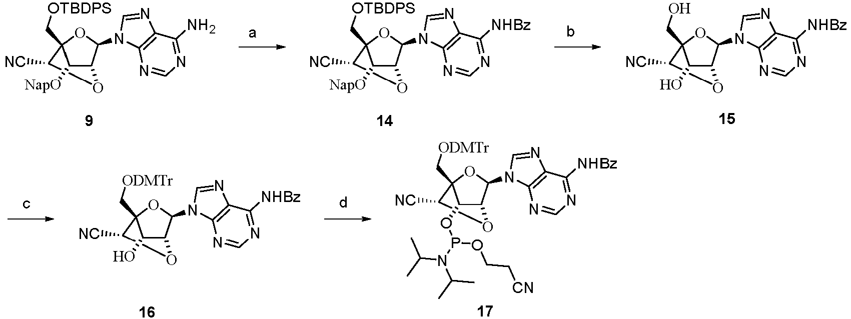

The reaction conditions were: (a) BzCl, pyridine, room temperature, 6 h; (b) (1) DDQ, DCM-H₂O (20/1), room temperature, 24 h; (2) Et₃N, Et₃N·3HF, THF, room temperature 12 h; (b) DMTrCl, pyridine, room temperature, 12 h; (c) 2-cyanoethyl *N*,*N*,*N'*,*N'*-tetraisopropylphosphorodiamidite, 1*H*-tetrazole, room temperature, 8 h.

14
At room temperature, benzoyl chloride (1.52 mL, 13.2 mmol) was added dropwise into 20 mL pyridine solution containing the nucleoside 9 (3.0 g, 4.4 mmol), the reaction was conducted at room temperature for 6 h, then monitored by TLC (DCM/ethyl acetate=10/1). 15% NaOH solution was added dropwise into the reaction solution to adjust a pH value to 8-9, and reacted at room temperature for 4 h. The reaction solution was diluted with ethyl acetate, washed successively with water, 1 N HCl solution, and saturated saline solution, dried over anhydrous sodium sulfate and filtered, the obtained filtrate was concentrated and purified by flash column (gradient elution: ethyl acetate/DCM=0%-60 %) to obtain 1.45 g of benzoyl-protected nucleoside 14 as a light yellow solid, with a yield of 42%. ¹H NMR (CDCl₃, 400 MHz) *δ* 8.62 (s, 1H), 8.20 (s, 1H), 8.07 (d, *J =* 7.0 Hz, 2H), 7.82-7.75 (m, 2H), 7.69-7.32 (m, 18H), 6.25 (s, 1H), 5.11 (s, 1H), 4.98 (s, 1H), 4.77 (s, 2H), 4.51 (d, *J* = 5.8 Hz, 1H), 4.16 (d, *J* = 12.5 Hz, 1H), 4.04 (d, *J* = 12.5 Hz, 1H), 1.07 (s, 9H); ¹³C NMR (CDCl₃, 100 MHz) *δ* 164.60, 152.64, 150.42, 149.70, 140.19, 135.55, 133.53, 133.42, 133.09, 132.99, 132.12, 132.09, 130.15, 128.94, 128.52, 128.00, 127.92, 127.91, 127.85, 127.69, 126.80, 126.38, 126.28, 125.42, 123.29, 114.42, 90.04, 86.43, 78.19, 77.54, 72.97, 70.17, 59.42, 29.70, 26.70, 19.26; ESI-MS (m/z) 787.32 [M + H]⁺.

15
DDQ (1.03 g, 3.81 mmol) was added into 21 mL of DCM-water (20:1) mixture containing the nucleoside derivative 14 (1.2 g, 1.52 mmol) at room temperature, and the mixture was reacted at room temperature for 24 h, the reaction was monitered by TLC (DCM/methanol=10/1). The reaction solution was subjected to vacuum concentration, extracted with ethyl acetate, the extract was washed with saturated sodium sulfite solution, sodium bicarbonate solution, and saturated saline solution in sequence, dried over anhydrous sodium sulfate and filtered, then the obtained filtrate was concentrated. The obtained residue was dissolved in 15 mL of THF, to which triethylamine (530 µL, 3.81 mmol) and triethylamine trihydrofuric acid (743 µL, 4.56 mmol) were added successively, and the resulting mixture was reacted at room temperature for 12 h, and the reaction was monitored by TLC (DCM/methanol=10/1). The reaction solution was added with 1.15 g of sodium bicarbonate solid and stirred until no bubbles were generated, then the reaction solution was subjected to vacuum concentration, and directly purified by flash column (gradient elution: DCM/methanol=0%-15%) to obtain 400 mg of CN-modified locked nucleoside 15, with a yield of 64%. ¹H NMR (CD₃OD, 400 MHz) *δ* 8.72 (s, 1H), 8.55 (s, 1H), 8.07 (d, *J =* 4.2 Hz, 2H), 7.66 (t, *J* = 4.2 Hz, 1H), 7.57 (t, *J =* 4.2 Hz, 2H), 6.30 (s, 1H), 5.05 (s, 1H), 4.88 (s, 1H), 4.64 (s, 1H), 4.02 (s, 2H); ¹³C NMR (CD₃OD, 100 MHz) *δ* 166.76, 152.06, 151.09, 149.81, 141.56, 132.46, 132.57, 128.38, 128.06, 123.91, 116.25, 89.47, 86.11, 80.84, 70.77, 70.43, 55.86; ESI-MS (m/z) 409.15 [M + H]⁺.

16
At room temperature, 4,4'-dimethoxytrityl chloride (481 mg, 1.42 mmol) was added into 5 mL pyridine solution containing the CN-modified locked nucleoside derivative 15 (300 mg, 0.75 mmol), the mixture was stirred to allow reaction at room temperature for 12 h, and the reaction was monitored by TLC (DCM/methanol=10/1). The reaction was quenched with 1 mL of methanol, and subjected to vacuum concentration, the residue was diluted with ethyl acetate, the diluent was washed with water and saturated saline solution in sequence, dried over Na₂SO₄, concentrated, and purified by flash column (gradient elution: ethyl acetate/DCM containing 5% methanol=0%-45%) to obtain 500 mg of 5'-*O*-DMTr-protected CN-modified locked nucleoside 16 as a light yellow solid, with a yield of 94%. ¹H NMR (DMSO-*d*₆, 400 MHz) *δ* 12.26 (s, 1H), 8.79 (s, 1H), 8.59 (s, 1H), 8.06 (d, *J =* 8.4 Hz, 2H), 7.67 (t, *J =* 7.4 Hz, 1H), 7.58 (t, *J =* 7.8 Hz, 2H), 7.46 (d, *J =* 7.4 Hz, 1H), 7.33-7.20 (m, 7H), 6.89 (d, *J* = 8.4 Hz, 4H), 6.33 (s, 1H), 6.30 (d, *J* = 4.3 Hz, 1H), 5.12 (s, 1H), 4.95 (s, 1H), 4.83 (d, *J =* 4.2 Hz, 1H), 3.73 (s, 6H), 3.68 (d, *J =* 11.2 Hz, 1H), 3.40 (d, *J =* 11.2 Hz, 1H); ¹³C NMR (DMSO-*d*₆, 100 MHz) *δ* 166.09, 158.65, 152.31, 152.02, 151.00, 144.95, 142.63, 135.66, 135.42, 133.77, 132.98, 130.34, 130.28, 128.98, 128.97, 128.33, 128.96, 127.26, 126.02, 118.26, 113.71, 88.34, 86.83, 85.69, 80.94, 72.59, 71.24, 60.23, 55.51; ESI-MS (m/z) 711.36 [M + H]⁺.

17
At room temperature, the nucleoside 16 (450 mg, 0.63 mmol) and 2-cyanoethyl *N*,*N*,*N'*,*N'*-tetraisopropylphosphorodiamidite (382 mg, 1.27 mmol) in 10 mL of DCM solution was added with 1*H*-tetrazole (35 mg, 0.51 mmol), reacted at room temperature for 8 h, and the reaction was monitored by TLC (DCM/ethyl acetate=5/2). The reaction solution was directly purified by flash column (gradient elution: ethyl acetate/DCM=0%-40%) to obtain 480 mg of phosphoramidite 17 as a slightly yellow foamy solid, with a yield of 83%. ³¹P NMR (152 MHz, DMSO-*d*₆) *δ* 149.19, 149.04; ESI-MS (m/z) 909.38 [M - H]⁻.

### Synthesis of R-6'-CN-LNA-G phosphoramidite monomer

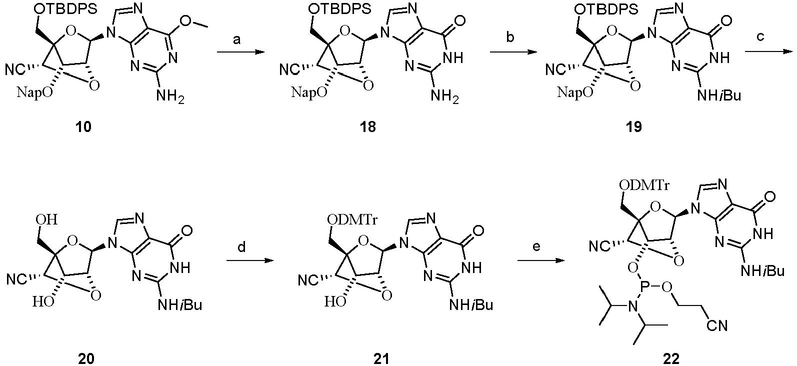

The reaction conditions were: (a) 2 N HCl, THF-CH₃OH (1/1), 60°C, 12 h; (b) isobutyryl chloride, Et₃N, DMAP, 1,4-dioxane, 100°C, 24 h; (c) (1) DDQ, DCM-H₂O (20/1), room temperature, 24 h; (2) Et₃N, Et₃N·3HF, THF, room temperature, 12 h; (d) DMTrCl, pyridine, room temperature, 12 h; (e) 2-Cyanoethyl *N*,*N*,*N'*,*N'*-tetraisopropylphosphorodiamidite, 1*H*-tetrazole, room temperature, 12 h.

18
17.5 mL of 2 N hydrochloric acid solution was added into 30 mL of THF-methanol solution (1/1 v/v) containing CN-modified locked nucleoside 10 (2.5 g, 3.5 mmol), and reacted at 60°C for 12 h to precipitate a white solid, the reaction was monitored by TLC (DCM/ethyl acetate=2/1). The insoluble white solid was filtered out, the filtrate was extracted with ethyl acetate, washed with water and saturated saline solution in sequence, dried over anhydrous sodium sulfate, filtered, then the obtained filtrate was concentrated, the filtrates were combined and purified by flash column (gradient elution: ethyl acetate/DCM containing 5% methanol=0%-60%), thus obtaining 1.4 g of 6-demethylated nucleoside 18 as a white solid, with a yield of 57%. ¹H NMR (DMSO-*d*₆, 400 MHz) *δ* 10.71 (s, 1H), 7.92-7.81 (m, 5H), 7.66 (m, 4H), 7.54-7.49 (m, 2H), 7.47-7.36 (m, 7H), 6.66 (br s, 2H), 6.04 (s, 1H), 5.29 (s, 1H), 5.13 (s, 1H), 4.92 (d, *J =* 12.0 Hz, 1H), 4.84 (d, *J =* 12.0 Hz, 1H), 4.78 (s, 1H), 4.21 (d, *J =* 12.2 Hz, 1H), 4.01 (d, *J =* 12.2 Hz, 1H), 0.94 (s, 9H); ¹³C NMR (DMSO-*d*₆, 100 MHz) *δ* 157.13, 154.49, 151.10, 135.62, 135.60, 135.29, 134.48, 133.17, 133.01, 132.46, 132.46, 130.52, 128.44, 128.15, 128.07, 126.75, 126.65, 126.56, 126.17, 117.70, 117.02, 88.24, 84.89, 78.86, 78.48, 72.02, 71.23, 59.76, 26.80, 19.20; ESI-MS (m/z) 699.39 [M + H]⁺, 721.48 [M + Na]⁺.

19
12 mL of 1,4-dioxane solution containing guanosine 18 (1.2 g, 1.72 mmol) was added with DMAP (105 mg, 0.86 mmol) and triethylamine (718 µL, 5.16 mmol), then added with isobutyryl chloride (550 µL, 5.16 mmol), reacted at 100°C for 24 h, and the reaction was monitored by TLC (DCM/methanol=10/1). After stopping the heating and allowing the reaction solution to cool to room temperature, 1 mL of methanol was added to quench the reaction, the product was extracted with ethyl acetate, the extract was washed with water and saturated saline solution in sequence, dried over anhydrous sodium sulfate and filtered, the obtained filtrate was concentrated, and purified by flash column (gradient elution: ethyl acetate/DCM=0%-80%) to obtain 1.1 g of isobutyryl-protected guanosine 19 as a slightly yellow foamy solid, with a yield of 83%. ¹H NMR (CDCl₃, 400 MHz) *δ* 12.00 (s, 1H), 9.02 (s, 1H), 7.78-7.74 (m, 3H), 7.69-7.63 (m, 5H), 7.52 (s, 1H), 7.47-7.28 (m, 9H), 5.86 (s, 1H), 4.87 (s, 1H), 4.76 (d, *J =* 11.6 Hz, 1H), 4.66 (d, *J =* 11.6 Hz, 1H), 4.47 (s, 1H), 4.33 (s, 1H), 4.13 (d, *J =* 12.0 Hz, 1H), 4.02 (d, *J =* 12.0 Hz, 1H), 2.70-2.66 (m, 1H), 1.29-1.24 (m, 6H), 1.08 (s, 9H); ¹³C NMR (CDCl₃, 100 MHz) *δ* 178.83, 155.24, 147.84, 146.82, 135.64, 135.49, 135.11, 133.37, 133.11, 132.95, 131.99, 131.93, 130.25, 130.22, 128.67, 128.07, 128.01, 127.74, 127.59, 127.25, 126.61, 126.53, 125.70, 131.62, 115.87, 86.71, 86.12, 78.40, 73.33, 71.03, 58.74, 36.40, 26.71, 19.78, 19.21, 18.99; ESI-MS (m/z) 870.60 [M + Et₃N + H]⁻.

20
DDQ (702 mg, 3.64 mmol) was added into 10.5 mL of DCM-water (20:1) mixture containing the nucleoside derivative 19 (800 mg, 1.04 mmol) at room temperature, and reacted at room temperature for 24 h, the reaction was monitered by TLC (DCM/methanol=10/1). The reaction solution was subjected to vacuum concentration, extracted with ethyl acetate, the extract was washed with saturated sodium sulfite solution, sodium bicarbonate solution, and saturated saline solution in sequence, dried over anhydrous sodium sulfate, filtered, and the obtained filtrate was concentrated. The obtained residue was dissolved in 10 mL of THF, to which triethylamine (363 µL, 2.60 mmol) and triethylamine trihydrofuric acid (509 µL, 3.12 mmol) were added successively, and the mixture was reacted at room temperature for 12 h, and the reaction was monitored by TLC (DCM/methanol=10/1). 786 mg of sodium bicarbonate solid was added into the reaction solution and stirred until no bubbles were generated, the reaction solution was subjected to vacuum concentration, and directly purified by flash column (gradient elution: DCM/methanol=0%-25%) to obtain 230 mg of CN-modified locked nucleoside 20, with a yield of 57%. ¹H NMR (CD₃OD, 400 MHz) *δ* 8.09 (s, 1H), 6.08 (s, 1H), 5.01 (s, 1H), 4.73 (s, 1H), 4.54 (s, 1H), 3.99 (s, 2H), 2.72-2.68 (m, 1H), 1.23-1.21 (m, 6H); ¹³C NMR (CD₃OD, 100 MHz) *δ* 180.35, 148.67, 148.20, 136.57, 120.03, 116.22, 89.37, 85.62, 80.91, 70.56, 70.35, 55.80, 46.48, 35.58, 17.92, 17.90, 7.84; ESI-MS (m/z) 391.08 [M + H]⁺, 413.05 [M + Na]⁺.

21
At room temperature, 4,4'-dimethoxytrityl chloride (260 mg, 0.77 mmol) was added into 5 mL pyridine solution containing the CN-modified locked nucleoside derivative 20 (150 mg, 0.384 mmol), the resulting mixture was stirred to allow reaction at room temperature for 12 h, and the reaction was monitored by TLC (DCM/methanol=5/1). The reaction was quenched with 2 mL of methanol and was subjected to vacuum concentration, the residue was diluted with ethyl acetate, the diluent was washed with water and saturated saline solution in sequence, dried over Na₂SO₄, and concentrated, then the concentrated solution was purified by flash column (gradient elution: ethyl acetate/DCM containing 5% methanol=0%-40%) to obtain 270 mg of 5'-*O*-DMTr-protected CN-modified locked nucleoside 21 as a light yellow powder, with a yield of 100%. ¹H NMR (DMSO-*d*₆, 400 MHz) *δ* 12.14 (s, 1H), 11.85 (s, 1H), 8.11 (s, 1H), 7.46 (d, *J* = 7.4 Hz, 2H), 7.33-7.21 (m, 7H), 6.90 (dd, *J* = 8.8, 1.4 Hz, 4H), 6.26 (d, *J* = 4.2 Hz, 1H), 6.08 (s, 1H), 5.08 (s, 1H), 4.77 (s, 1H), 4.57 (d, *J =* 4.2 Hz, 1H), 3.74 (s, 6H), 3.68 (d, *J =* 11.2 Hz, 1H), 3.39 (d, *J =* 11.2 Hz, 1H), 2.82-2.75 (m, 1H), 1.14 (d, *J =* 6.8 Hz, 6H); ¹³C NMR (DMSO-*d*₆, 100 MHz) *δ* 180.73, 158.66, 155.22, 148.89, 148.58, 144.97, 136.87, 135.59, 135.40, 130.34, 130.31, 128.33, 128.21, 127.27, 120.65, 118.31, 113.71, 88.30, 86.87, 84.93, 80.95, 72.28, 71.18, 60.22, 55.51, 35.27, 19.33, 19.28; ESI-MS (m/z) 691.36 [M - H]⁻.

22
At room temperature, the nucleoside 21 (180 mg, 0.26 mmol) and 2-cyanoethyl *N*,*N*,*N'*,*N'*-tetraisopropylphosphorodiamidite (157 mg, 0.52 mmol) in 10 mL of DCM solution was added with 1*H*-tetrazole (15 mg, 0.21 mmol), reacted at room temperature for 12 h, and the reaction was monitored by TLC (DCM/ethyl acetate=2/1). The reaction solution was directly purified by flash column (gradient elution: ethyl acetate/DCM=0%-40%) to obtain 180 mg of phosphoramidite 22 as a slightly yellow waxy solid, with a yield of 78%. ³¹P NMR (152 MHz, DMSO-*d*₆) *δ* 149.26, 149.23; ESI-MS (m/z) 893.40 [M + H]⁺.

### Synthesis of R-6'-CN-LNA-^{m}C phosphoramidite monomer

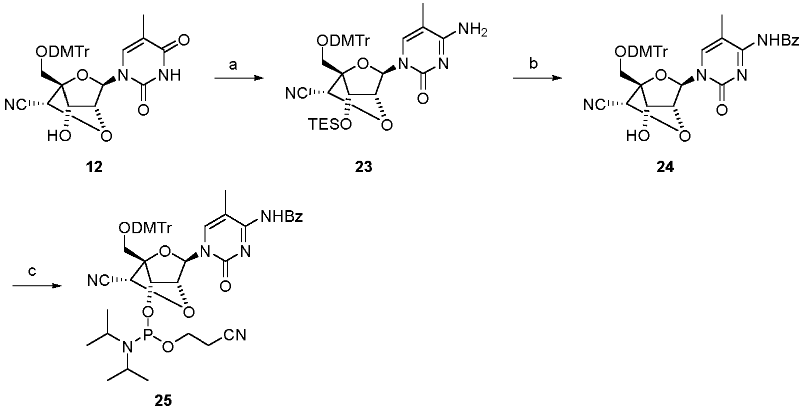

The reaction conditions were: (a) (1) TESCl, Et₃N, acetonitrile, 0°C to room temperature, 3 h; (2) 1,2,4-triazole, POCl₃, acetonitrile, 0°C to room temperature, 2 h; (3) NH₄OH, 1,4-dioxane, room temperature, 2 h; (b) (1) Bz₂O, acetonitrile, room temperature, 20 h; (2) TBAF, THF, room temperature, 12 h; (c) 2-Cyanoethyl *N*,*N*,*N'*,*N'*-tetraisopropylphosphorodiamidite, 1H-tetrazole, DCM, room temperature, 5 h.

23
At 0°C, triethylsilyl chloride (7.0 mL, 41.8 mmol) was added to the acetonitrile solution (100 mL) containing nucleotide 12 (5.0 g, 8.37 mmol) and triethylamine (23.3 mL, 167 mmol), and reacted at room temperature for 3 h. The reaction solution was added with 1,2,4-triazole (8.67 g, 125.55 mmol), stirred for 10 min, and cooled to 0°C, POCl₃ (2.34 mL, 25.11 mmol) was added dropwise to the reaction solution, reacted at room temperature for 2 h, and the reaction was monitored by TLC (DCM/methanol=10/1). The reaction solution was poured into 300 mL of ice water, extracted with ethyl acetate, the extract was washed with water, saturated sodium bicarbonate solution, and saturated saline solution in sequence, dried over anhydrous Na₂SO₄, filtered, and the obtained filtrate was concentrated. The residue was dissolved in 60 mL of 1,4-dioxane, added with 25% ammonia water (6.25 mL, 92 mmol), and stirred at room temperature for 2 h. The reaction solution was subjected to vacuum concentration, diluted with ethyl acetate, the resulting organic layer was washed with water and saturated saline solution, dried over anhydrous Na₂SO₄, filtered, and the obtained filtrate was concentrated, and purified by flash column (gradient elution: methanol/DCM=0%-15%) to obtain 5.5 g of aminonucleoside 23 as a light yellow solid, with a yield of 92%. ¹H NMR (DMSO-*d*₆, 400 MHz) *δ* 7.52-7.25 (m, 12H), 6.96-6.91 (m, 6H), 5.66 (s, 1H), 4.93 (s, 1H), 4.45 (s, 1H), 4.40 (s, 1H), 3.75 (s, 6H), 3.54 (d, *J =* 11.2 Hz, 1H), 3.41 (d, *J =* 11.2 Hz, 1H), 1.72 (s, 3H), 0.78 (t, *J =* 7.9 Hz, 9H), 0.53-0.41 (m, 6H); ¹³C NMR (DMSO-*d*₆, 100 MHz) *δ* 166.09, 158.81, 158.79, 154.95, 144.78, 136.54, 135.38, 135.17, 130.30, 130.14, 128.43, 128.07, 127.46, 117.58, 113.81, 113.77, 101.92, 88.29, 87.59, 86.82, 80.77, 71.60, 71.09, 58.50, 55.55, 14.01, 6.78, 4.42; ESI-MS (m/z) 733.31 [M+ Na]⁺.

24
An acetonitrile (60 mL) solution containing the aminonucleoside 23 (5.5 g, 7.73 mmol) was added with benzoic anhydride 23 (3.85 g, 12 mmol), the reaction was conducted by stirring at room temperature for 20 h, and the reaction was quenched with 5 mL of water. TBAF (4.38 g, 19.3 mmol) was added to the reaction mixture, stirred at room temperature for 12 h, 15% NaOH was added to adjust the pH to 10, and stirring was continued for 3 h. The reaction solution was diluted with ethyl acetate, washed with water, and then the organic layer was washed with water and saturated saline solution, dried over anhydrous Na₂SO₄, concentrated, and purified by flash column (gradient elution: ethyl acetate/DCM=0%-40%) to obtain 3.1 g of 5'-*O*-DMTr-protected nucleoside 24 as a white foamy solid, with a yield of 57%. ¹H NMR (DMSO-*d*₆, 400 MHz) *δ* 13.06 (br s, 1H), 8.19 (s, 2H), 7.69-7.25 (m, 13H), 6.94 (d, *J =* 8.3 Hz, 4H), 6.32 (s, 1H), 5.70 (s, 1H), 5.06 (s, 1H), 4.53 (s, 1H), 4.38 (d, *J* = 4.1 Hz, 1H), 3.73 (d, *J =* 11.0 Hz, 1H), 3.40 (d, *J =* 11.0 Hz, 1H), 1.84 (s, 3H); ¹³C NMR (DMSO-*d*₆, 100 MHz) *δ* 178.64, 159.43, 158.75, 147.49, 144.93, 137.11, 135.53, 135.32, 133.07, 130.37, 130.32, 129.85, 128.83, 128.46, 128.21, 127.41, 117.77, 113.82, 110.12, 88.75, 87.28, 80.55, 71.06, 70.70, 58.88, 55.56, 13.66; ESI-MS (m/z) 701.32 [M + H]⁺.

25
The nucleoside 24 (1.7 g, 2.42 mmol) and 2-cyanoethyl *N*,*N*,*N'*,*N'*-tetraisopropylphosphorodiamidite (1.1 g, 3.63 mmol) in 20 mL of anhydrous DCM were added with 1H-tetrazole (135 mg, 1.94 mmol), and reacted at room temperature for 5 h. The saturated NaHCO₃ solution was added into the obtained reaction solution, extracted with DCM, the resulting organic layer was washed with saturated saline solution, dried with MgSO₄, concentrated, and the purified by flash column (gradient elution: ethyl acetate/DCM=0%-20%) to obtain 1.83 g of phosphoramidite 25 as a white foamy solid, with a yield of 84%. ³¹P NMR (152MHz, DMSO-*d*₆) *δ*149.20, 148.47; ESI-MS (m/z) 901.46 [M + H]⁺.

### Part II: Synthesis of S-6'-CN-LNA

### Synthesis of S-6'-Allyl-LNA

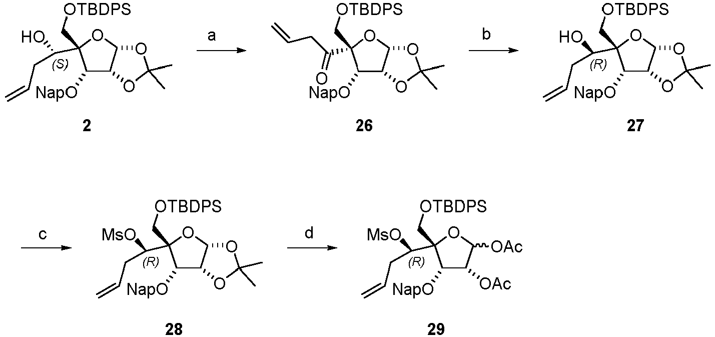

The reaction conditions were: (a) 2-iodoacylbenzoic acid, acetonitrile, refluxing, 11 h; (b) NaBH₄, LiCl, MeOH, -40°C, 8 h; (c) methanesulfonyl chloride, DMAP, pyridine, room temperature, 32 h; (d) (1) FeCl₃·6H₂O, DCM, 0°C to room temperature, 3 h; (2) Ac₂O, DMAP, pyridine, DCM, room temperature, 4 h.

26
The compound 2 (105 g, 0.164 mol) in 500 mL of acetonitrile was added with 2-iodoacylbenzoic acid (69 g, 0.25 mol), heated to allow refluxing for 6 h, 2-iodoacylbenzoic acid (13.8 g, 0.05 mol) was added again, the refluxing was continued for 5 h, and the reaction was monitored by TLC (petroleum ether/ethyl acetate=5/1). The reaction solution was filtered through diatomaceous earth, and the filtrate was concentrated and purified by flash column (gradient elution: ethyl acetate/petroleum ether=0%-20%) to obtain 93 g of colorless thick compound 26, with a yield of 89%. ¹H NMR (CDCl₃, 400 MHz) *δ* 7.85-7.70 (m, 4H), 7.60-7.53 (m, 4H), 7.48-7.45 (m, 2H), 7.40-7.27 (m, 7H), 6.08 (d, *J =* 4.0 Hz, 1H), 5.98-5.83 (m, 1H), 5.13-5.01 (m, 2H), 4.90 (d, *J =* 12.0 Hz, 1H), 4.86 (t, *J =* 8.0, 4.0 Hz, 1H), 4.63 (d, *J =* 12.0 Hz, 1H), 4.23 (d, *J =* 8.0 Hz, 1H), 3.92 (d, *J* = 12.0 Hz, 1H), 3.72 (d, *J =* 12.0 Hz, 1H), 3.66-3.55 (m, 2H), 1.61 (s, 3H), 1.43 (s, 3H), 0.98 (s, 9H); ¹³C NMR (CDCl₃, 100 MHz) *δ* 207.44, 135.53, 134.92, 134.83, 133.23, 133.06, 132. 49, 132.46, 130.73, 129.99, 129.91, 128.12, 127.97, 127.87, 127.75, 127.71, 126.57, 126.09, 125.93, 125.82, 118.44, 114.93, 106.94, 96.44, 81.14, 80.15, 73.55, 44.95, 27.61, 26.81, 26.68, 19.13; ESI-MS (m/z) 635.40 [M - H]⁻.

27
At room temperature, lithium chloride (18 g, 0.424 mol) in 200 mL of methanol was added into 400 mL of THF containing the compound 26 (108 g, 0.17 mol), the obtained reaction solution was cooled to -40°C and added with sodium borohydride (4.3 g, 0.113 mol), after 4 hours of reaction, the reaction solution was added with sodium borohydride (4.3 g, 0.113 mol), reacted for 4 h, and the reaction was monitored by TLC (petroleum ether/ethyl acetate=5/1). The reaction solution was poured into a beaker and quenched with saturated ammonium chloride solution (100 mL), the resulting mixture was stirred until no bubbles were generated and extracted with ethyl acetate (1 L), the extract was washed with water and saturated saline solution in sequence, dried over anhydrous sodium sulfate, filtered, the filtrate was concentrated and purified by flash column (gradient elution: ethyl acetate/petroleum ether=0%-20%) to obtain 90 g of colorless thick compound 27, with a yield of 83%. ¹H NMR (CDCl₃, 400 MHz) *δ* 7.86-7.79 (m, 4H), 7.59-7.57 (m, 2H), 7.51-7.46 (m, 5H), 7.41-7.28 (m, 6H), 5.91-5.83 (m, 2H), 5.01-4.97 (m, 3H), 4.78 (dd, *J =* 5.3, 3.9 Hz, 1H), 4.65 (d, *J =* 12.0 Hz, 1H), 4.51 (d, *J* = 5.4 Hz, 1H), 4.19 (dd, *J* = 10.0, 2.1 Hz, 1H), 3.76 (d, *J =* 10.7 Hz, 1H), 3.54 (d, *J =* 10.7 Hz, 1H), 2.36-2.31 (m, 1H), 2.09-2.02 (m, 1H), 1.67 (s, 3H), 1.39 (s, 3H), 0.91 (s, 9H); ¹³C NMR (CDCl₃, 100 MHz) *δ* 136.40, 135.53, 135.51, 134.80, 133.22, 132.16, 132.88, 132.74, 129.85, 129.78, 128.41, 127.94, 127.78, 126.85, 126.29, 126.14, 125.67, 161.13, 114.38, 104.73, 90.15, 79.74, 78.12, 72.80, 71.04, 64.51, 35.91, 26.98, 26.81, 26.75, 19.11; ESI-MS (m/z) 637.41 [M - H]⁻.

28
The compound 27 (90 g, 0.14 mol) and DMAP (1.71 g, 14 mmol) were dissolved in 400 mL of pyridine, methanesulfonyl chloride (16.4 mL, 0.21 mol) was added dropwise, and the mixture was reacted at room temperature for 20 h, methanesulfonyl chloride (5.5 mL, 0.07 mol) was added, and the reaction was continued for 12 h, the reaction was monitored by TLC (petroleum ether/ethyl acetate=5/1). 50 mL of methanol was added to the reaction solution to quench the reaction, vacuum concentration was conducted to remove excess pyridine, the residue was diluted with ethyl acetate, washed with water, 1 N HCl solution, and saturated saline solution in sequence, dried over anhydrous sodium sulfate, filtered, and the filtrate was concentrated to obtain 100 g of methanesulfonyl compound 28 as a slightly yellow sticky substance, with a yield of 100%. ¹H NMR (DMSO-*d*₆, 400 MHz) *δ* 7.95-7.87 (m, 4H), 7.59-7.38 (m, 13H), 5.85 (d, *J =* 4.0 Hz, 1H), 5.68-5.58 (m, 1H), 5.13 (dd, *J =* 9.8, 1.4 Hz, 1H), 5.07 (t, *J =* 5.0 Hz, 1H), 4.98-4.91 (m, 3H), 4.69 (d, *J =* 11.8 Hz, 1H), 4.47 (d, *J =* 5.4 Hz, 1H), 3.76 (d, *J =* 10.8 Hz, 1H), 3.71 (d, *J =* 10.8 Hz, 1H), 3.08 (s, 3H), 2.83-2.78 (m, 1H), 2.16-2.08 (m, 1H), 1.58 (s, 3H), 1.37 (s, 3H), 0.88 (s, 9H); ¹³C NMR (CDCl₃, 100 MHz) *δ* 135.54, 135.59, 135.30, 134.36, 133.24, 133.15, 132.72, 132.68, 130.50, 130.47, 128.58, 128.43, 128.35, 128.21, 127.32, 126.79, 126.61, 126.48, 118.25, 124.07, 105.02, 88.46, 85.65, 79.89, 77.62, 72.51, 63.50, 36.01, 27.06, 26.95, 26.82, 26.58, 19.07; ESI-MS (m/z) 734.39 [M + NH₄]⁺, 739.34 [M + Na]⁺.

**29**
1,000 mL of DCM solution containing the compound 28 (100 g, 0.14 mol) was cooled to 0°C and added with ferric chloride hexahydrate (15.1 g, 56 mmol), the obtained mixture was reacted at room temperature for 3 h, the reaction was monitored by TLC (petroleum ether/ethyl acetate=4/1). The reaction solution was poured into 2 L of water, and extracted with DCM, the resulting organic layer was washed with water, saturated sodium bicarbonate solution, and saturated saline solution in sequence, dried over anhydrous magnesium sulfate and filtered, the filtrate was subjected to vacuum concentration and dried under vacuum. The obtained thick substance was dissolved in 1 L of DCM, pyridine (113 mL, 1.4 mol), DMAP (2.56 g, 21 mol) were added in sequence, and acetic anhydride (79 mL, 0.84 mol) was added dropwise, the resulting mixture was subjected to reaction at room temperature for 4 h, the reaction was monitored by TLC (petroleum ether/ethyl acetate=4/1). The reaction solution was poured into 2 L of water and extracted with DCM, the resulting organic layer was washed successively with water, 1 N HCl solution, saturated sodium bicarbonate solution, and saturated saline solution, dried over anhydrous magnesium sulfate, filtered, then the obtained filtrate was concentrated, and flash column-purified (gradient elution: petroleum ether/ethyl acetate=0%-60%) to obtain 95 g of the epimer mixture of a diacetyl compound 29 as a colorless thick substance, with a yield of 89%. ESI-MS (m/z) 778.39 [M + NH₄]⁺, 783.44 [M + Na]⁺.

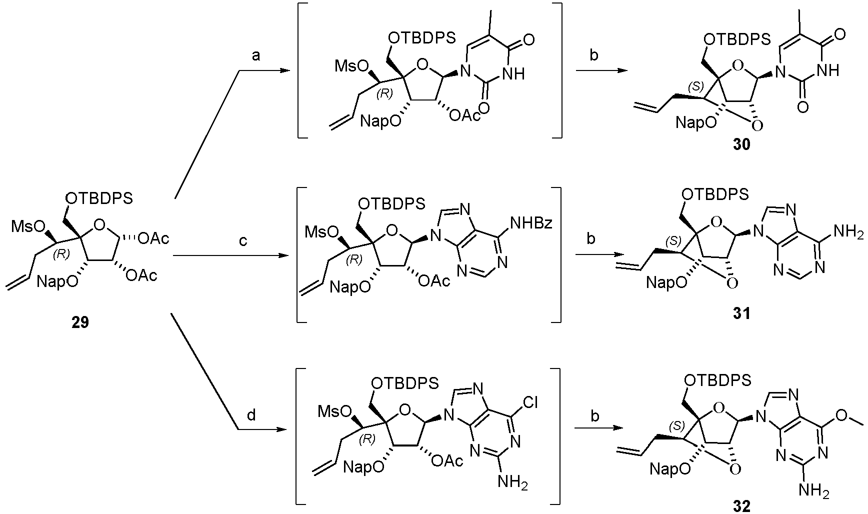

The reaction conditions were: (a) thymine, BSA, TMSOTf, acetonitrile, 80°C, 3 h; (b) K₂CO₃, MeOH, room temperature, 16 h; (c) N6-benzoyladenine, BSA, TMSOTf, toluene, 100°C, 3 h; (d) 6-chloroguanine, BSA, TMSOTf, toluene, 100°C, 3 h.

30
BSA(29 mL, 120 mmol) was added into 50 mL acetonitrile suspension containing thymine (7.6 g, 60 mmol), and stirred at room temperature for 20 min until the thymine was dissolved. After the system became clear, 200 mL of acetonitrile solution containing diacetylated sugar 29 (23 g, 30 mmol) was added, after rapid dropwise addition of TMSOTf (8.3 mL, 45 mmol), the obtained mixture was heated to 80°C to allow reaction by stirring for 3 h, the reaction was monitored by TLC (petroleum ether/ethyl acetate=5/1). After stopping heating and allowing the reaction solution to cool to room temperature, the reaction solution was poured into ethyl acetate, added with a semisaturated NaHCO₃ solution (400 mL), and stirred for 20 min. The insoluble solid was filtered off with diatomaceous earth, while the organic layer was separated and then washed with water and saturated saline solution in sequence, dried over anhydrous NaSO₄, filtered, the filtrate was concentrated, and dried under vacuum. The obtained residue was dissolved in 250 mL of methanol, added with K₂CO₃ (12.4 g, 90 mmol), and reacted at room temperature for 16 h, the reaction was monitored by TLC (DCM/ethyl acetate=10/3). After the reaction solution was concentrated, the residue was extracted with ethyl acetate, washed with water and saturated saline solution in sequence, dried over anhydrous NaSO₄, filtered, the filtrate was concentrated, and purified by flash column (gradient elution: ethyl acetate/DCM=0%-25%), such that 15.4 g of an LNA derivative 30 was obtained as a white foamy solid, with a yield of 75%. ¹H NMR (CDCl₃, 400 MHz) *δ* 8.92 (br s, 1H), 7.84-7.64 (m, 8H), 7.51-7.29 (m, 10H), 5.77-5.59 (m, 1H), 5.65 (s, 1H), 5.01-4.96 (m, 2H), 4.84 (d, *J =* 11.4 Hz, 1H), 4.73 (s, 1H), 4.67 (d, *J =* 11.4 Hz, 1H), 4.07-3.99 (m, 4H), 2.68-2.60 (m, 1H), 2.26-2.19 (m, 1H), 1.58 (s, 3H), 1.09 (s, 9H); 13C NMR (CDCl3, 100 MHz) *δ* 163.79, 149.75, 135.59, 135.35, 134.36, 134.13, 134.01, 133.14, 133.10, 132.80, 132.34, 130.11, 130.06, 128.45, 127.99, 127.95, 127.87, 127.75, 126.91, 126.45, 126.30, 125.75, 117.38, 110.34, 89.58, 87.12, 84.02, 76.90, 72.53, 59.27, 35.33, 29.72. 26.94, 19.44, 12.15; ESI-MS (m/z) 689.43 [M + H]⁺, 711.34 [M + Na]⁺.

31
BSA (17 mL, 68 mmol) was added into 25 mL toluene suspension containing N6-benzoyladenine (6.1 g, 25.5 mmol), and heated to 60°C for reaction until the N6-benzoyladenine was dissolved. After the system became clear, the reaction solution was cooled to room temperature, 70 mL of toluene solution containing diacetylated sugar 29 (13 g, 17 mmol) was added, after rapid dropwise addition of TMSOTf (4.63 mL, 25.5 mmol), the obtained mixture was heated to 100°C to allow reaction by stirring for 3 h, the reaction was monitored by TLC (petroleum ether/ethyl acetate=5/1). After stopping heating and allowing the reaction solution to cool to room temperature, the reaction solution was poured into ethyl acetate, added with a semisaturated NaHCO₃ solution (400 mL), and stirred for 20 min. The insoluble solid was filtered off with diatomaceous earth, while the organic layer was separated and then washed by water and saturated saline solution in sequence, dried over anhydrous NaSO₄, filtered, the filtrate was concentrated, and dried under vacuum, and purified by flash column (gradient elution: ethyl acetate/DCM=0%-40%) to obtain 9.1 g of nucleoside product, with a yield of 74%. ESI-MS (m/z) 940.04 [M + H]⁺.

The product (8.5 g, 9 mmol) was dissolved in 90 mL of methanol, added with K₂CO₃ (6.25 g, 45 mmol) was added, and reacted at room temperature for 16 h, the reaction was monitored by TLC (DCM/ethyl acetate=10/3). After the reaction solution was concentrated, the residue was extracted with ethyl acetate, washed with water and saturated saline solution in sequence, dried over anhydrous NaSO₄, filtered, then the filtrate was concentrated and purified by flash column (gradient elution: ethyl acetate/DCM=0%-100%), such that 4.9 g of an LNA derivative 31 was obtained as a white powder, with a yield of 78%. ¹H NMR (CDCl₃, 400 MHz) *δ* 8.27 (s, 1H), 7.98 (s, 1H), 7.81-7.67 (m, 7H), 7.62 (s, 1H), 7.49-7.31 (m, 9H), 6.02 (s, 1H), 5.86-5.76 (m, 3H), 5.06-5.02 (m, 2H), 4.95 (s, 1H), 4.74 (d, *J =* 11.6 Hz, 1H), 4.66 (d, *J =* 11.6 Hz, 1H), 4.27 (s, 1H), 4.18 (dd, *J =* 9.3, 4.6 Hz, 1H), 4.09 (d, *J =* 11.9 Hz, 1H), 4.05 (d, *J =* 11.9 Hz, 1H), 2.77-2.69 (m, 1H), 2.36-2.30 (m, 1H), 1.07 (s, 9H); ¹³C NMR (CDCl₃, 100 MHz) *δ* 155.16, 152.74, 148.84, 138.22, 135.69, 135.63, 134.35, 134.40, 133.10, 133.06, 132.79, 132.62, 129.99, 129.97, 128.36, 127.92, 127.85, 127.82, 127.71, 126.92, 126.32, 126.19, 125.64, 120.14, 117.16, 89.04, 86.48, 84.57, 78.06, 72.72, 59.50, 35.31, 26.78, 19.27; ESI-MS (m/z) 698.36 [M + H]⁺.

32
BSA (13 mL, 52.6 mmol) was added into 25 mL toluene suspension containing 6-chloroguanine (3.34 g, 19.7 mmol), and heated to 60°C for reaction until the 6-chloroguanine was dissolved. After the system became clear, the reaction solution was cooled to room temperature, 75 mL of toluene solution containing diacetylated sugar 29 (10 g, 13.1 mmol) was added, after rapid dropwise addition of TMSOTf (3.6 mL, 19.7 mmol), the obtained mixture was heated to 100°C to allow reaction by stirring for 3 h, the reaction was monitored by TLC (petroleum ether/ethyl acetate=5/1). After stopping heating and allowing the reaction solution to cool to room temperature, the reaction solution was poured into ethyl acetate, added with the semisaturated NaHCO₃ solution (400 mL), and stirred for 20 min. The insoluble solid was filtered off with diatomaceous earth, while the organic layer was separated and then washed by water and saturated saline solution in sequence, dried over anhydrous NaSO₄, filtered, the filtrate was concentrated, and dried under vacuum. The obtained residue was dissolved in 100 mL of methanol, added with K₂CO₃ (7.25 g, 52.6 mmol), and reacted at room temperature for 16 h, the reaction was monitored by TLC (DCM/ethyl acetate=10/3). After the reaction solution was concentrated, the residue was extracted with ethyl acetate, washed with water and saturated saline solution in sequence, dried over anhydrous NaSO₄, filtered, the filtrate was concentrated, and purified by flash column (gradient elution: ethyl acetate/DCM=0%-30%), such that 6.07 g of an LNA derivative 32 was obtained as a white foamy solid, with a yield of 63%. ¹H NMR (CDCl₃, 400 MHz) *δ* 7.82-7.65 (m, 9H), 7.49-7.30 (m, 9H), 5.91 (s, 1H), 5.85-5.76 (m, 1H), 5.06-5.02 (m, 2H), 4.86 (br s, 2H), 4.81 (s, 1H), 4.73 (d, *J* = 11.5 Hz, 1H), 4.64 (d, *J =* 11.5 Hz, 1H), 4.27 (s, 1H), , 4.09-4.01 (m, 5H), 2.76-2.69 (m, 1H), 2.35-2.28 (m, 1H), 1.07 (s, 9H); ¹³C NMR (CDCl₃, 100 MHz) *δ* 161.48, 159.29, 152.47, 135.70, 135.55, 134.47, 124.36, 133.12, 133.04, 132.74, 132.58, 129.98, 129.95, 128.34, 127.91, 127.88, 127.84, 127.70, 126.60, 125.59, 117.15, 116.13, 88.77, 86.19, 84.49, 78.23, 77.15, 72.78, 55.50, 53.90, 35.31, 29.75, 26.76, 19.24; ESI-MS (m/z) 728.39 [M + H]⁺.

### Synthesis of S-6'-CN-LNA

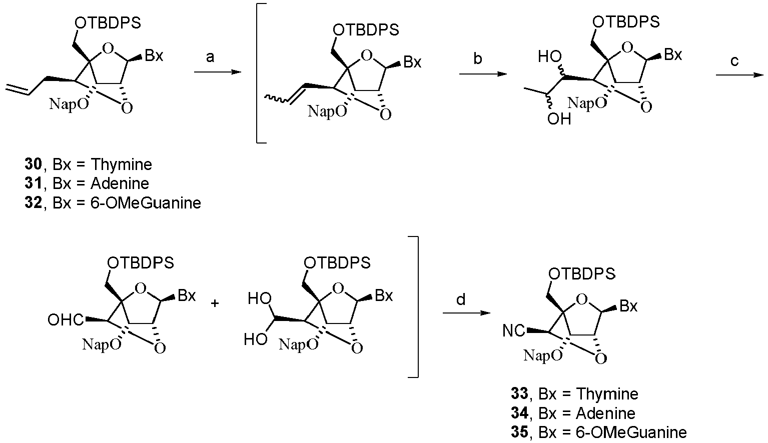

The reaction conditions were: (a) (1) carbonylchlorohydridotris(triphenylphosphine)ruthenium (II), EtOH, 80°C, 12-48 h; (b) potassium osmate dihydrate, 50% NMO, THF t-BuOH, H₂O, 60°C, 8-12 h; (c) NaIO₄, EtOH-THF (2/1 v/v), H₂O, room temperature, 12 h; (d) NH₄OH, I₂, room temperature, THF, EtOH, 24 h.

33
Carbonylchlorohydridotris(triphenylphosphine)ruthenium (II) (345 mg, 0.36 mmol, 2.5% mol) was added into 120 mL of absolute ethanol solution containing allyl nucleoside 30 (10.0 g, 14.5 mmol), and heated to allow reaction at 80°C for 12 h. The reaction solution was concentrated directly, the residue was dissolved in 80 mL of THF, and added with 10 mL of tert-butanol, 10 mL of water, potassium osmate dihydrate (26.7 mg, 72.6 µmol, 0.5% mol) and 50% *N*-methylmorpholine-*N*-oxide (4.53 mL, 10.9 mmol) in sequence, and reacted at 60°C for 8 h, the reaction was monitored by TLC (DCM/methanol=10/1). After the reaction was quenched with saturated sodium sulfite solution, the product was extracted with ethyl acetate, washed with water, and saturated saline solution in sequence, dried over anhydrous sodium sulfate, filtered, then the filtrate was concentrated, and purified by flash column (gradient elution: ethyl acetate/DCM=0%-60%) to obtain 9.3 g of a dihydroxylated isomeric mixed product.

The above dihydroxylated product was dissolved in 60 mL of ethanol-THF solution, 10 mL of NaIO₄ (3.32 g, 15.5 mmol) aqueous solution was added dropwise, and stirred at room temperature for 12 h. After filtering out the insoluble solid, 25% ammonia water (5.5 mL, 72.6 mmol) was added into the obtained filtrate, and then I₂ (1.84 g, 7.26 mmol) was added in batches and reacted at room temperature for 24 h. After quenching the reaction with saturated sodium sulfite solution, the product was extracted with ethyl acetate, washed with water, washed with saturated saline solution, dried over anhydrous sodium sulfate, filtered, then the filtrate was concentrated, and purified by flash column (gradient elution: ethyl acetate/DCM=0%-30%), thus obtaining 3.6 g of slightly yellow foamy solid product 33, with a yield of 37%. ¹H NMR (CDCl₃, 400 MHz) *δ* 8.87 (br s, 1H), 7.82-7.74 (m, 4H), 7.68-7.63 (m, 4H), 7.50-7.29 (m, 9H), 7.18 (d, *J =* 1.2 Hz, 1H), 5.62 (s, 1H), 4.93 (d, *J =* 11.8 Hz, 1H), 4.85 (s, 1H), 4.76 (d, *J =* 11.8 Hz, 1H), 4.69 (s, 1H), 4.30 (d, *J =* 12.2 Hz, 1H), 4.25 (d, *J =* 12.2 Hz, 1H), 4.07 (s, 1H), 1.58 (d, *J =* 1.2 Hz, 3H), 1.08 (s, 9H);¹³_{C} NMR (CDCl₃, 100 MHz) *δ* 163.31, 149.51, 135.49, 135.36, 133.37, 133.33, 133.14, 133.11, 132.21, 131.87, 130.27, 130.23, 128.55, 128.01, 127.72, 126.90, 126.43, 126.35, 125,44, 114.18, 111.17, 90.08, 87.22, 78.12, 76.00, 72.69, 69.48, 60.40, 58.82, 26.83, 19.40, 12.18; ESI-MS (m/z) 674.28 [M + H]⁺.

34
The carbonylchlorohydridotris(triphenylphosphine)ruthenium (II) (167 mg, 0.175 mmol, 2.5% mol) was added into 50 mL of absolute ethanol solution containing allyl nucleoside derivative 31 (4.9 g, 7.0 mmol), and heated to allow reaction at 80°C for 36 h. The reaction solution was concentrated directly, and the residue was dissolved in 40 mL of THF, added with 5 mL of tert-butanol, 5 mL of water, potassium osmate dihydrate (26 mg, 70 µmol, 1% mol) and 50% N-methylmorpholine-N-oxide (2.2 mL, 10.5 mmol) in sequence, and reacted at 60°C for 8 h. After the reaction was quenched with saturated sodium sulfite solution, the product was extracted with ethyl acetate, washed with water, washed with saturated saline solution, dried over anhydrous sodium sulfate, filtered, the obtained filtrate was concentrated, and the residue was purified by flash column (gradient elution: ethyl acetate/DCM containing 5% methanol=0%-100%) to obtain 3.6 g of the dihydroxylated isomeric mixed product. The above dihydroxylated product was dissolved 40 mL of ethanol-THF solution, 8 mL of NaIO₄ (1.58 g, 7.4 mmol) aqueous solution was added dropwise, and stirred at room temperature for 12 h. After filtering out the insoluble solid, 25% ammonia water (3.05 mL, 49.2 mmol) was added into the obtained filtrate, and then I₂ (1.25 g, 4.92 mmol) was added in batches and reacted at room temperature for 24 h. After quenching the reaction with saturated sodium sulfite solution, the product was extracted with ethyl acetate, washed with water, washed with saturated saline solution, dried over anhydrous sodium sulfate, filtered, then the filtrate was concentrated, and purified by flash column (gradient elution: ethyl acetate/DCM containing 5% methanol=0%-60%), thus obtaining 1.6 g of white foamy solid product 34, with a yield of 48%. ¹H NMR (CDCl₃, 400 MHz) *δ* 8.22 (s, 1H), 7.80-7.75 (m, 3H), 7.70-7.66 (m, 6H), 7.48-7.31 (m, 9H), 5.96 (s, 1H), 5.85 (br s, 2H), 5.19 (s, 1H), 4.88-4.77 (m, 3H), 4.36 (s, 1H), 4.32 (d, *J* = 12.2 Hz, 1H), 4.19 (d, *J* = 12.2 Hz, 1H), 1.06 (s, 9H); ¹³C NMR (CDCl₃, 100 MHz) *δ* 155.43, 153.10, 148.62, 135.58, 130.12, 128.48, 127.98, 127.91, 127.89, 127.70, 126.83, 126.33, 125.47, 119.91, 114.52, 89.84, 86.36, 78.22, 75.52, 72.86, 70.11, 59.39, 29.70, 19.27; ESI-MS (m/z) 683.35 [M + H]⁺.

35
The carbonylchlorohydridotris(triphenylphosphine)ruthenium (II) (238 mg, 0.25 mmol, 2.5% mol) was added into 120 mL of absolute ethanol solution containing allyl nucleoside 32 (7.2 g, 10 mmol), and heated to allow reaction at 80°C for 48 h. The reaction solution was concentrated directly, and the residue was dissolved in 50 mL of THF, added with 10 mL of tert-butanol and 10 mL of water, potassium osmate dihydrate (36 mg, 10 µmol, 1% mol) and 50% N-methylmorpholine-N-oxide (4.0 mL, 20 mmol) in sequence, and reacted at 60°C for 8 h, the reaction was monitored by TLC (DCM/methanol=10/1). After the reaction was quenched with saturated sodium sulfite solution, the product was extracted with ethyl acetate, washed with water, washed with saturated saline solution, dried over anhydrous sodium sulfate, filtered, the filtrate was concentrated, and purified by flash column (gradient elution: ethyl acetate/DCM=0%-40%) to obtain 5.6 g of the dihydroxylated isomeric mixed product.

The above dihydroxylated product was dissolved in 60 mL of ethanol-THF solution, 10 mL of NaIO₄ (3.54 g, 7.35 mmol) aqueous solution was added dropwise, and stirred at room temperature for 12 h. After filtering out the insoluble solid, 25% ammonia water (5.5 mL, 73.5 mmol) was added into the obtained filtrate, and then I₂ (1.87 g, 7.35 mmol) was added in batches and reacted at room temperature for 24 h. After quenching the reaction with saturated sodium sulfite solution, the product was extracted with ethyl acetate, washed with water, washed with saturated saline solution, dried over anhydrous sodium sulfate, filtered, then the filtrate was concentrated, and purified by flash column (gradient elution: ethyl acetate/DCM=0%-40%), thus obtaining 2.5 g of white foamy solid product 35, with a yield of 48%. ¹H NMR (CDCl₃, 400 MHz) *δ* 7.80-7.66 (m, 8H), 7.58 (s, 1H), 7.47-7.31 (m, 9H), 5.86 (s, 1H), 5.03 (s, 1H), 4.81-4.76 (m, 5H), 4.34 (s, 1H), 4.31 (d, *J* = 12.2 Hz, 1H), 4.19 (d, *J* = 12.2 Hz, 1H), 4.06 (s, 3H), 1.06 (s, 9H); ¹³C NMR (CDCl₃, 100 MHz) *δ* 161.63, 159.37, 152.27, 136.01, 135.60, 135.58, 133.75, 133.11, 132.19, 132.16, 130.13, 128.49, 127.99, 127.96, 127.69, 126.67, 126.31, 126.23, 125.38, 115.99, 114.61, 89.67, 86.10, 78.31, 77.64, 72.91, 70.05, 59.41, 53.97, 26.70, 19.25; ESI-MS (m/z) 713.40 [M + H]⁺.

### Synthesis of S-6'-CN-LNA phosphoramidite monomer

### Synthesis of S-6'-CN-LNA-T phosphoramidite monomer

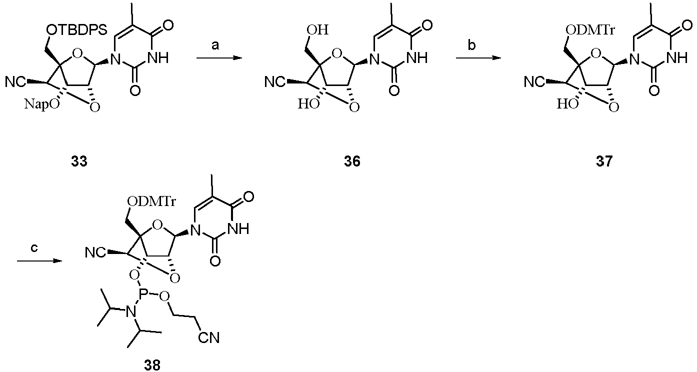

The reaction conditions were: (IV. a) (1) DDQ, DCM-H₂O (20/1), room temperature, 24 h; (2) Et₃N, Et₃N·3HF, THF, room temperature 12 h; (b) DMTrCl, pyridine, room temperature, 12 h; (c) 2-cyanoethyl *N,N,N',N'*-tetraisopropylphosphorodiamidite, 1H-tetrazole, room temperature, 5 h.

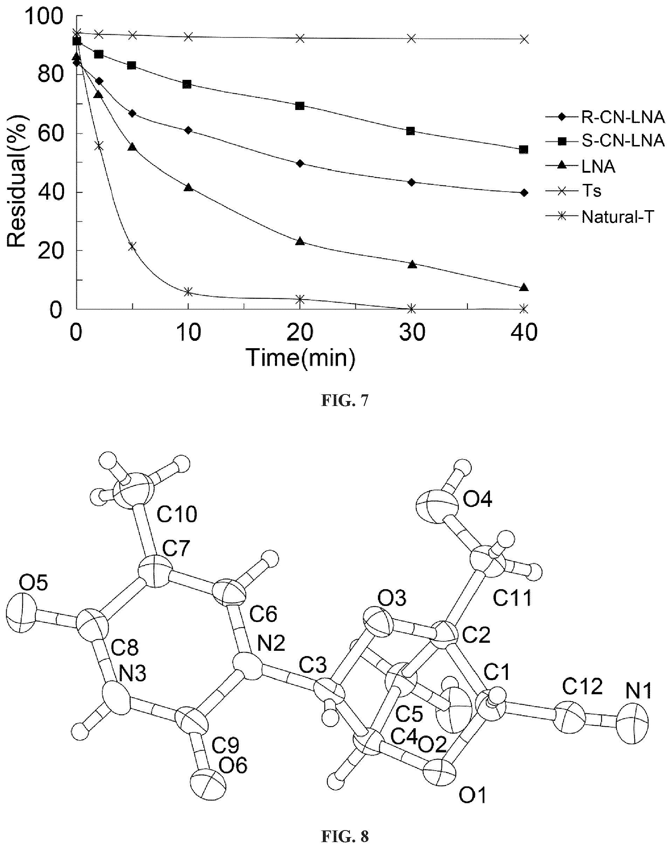

36
DDQ (420 mg, 1.86 mmol) was added into the nucleoside derivative 33 (3.6 g, 5.34 mmol) in 42 mL of DCM-water (20:1) mixture at room temperature, and reacted at room temperature for 24 h, the reaction was monitered by TLC (DCM/ethyl acetate=10/1). The reaction solution was subjected to vacuum concentration, the concentrated solution was extracted with ethyl acetate, washed with saturated sodium sulfite solution, sodium bicarbonate solution, and saturated saline solution in sequence, dried over anhydrous sodium sulfate, filtered, and the filtrate was concentrated. The obtained residue was dissolved in 50 mL of THF, to which triethylamine (1.78 mL, 13.35 mmol) and triethylamine trihydrofuric acid (2.61 mL, 16 mmol) were added successively, the reaction was conducted at room temperature for 12 h and then monitored by TLC (DCM/methanol=10/1). 10 g of sodium bicarbonate solid was added into the reaction solution and stirred until no bubbles were generated, the reaction solution was subjected to vacuum concentration, and directly purified by flash column (gradient elution: methanol/DCM=0%-20%) to obtain 1.1 g of slightly yellowish white solid 36, with a yield of 70%. ¹H-NMR (400 MHz, MeOH-*d*₄) *δ* 7.62 (s, 1H), 5.86 (s, 1H), 4.78 (s, 1H), 4.53 (s, 1H), 4.22 (s, 1H), 4.17 (d, *J =* 12.0 Hz, 1H), 4.13 (d, *J =* 12.0 Hz, 1H), 1.89 (s, 3H); ¹³C-NMR (100 MHz, MeOH-*d*₄) *δ* 164.97, 150.36, 134.89, 114.74, 109.74, 90.36, 86.75, 80.78, 69.56, 68.45, 55.87. 11.19; ESI-MS (m/z) 296.13 [M + H]⁺. The crystal structure of the S-6'-CN-LNA-T monomer (compound 36) was shown in FIG. 8.

37
4,4'-dimethoxytrityl chloride (3.4 g, 10 mmol) was added into 15 mL pyridine solution containing the CN-modified locked nucleoside derivative 36 (2.0 g, 6.77 mmol), stirred to allow reaction at room temperature for 12 h, and the reaction was monitored by TLC (DCM/methanol=10/1). The reaction was quenched with 50 mL of methanol, and subjected to vacuum concentration, the residue was diluted with ethyl acetate, washed with water and saturated saline solution in sequence, dried over Na₂SO₄, concentrated, and purified by flash column (gradient elution: methanol/DCM=0%-10%) to obtain 2.6 g of 5'-*O*-DMTr-protected nucleoside 37 as a white solid, with a yield of 64%. ¹H-NMR (400 MHz, DMSO-*d*₆) *δ* 11.44 (s, 1H), 7.46-7.24 (m, 10H), 6.92 (dd, *J* = 6.0, 1.4 Hz, 4H), 6.30 (s, 1H), 5.53 (s, 1H), 5.11 (s, 1H), 3.79 (d, *J* = 7.9 Hz, 1H), 3.74 (s, 6H), 3.49 (d, *J* = 7.4 Hz, 1H), 1.60 (s, 3H); 13C-NMR (100 MHz, DMSO-d6) *δ* 162.66, 157.16, 148.78, 143.44, 134.16, 133.74, 132.93, 128.73, 128.65, 126.87, 126.54, 126.80, 114.35, 112.20, 107.85, 87.62, 85.06, 84.89, 79.26, 68.84, 67.42, 57.44, 53.97, 11.15; ESI-MS 620.20 (m/z) [M + Na]⁺.

38
The nucleoside 37 (2.0 g, 3.35 mmol) and 2-cyanoethyl *N*,*N*,*N'*,*N'*-tetraisopropylphosphorodiamidite (1.51 g, 5 mmol) in 20 mL of DCM solution was added with 1H-tetrazole (230 mg, 3.35 mmol), the obtained mixture was reacted at room temperature for 5 h, and the reaction was monitored by TLC (ethyl acetate/DCM=5/1). The saturated NaHCO₃ solution was added into the obtained reaction solution, extracted with DCM, the extract was washed with saturated saline solution, dried with MgSO₄, concentrated, and the concentrated solution was purified by flash column (gradient elution: ethyl acetate/DCM=0%-20%) to obtain 2.1 g of phosphoramidite 38 as a white foamy solid, with a yield of 79%. ³¹P-NMR (152 MHz, DMSO-*d*₆) *δ* 148.95, 148.35; ESI-MS (m/z) 798.33 [M + H]⁺.

### Synthesis of S-6'-CN-LNA-A phosphoramidite monomer

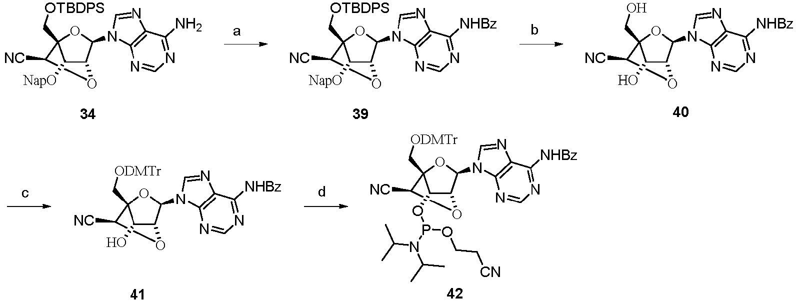

The reaction conditions were: (a) BzCl, pyridine, room temperature, 6 h; (b) (1) DDQ, DCM-H₂O (20/1), room temperature, 24 h; (2) Et₃N, Et₃N·3HF, THF, room temperature 12 h; (b) DMTrCl, pyridine, room temperature, 8 h; (c) 2-cyanoethyl *N*,*N*,*N'*,*N'*-tetraisopropylphosphorodiamidite, 1*H*-tetrazole, room temperature, 8 h.

39
At room temperature, benzoyl chloride (422 µL, 3.66 mmol) was added dropwise to 5 mL pyridine solution containing the nucleoside 34 (1.0 g, 1.46 mmol), and reacted at room temperature for 4 h, the benzoyl chloride (100 µL, 0.87 mmol) was added, and the reaction was continued for 2 h. 15% NaOH solution was added dropwise into the reaction solution to adjust the pH value of 8, and the the reaction solution was stirred at room temperature for 2 h. The product was diluted with ethyl acetate, washed successively with water, 1 N HCl solution, and saturated saline solution, dried over anhydrous sodium sulfate, filtered, the filtrate was concentrated, and purified by flash column (gradient elution: ethyl acetate/DCM=0%-45%) to obtain 1.05 g of slightly yellow foamy solid, with a yield of 91%. ¹H NMR (CDCl₃, 400 MHz) *δ* 9.02 (br s, 1H), 8.65 (s, 1H), 8.03 (d, *J* = 7.2 Hz, 2H), 7.99 (s, 1H), 7.79-7.34 (m, 20H), 6.03 (s, 1H), 5.19 (s, 1H), 4.89 (s, 1H), 4.87 (d, *J* = 12.2 Hz, 1H), 4.82 (d, *J* = 12.2 Hz, 1H), 4.34-4.31 (m, 2H), 4.19 (d, *J* = 12.2 Hz, 1H), 1.06 (s, 9H); ¹³C NMR (CDCl₃, 100 MHz) *δ* 164.60, 152.64, 150.42, 149.70, 140.19, 135.55, 133.53, 133.42, 133.09, 132.99, 132.12, 132.09, 130.15, 128.94, 128.52, 128.00, 127.92, 127.91, 127.85, 127.69, 126.80, 126.38, 126.28, 125.42, 123.29, 114.42, 90.04, 86.43, 78.19, 77.54, 72.97, 70.17, 59.42, 29.70, 26.70, 19.26; ESI-MS (m/z) 787.32 [M + H]⁺.

40
DDQ (780 mg, 3.44 mmol) was added to the nucleoside derivative 39 (900 mg, 1.14 mmol) in 10 mL of DCM-water (20:1) mixture at room temperature, and reacted at room temperature for 24 h, the reaction was monitered by TLC (DCM/methanol=10/1). The reaction solution was subjected to vacuum concentration, extracted with ethyl acetate, washed with saturated sodium sulfite solution, sodium bicarbonate solution, and saturated saline solution in sequence, dried over anhydrous sodium sulfate, filtered, and the filtrate was concentrated. The obtained residue was dissolved in 15 mL of THF, to which triethylamine (400 µL, 2.85 mmol) and triethylamine trihydrofuric acid (550 µL, 3.42 mmol) were added successively, and the reaction was conducted at room temperature for 12 h, and the reaction was monitored by TLC (DCM/methanol=10/1). 1.0 g of sodium bicarbonate solid was added into the reaction solution and stirred until no bubbles were generated, the reaction solution was subjected to vacuum concentration, and directly purified by flash column (gradient elution: DCM/methanol=0%-20%) to obtain 340 mg of CN-modified locked nucleoside 40, with a yield of 73%. ¹H NMR (CD₃OD, 400 MHz) *δ*; ESI-MS (m/z) 409.19 [M + H]⁺, 431.16 [M + Na]⁺.

41
At room temperature, 4,4'-dimethoxytrityl chloride (373 mg, 1.1 mmol) was added into 5 mL pyridine solution containing the CN-modified locked nucleoside derivative 40 (300 mg, 0.735 mmol), stirred to allow reaction at room temperature for 8 h, and the reaction was monitored by TLC (DCM/methanol=10/1). The reaction was quenched with 1 mL of methanol, vacuum concentration was conducted, the residue was diluted with ethyl acetate, the diluted solution was washed with water and saturated saline solution in sequence, the obtained product was dried over Na₂SO₄ and concentrated, then the concentrated solution was purified by flash column (gradient elution: ethyl acetate/DCM containing 5% methanol=0%-80%) to obtain 330 mg of 5'-*O*-DMTr-protected CN-modified locked nucleoside 41 as a light yellow solid, with a yield of 64%. ¹H NMR (DMSO-*d*₆, 400 MHz) *δ* 11.26 (br s, 1H), 8.79 (s, 1H), 8.54 (s, 1H), 8.06 (d, *J =* 7.3 Hz, 2H), 7.67 (t, *J =* 7.3 Hz, 1H), 7.57 (t, *J =* 7.8 Hz, 2H), 7.46 (d, *J =* 7.4 Hz, 2H), 7.34-7.24 (m, 6H), 7.25 (t, *J* = 7.2 Hz, 1H), 6.90 (d, *J* = 8.9 Hz, 4H), 6.33 (d, *J* = 3.5 Hz, 1H), 6.23 (s, 1H), 5.36 (s, 1H), 4.92 (s, 1H), 4.70 (d, *J* = 3.5 Hz, 1H), 3.85 (d, *J =* 11.1 Hz, 1H), 3.74 (s, 6H), 3.47 (d, *J =* 11.1 Hz, 1H); ¹³C NMR (DMSO-*d*₆, 100 MHz) *δ* 166.10, 158.64, 152.33, 152.02, 150.97, 145.07, 142.33, 135.88, 135.38, 130.25, 130.19, 128.98, 128.96, 128.36, 128.12, 126.03, 116.03, 113.73, 113.69, 89.29, 86.26, 85.39, 81.12, 71.78, 69.40, 60.09, 55.52; ESI-MS (m/z) 711.37 [M + H]⁺.

42
At room temperature, the nucleoside 41 (240 mg, 0.34 mmol) and 2-cyanoethyl *N*,*N*,*N'*,*N'*-tetraisopropylphosphorodiamidite (203 mg, 0.75 mmol) in 10 mL of DCM solution was added with 1H-tetrazole (21 mg, 0.3 mmol), reacted at room temperature for 8 h, and the reaction was monitored by TLC (DCM/methanol=15/1). The reaction solution was directly purified by flash column (gradient elution: ethyl acetate/DCM=0%-40%) to obtain 280 mg of phosphoramidite 42 as a slightly yellow foamy solid, with a yield of 90%. ³¹P NMR (152 MHz, DMSO-*d*₆) *δ* 149.56, 149.25; ESI-MS (m/z) 909.38 [M - H]⁻.

### Synthesis of S-6'-CN-LNA-G phosphoramidite monomer

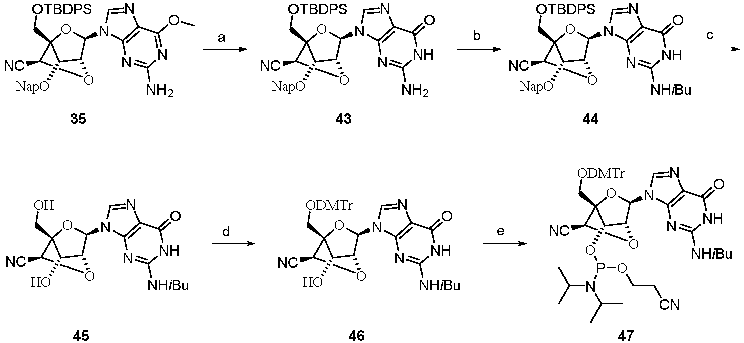

The reaction conditions were: (a) 2 N HCl, THF-CH₃OH (1/1), 60°C, 24 h; (b) isobutyryl chloride, Et₃N, DMAP, toluene, 100°C, 12 h; (c) (1) DDQ, DCM-H₂O (20/1), room temperature, 24 h; (2) Et₃N, Et₃N·3HF, THF, room temperature, 12 h; (d) DMTrCl, pyridine, room temperature, 12 h; (e) 2-Cyanoethyl *N,N,N',N'*-tetraisopropylphosphorodiamidite, 1H-tetrazole, room temperature, 6 h.

43
15.4 mL of 2 N hydrochloric acid solution was added into 24 mL of THF-methanol solution (1/1 v/v) containing CN-modified locked nucleoside 35 (2.2 g, 3.08 mmol), and reacted at 60°C for 24 h, the reaction was monitored by TLC (DCM/ethyl acetate=2/1). The product was extracted with ethyl acetate, the extract was washed with water and then washed with saturated saline solution, the obtained product was dried over anhydrous sodium sulfate and filtered, the obtained filtrate was concentrated, and purified by flash column (gradient elution: methanol/DCM=0%-10%), thus obtaining 1.48 g of guanosine 43 as a white solid, with a yield of 69%. ¹H NMR (CDCl₃, 400 MHz) *δ* 11.94 (br s, 1H), 7.72-7.64 (m, 9H), 7.48-7.16 (m, 10H), 6.53 (br s, 2H), 5.75 (s, 1H), 4.81-4.70 (m, 3H), 4.28-4.25 (m, 2H), 4.17 (d, *J* = 12.2 Hz, 1H), 1.03 (s, 9H); ¹³C NMR (CDCl₃, 100 MHz) *δ* 158.96, 153.85, 150.40, 135.56, 133.07, 133.03, 132.17, 132.13, 130.15, 129.06, 128.41, 128.25, 128.02, 127.91, 127.66, 126.67, 126.34, 126.26, 125.41, 125.33, 117.21, 114.58, 89.76, 85.91, 78.28, 72.87, 69.98, 59.39, 26.72, 19.28; ESI-MS (m/z) 699.29 [M + H]⁺.

44
DMAP (105 mg, 0.86 mmol) and triethylamine (718 µL, 5.16 mmol) were added into 12 mL of toluene solution containing guanosine 43 (1.2 g, 1.72 mmol), then added with isobutyryl chloride (550 µL, 5.16 mmol) and reacted at 100°C for 12 h, the reaction was monitored by TLC (DCM/methanol=10/1). After stopping the heating and allowing the reaction solution to cool to room temperature, 1 mL of methanol was added to quench the reaction, the product was extracted with ethyl acetate, washed with water and saturated saline solution in sequence, the obtained product was dried over anhydrous sodium sulfate and filtered, the obtained filtrate was concentrated, and purified by flash column (gradient elution: ethyl acetate/DCM=0%-80%) to obtain 1.1 g of isobutyryl-protected guanosine 44 as a slightly yellow foamy solid, with a yield of 83%. ¹H NMR (CDCl₃, 400 MHz) *δ* 11.97 (s, 1H), 8.78 (s, 1H), 7.75-7.60 (m, 9H), 7.44-7.32 (m, 9H), 5.60 (s, 1H), 4.79 (d, *J* = 12.0 Hz, 1H), 4.69 (s, 1H), 4.65 (d, *J* = 12.0 Hz, 1H), 4.62 (s, 1H), 4.27 (d, *J =* 12.3 Hz, 1H), 4.18-4.14 (m, 2H), 2.68-2.61 (m, 1H), 1.28-1.24 (m, 6H), 1.06 (s, 9H); ¹³C NMR (CDCl₃, 100 MHz) *δ* 178.64, 155.20, 147.76, 146.66, 135.53, 132.02, 128.45, 128.04, 127.97, 127.78, 127.57, 126.81, 126.49, 126.38, 125.47, 114.47, 89.81, 86.06, 78.45, 73.25, 69.89, 59.26, 36.40, 26.70, 19.25, 19.08, 18.85; ESI-MS (m/z) 767.39 [M - H]⁻.

45
DDQ (885 mg, 3.9 mmol) was added to the nucleoside derivative 44 (1.0 g, 1.3 mmol) in 16 mL of DCM-water (20:1) mixture at room temperature, and reacted at room temperature for 24 h, the reaction was monitered by TLC (DCM/methanol=10/1). The reaction solution was subjected to vacuum concentration, the concentrated solution was extracted with ethyl acetate, the extract was washed with saturated sodium sulfite solution, sodium bicarbonate solution, and saturated saline solution in sequence, dried over anhydrous sodium sulfate, and filtered, then the filtrate was concentrated. The obtained residue was dissolved in 10 mL of THF, added with triethylamine (453 µL, 3.25 mmol) and triethylamine trihydrofuric acid (636 µL, 3.9 mmol) successively, and then reacted at room temperature for 12 h, and the reaction was monitored by TLC (DCM/methanol=10/1). 700 mg of sodium bicarbonate solid was added into the reaction solution and stirred until no bubbles were generated, the reaction solution was subjected to vacuum concentration, and directly purified by flash column (gradient elution: DCM/methanol=0%-25%) to obtain 380 mg of CN-modified locked nucleoside 45, with a yield of 75%. ¹H NMR (MeOH-*d*₄, 400 MHz) *δ* 8.03 (s, 1H), 5.91 (s, 1H), 4.88 (s, 1H), 4.78 (s, 1H), 4.45 (s, 1H), 4.17 (s, 2H), 2.74-2.67 (m, 1H), 1.23 (d, *J* = 6.9 Hz, 6H); ¹³C NMR (MeOH-*d*₄, 100 MHz) *δ* 181.72, 150.01, 149.42, 137.97, 121.52, 116.12, 91.68, 87.21, 82.33, 71.84, 70.25, 57.76, 36.36, 19.28; ESI-MS (m/z) 389.09 [M - H]⁻.

46
At room temperature, 4,4'-dimethoxytrityl chloride (390 mg, 1.15 mmol) was added into 5 mL pyridine solution containing the CN-modified locked nucleoside derivative 45 (300 mg, 0.77 mmol), stirred to allow reaction at room temperature for 12 h, and the reaction was monitored by TLC (DCM/methanol=5/1). The reaction was quenched with 2 mL of methanol, vacuum concentration was conducted, the residue was diluted with ethyl acetate, the diluted solution was washed with water and saturated saline solution in sequence, dried over Na₂SO₄, concentrated, and purified by flash column (gradient elution: methanol/DCM=0%-10%) to obtain 520 mg of 5'-*O*-DMTr-protected CN-modified locked nucleoside 46 as a light yellow powder, with a yield of 97%. ¹H NMR (DMSO-d₆, 400 MHz) *δ* 12.13 (s, 1H), 11.86 (s, 1H), 8.06 (s, 1H), 7.45-7.22 (m, 9H), 8.91 (d, *J =* 8.8 Hz, 4H), 6.29 (d, *J =* 3.5 Hz, 1H), 5.95 (s, 1H), 5.29 (s, 1H), 4.75 (s, 1H), 4.48 (d, *J* = 3.5 Hz, 1H), 3.86 (d, *J* = 11.2 Hz, 1H), 3.74 (s, 6H), 3.46 (d, *J =* 11.2 Hz, 1H), 2.82-2.75 (m, 1H), 1.13 (d, *J* = 6.8 Hz, 6H); ¹³C NMR (DMSO-*d*₆, 100 MHz) *δ* 180.74, 158.67, 155.20, 148.86, 145.08, 136.74, 135.82, 135.37, 130.26, 130.21, 128.35, 128.11, 127.29, 120.82, 115.96, 113.72, 113.68, 81.22, 86.29, 80.07, 71.35, 69.42, 60.01, 55.52, 35.24, 19.31; ESI-MS (m/z) 691.36 [M - H]⁻.

47
At room temperature, the guanosine 46 (380 mg, 0.55 mmol) and 2-cyanoethyl *N*,*N*,*N'*,*N'*-tetraisopropylphosphorodiamidite (330 mg, 1.1 mmol) in 10 mL of DCM solution was added with 1*H*-tetrazole (31 mg, 0.44 mmol), reacted at room temperature for 6 h, and the reaction was monitored by TLC (DCM/ethyl acetate=2/1). The reaction solution was directly purified by flash column (gradient elution: ethyl acetate/DCM=0%-50%) to obtain 380 mg of phosphoramidite 47 as a slightly yellow waxy solid, with a yield of 77%. ³¹P NMR (152 MHz, DMSO-*d*₆) *δ* 149.69, 149.09; ESI-MS (m/z) 891.42 [M - H]⁻.

### Synthesis of S-6'-CN-LNA-^{m}C phosphoramidite monomer

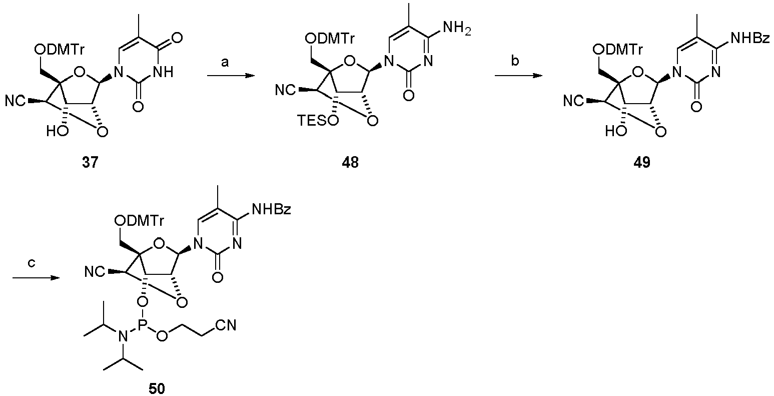

The reaction conditions were: (a) (1) TESCl, Et₃N, acetonitrile, 0°C to room temperature, 3 h; (2) 1,2,4-triazole, POCl₃, acetonitrile, 0°C to room temperature, 2 h; (3) NH₄OH, 1,4-dioxane, room temperature, 2 h; (b) (1) B_{z2}O, acetonitrile, room temperature, 20 h; (2) TBAF, THF, room temperature, 12 h; (c) 2-Cyanoethyl *N*,*N*,*N'*,*N'*-tetraisopropylphosphorodiamidite, 1*H*-tetrazole, DCM, room temperature, 5 h.

48
At 0°C, triethylsilyl chloride (4.78 mL, 28.45 mmol) was added into acetonitrile solution (50 mL) containing thymidine 37 (3.4 g, 5.69 mmol) and triethylamine (15.8 mL, 113.8 mmol), and stirred at room temperature for 3 h. 1,2,4-triazole (5.9 g, 85.35 mmol) was added into the reaction solution to continue stirring for 10 min. POCl₃ (1.6 mL, 17.07 mmol) was added dropwise at 0°C and reacted at room temperature for 2 h. The reaction solution was poured into 300 mL of ice water, extracted with ethyl acetate, the extract was washed with water, saturated NaHCO₃ solution, and saturated saline solution in sequence, the obtained product was dried over anhydrous Na₂SO₄ and filtered, and the obtained filtrate was concentrated. The residue was dissolved in 30 mL of 1,4-dioxane, added with ammonia water (4.25 mL), and stirred at room temperature for 2 h. The reaction solution was subjected to vacuum concentration, the concentrated solution was extracted with ethyl acetate, the extract was washed with water and saturated saline solution in sequence, dried over anhydrous Na₂SO₄, and filtered, then the obtained filtrate was concentrated. The obtained concentrated solution was purified by flash column (gradient elution: methanol/DCM=0%-15%) to obtain 3.9 g of aminonucleoside 48 as a pale yellow solid with a yield of 96%. ¹H NMR (DMSO-*d*₆, 400 MHz) *δ* 7.50-7.44 (m, 4H), 7.37-7.25 (m, 8H), 6.93 (d, *J =* 8.6 Hz, 4H), 5.55 (s, 1H), 5.09 (s, 1H), 4.46 (s, 1H), 4.35 (s, 1H), 3.75 (s, 6H), 3.68 (d, *J =* 11.2 Hz, 1H), 3.53 (d, *J =* 11.2 Hz, 1H), 1.70 (s, 3H), 0.81-0.74 (m, 9H), 0.55-0.43 (m, 6H); ¹³C NMR (DMSO-d₆, 100 MHz) *δ* 166.07, 158.80, 158.77, 154.96, 144.83, 136.61, 135.63, 135.18, 130.24, 130.08, 128.43, 128.03, 127.44, 115.51, 113.76, 101.81, 88.80, 87.37, 86.49, 80.72, 71.31, 69.03, 58.57, 55.55, 13.94, 6.75, 4.45; ESI-MS (*m*/*z*) 733.31 [M + Na]⁺.

49
Acetonitrile (20 mL) solution containing the aminonucleoside 48 (3.9 g, 5.49 mmol) was added with benzoic anhydride (2.73 g, 12 mmol), and stirred at room temperature for 20 h for reaction, the reaction was quenched with 5 mL of water. TBAF (3.47 g, 11 mmol) was added to the reaction mixture, reacted at room temperature for 12 h, 15% NaOH was added to adjust the pH to 10, and stirring was continued for 3 h. The solution after reaction was extracted with ethyl acetate, washed with water and saturated saline solution in sequence, the obtained product was dried over anhydrous Na₂SO₄ and filtered, the filtrate was concentrated, and purified by flash column (gradient elution: ethyl acetate/DCM=0%-40%), such that 3.2 g of nucleoside 49 was obtained as a white foamy solid, with a yield 83%. ¹H NMR (DMSO-d₆, 400 MHz) *δ* 13.08 (s, 1H), 8.21 (d, *J* = 7.6 Hz, 1H), 7.62-7.27 (m, 12H), 6.95 (d, *J =* 8.6 Hz, 4H), 6.40 (d, *J =* 3.2 Hz, 1H), 5.61 (s, 1H), 5.18 (s, 1H), 4.57 (s, 1H), 4.31 (d, *J* = 3.2 Hz, 1H), 3.86 (d, *J* = 11.2 Hz, 1H), 3.76 (s, 6H), 3.53 (d, *J =* 11.2 Hz, 1H), 1.86 (s, 3H); ¹³C NMR (DMSO-d₆, 100 MHz) *δ* 178.62, 170.82, 159.46, 147.48, 145.03, 137.25, 137.10, 135.30, 130.31, 130.25, 129.86, 128.80, 128.46, 128.12, 127.39, 115.87, 113.78, 110.13, 89.52, 87.06, 86.50, 80.60, 70.34, 69.06, 59.99, 55.55, 13.62; ESI-MS (*m*/*z*) 701.32 [M + H]⁺.

50
The nucleoside 49 (2.3 g, 3.28 mmol) and 2-cyanoethyl *N*,*N*,*N'*,*N'*-tetraisopropylphosphorodiamidite (1.2 g, 3.93 mmol) in 25 mL of anhydrous DCM were added with 1*H*-tetrazole (230 mg, 3.28 mmol), and reacted at room temperature for 5 h. The saturated NaHCO₃ solution was added into the obtained reaction solution, extracted with DCM, the resulting organic layer was washed with saturated saline solution, dried with anhydrous MgSO₄, and filtered, the filtrate was concentrated and purified by flash column (gradient elution: ethyl acetate/DCM=0%-20%) to obtain 2.13 g of phosphoramidite 50 as a white foamy solid, with a yield of 72%. ³¹P-NMR (152 MHz, DMSO-*d*₆) *δ* 149.23, 147.95; ESI-MS (m/z) 901.46 [M + H]⁺.

### Experimental Example 1 CN-LNA-modified ASO-protein interaction

### Experimental method

### Thermal denaturation test (Tₘ value)

Annealing buffer: 10 mM Na₃PO₄, 100 mM NaCl, pH 7.2. Annealing method: two oligonucleotide single strands were diluted with the annealing buffer to the final concentration of 2 µM, heated in a 95°C water bath for 5 min, slowly cooled to room temperature, and placed in a 4°C refrigerator overnight. Tₘ determination method: 100 µL of the sample to be tested was added into the cuvette and covered tightly with a thermal isolation lid. 15°C was selected as the starting measurement temperature, 90°C was selected as the ending temperature, the temperature rising rate was 0.5°C/min, the A260 reading rate was 1 time/°C, and finally the Tₘ value was detected by instrument. Each sample was measured 3 times, and an average was taken as the final result.

### Immunofluorescence assay:

1) Cell preparation: HeLa cells were inoculated into a 20 mm confocal culture dish the day before, with the cell density of 2×10⁵. The 20 mm confocal culture dish was placed in a CO₂ incubator for culture.
2) Preparation of PS-ASOs: RNase- and DNase-free water was added to dissolve ON1, ON2, and ON3 powders into 100 µM solutions.
3) Transfection: 10 µL of Lipofectamine 2000 (Solarbio) was added into Opti-MEM^{®} Medium to prepare 250 µL transfection reagent. The solution prepared above was mixed with 250 µL of the Lipofectamine 2000 transfection reagent at the transfection amount of 4 µg/dish to obtain a mixed solution, and 500 µL of the mixed solution was added to each dish 5 min later. The mixed solution of transfection reagent was removed and the cells were washed 3 times with PBS. 500 µL of DMEM (SIGMA) was added to each well to obtain cell maintenance solution, and the 20 mm confocal culture dish was placed in a CO₂ incubator for transfection for 5 min, 2 h, and 4 h separately.
4) Fixation: the waste liquid was discarded, the cells were washed 3 times with PBS, then fixated with 4% paraformaldehyde at room temperature for 30 min, and then permeated with 0.1% Triton X-100 (prepared with PBS) for 5 min.
5) Blocking: the fixated cells were blocked with a blocking buffer (prepared with 1 mg/mL BSA and PBS) for 30 min at room temperature.
6) Primary antibody binding: the primary antibody (NONO, Cell signaling) was diluted in the blocking buffer, incubated at room temperature for 1 h, then placed in a 4°C refrigerator overnight, and washed 3 times with the washing buffer (0.1% Tween prepared in PBS, washed every 5 min).
7) Secondary antibody binding: the secondary antibody (Anti-rabbit IgG, Cell signaling) was diluted with the blocking buffer, incubated at room temperature for 1 h, and finally the cells were washed 3 times with the washing buffer.
8) Sealing and detection: the Prolong antifade mountant (Cell signaling) containing 4',6-diamidino-2-phenylimidole (DAPI) was added dropwise into the petri dish, and finally the sealed cell section was observed and photographed with an inverted fluorescence microscope.

### Caspase 3/7 activity experimental method

1) Cell preparation: HeLa cells (Beina Biotech) were inoculated into a 96-well cell culture plate the day before, at the cell density of 5×10⁴. The 96-well cell culture plate was placed into a CO₂ incubator for culture.
2) Preparation of PS-ASOs: RNase- and DNase-free water was added to dissolve ON1, ON2, and ON3 powders into 10 µM solutions. The 10 µM solutions of ON1, ON2, and ON3 were diluted with Opti-MEM^{®} Medium (Thermofisher) into solutions of 1,000 nM, 500 nM, 250 nM, 125 nM, 62.5 nM, and 0 nM.
3) Transfection: 2 µL of Lipofectamine 2000 (Thermofisher) was added into the Opti-MEM^{®} Medium to prepare 100 µL of the transfection reagent. 100 µL of PS ASOs solutions of different concentrations were separately mixed well with 100 µL of the Lipofectamine 2000 transfection reagent to obtain mixed solutions, and 50 µL of the mixed solution was added to each well 5 min later. The mixed solution of transfection reagent was removed and then was washed 3 times with PBS 4 min after the transfection. 100 µL of DMEM (Thermofisher) was added to each well to obtain cell maintenance solution, and the 96-well cell culture plate was placed in a CO₂ incubator to continue culturing for 8 h.
4) Detection: the 96-well cell plate was taken out from the CO₂ incubator, then 100 µL of Caspase-Glo 3/7 Reagent (Promega) was added to each well, incubated for 30 min, and the luminescence signal of each well was read with a multifunctional fluorescence microplate reader (Promega).

### Experiment content

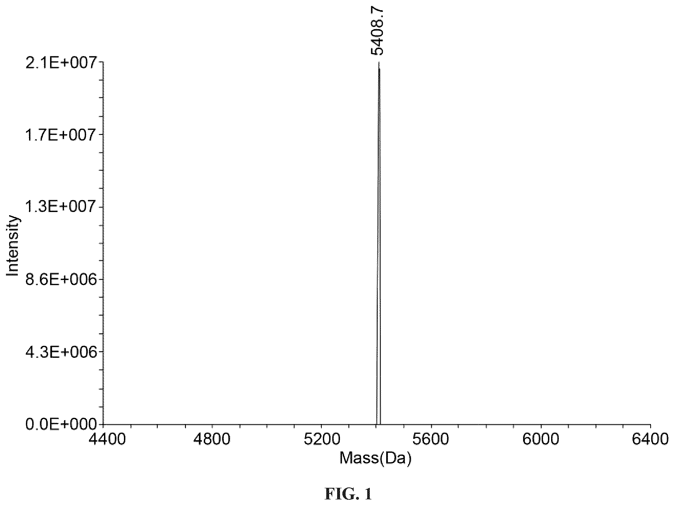

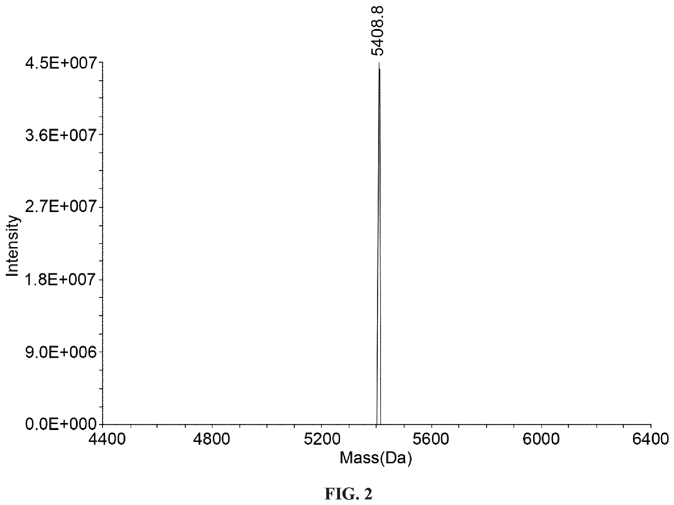

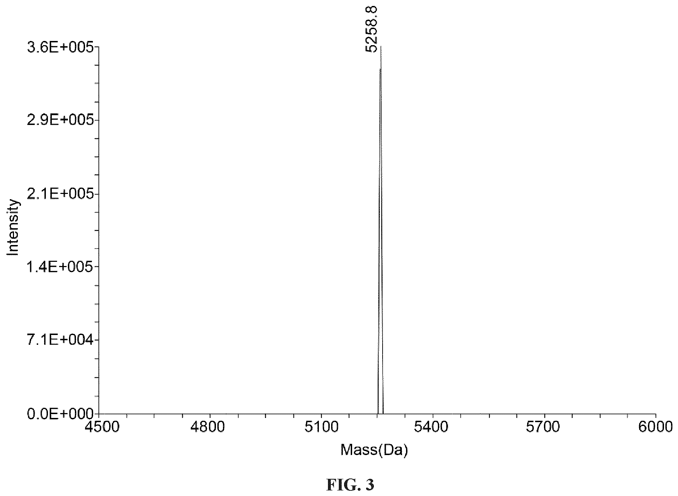

Intracellular ASO-protein interactions are closely related to the druggability of PS-ASOs. Differences in chemical modifications may significantly affect ASO-protein interactions, thereby affecting the subcellular distribution of binding proteins and inducing apoptosis toxicity. In order to detect the effect of CN-LNA modification on the interaction between ASO and intracellular proteins, the toxic 3-10-3 gapmer PS ASO sequence 449093 (5'-TTCAGTCATGACTTCC-3' (SEQ ID NO: 1)) reported in the literature was selected as the template sequence (*Nature Biotech.,* 2019, 37, 640) for study. The corresponding T and ^{m}C of the synthesized R-CN-LNA and S-CN-LNA were introduced into two ends of 449093 through the solid-phase phosphoramidite method to synthesize ON1 and ON2, respectively, while LNA-modified 449093 (ON3) was regarded as a control. The mass spectrometry identification data were shown in Table 1, and the corresponding mass spectra were shown in FIG. 1 to FIG. 3.

First, the hybridization properties of ON1 to ON3 and a target RNA were measured, and their Tₘ values were: 67.04°C, 63.96°C, and 66.59°C, respectively. The results showed that compared with LNA-modified PS ASO ON3, CN-LNA-modified PS-ASOs ON1 to ON2 could still maintain high affinity with the target RNA.

Secondly, the intracellular paraspeckle protein P54nrb was used as a model protein to investigate the effect of CN-LNA modification on the interaction between ASO and intracellular proteins. The paraspeckle protein P54nrb can bind to toxic ASO (with binding affinity at a low nmol level), change its distribution to enter nucleolus for aggregation, and this effect is obvious and positively correlated with ASO toxicity. The experimental results were expressed by the aggregation of the paraspeckle protein P54nrb in the nucleolus.

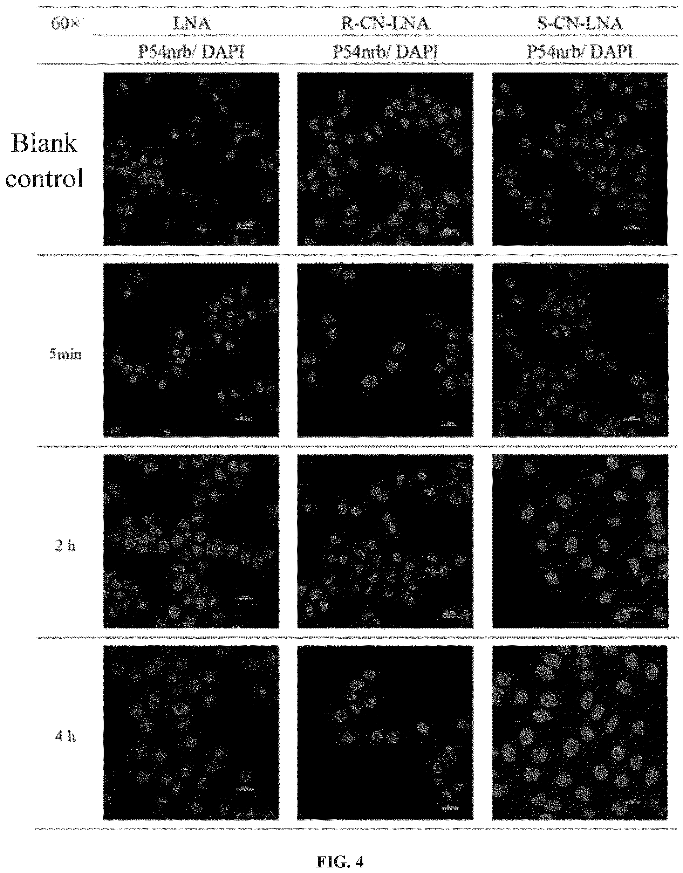

Cells were transfected with ON1, ON2, and ON3, and the transfection times were set as 5 min, 2 h, and 4 h, respectively. The protein localization of P54nrb protein was observed and photographed under an inverted fluorescence microscope. The results were shown in FIG. 4 (scale bar: 10 µm): (1) The distribution of P54nrb in the nucleus at 5 min was the same as the blank control, the nucleoli were clear, P54nrb was evenly distributed in the nucleus, LNA and CN-LNA-modified ASO did not change the distribution of P54nrb, and no aggregation was observed in the nucleolus. (2) For the LNA group, when the time was extended to 2 h and 4 h, respectively, there was significant P54nrb aggregation in the LNA group. The longer the incubation time, the more aggregation in the nucleolus, indicating that LNA-modified ASO had strong binding affinity to intracellular proteins, which was easily to interfere with the normal distribution and function of intracellular proteins, resulting in toxic side effects. (3) From the perspective of the R-CN-LNA group, there was no obvious aggregation of P54nrb protein in the nucleolus at 2 h after the R-CN-LNA modified sequence ON1, and the red fluorescence was evenly distributed in the nucleus. However, at 4 h, some cells showed obvious P54nrb aggregation, but it was obvious that the degree of aggregation was significantly lower than that of the LNA group at the corresponding time point. (4) Regardless of whether it was 2 h or 4 h, the S-CN-LNA group showed no visible change in P54nrb distribution at each time point, and P54nrb was still uniformly distributed in the cytoplasm.

**Table 1 Oligonucleotide sequences ON1 to ON4 and corresponding mass spectrometry data thereof**

| # | Oligonucleotides (5'-3') | Calculated MS | Found MS [M-H] | Modification structure | Remarks |
|---|---|---|---|---|---|
| ON1 | T^{R}T^{R}C^{R}AGTCATGACTT^{R}C^{R}C^{R} | 5407.2 | 5408.7 | R-CN-LNA | FIG. 1 |
| ON2 | T^{S}T^{S}C^{S}AGTCATGACTT^{S}C^{S}C^{S} | 5407.2 | 5408.8 | S-CN-LNA | FIG. 2 |
| ON3 | TTCAGTCATGACTTCC | 5257.2 | 5258.8 | LNA | FIG. 3 |
| ON4 | TTTTTTTTT^{S}T | 3033.0 | 3032.8 | S-CN-LNA | FIG. 6 |

On the basis of the current immunofluorescence assay, in order to further quantify the effects of LNA, R-CN-LNA, and S-CN-LNA modifications on the distribution of P54nrb within the nucleus, the percentage of cells with "significant aggregation" of P54nrb in the nucleolus to total cells in each group of LNA, R-CN-LNA and S-CN-LNA at 10 min, 30 min, 1 h, 2 h, and 4 h was statistically analyzed, respectively. As shown in Table 2, the number of cells in the LNA group in which P54nrb significantly aggregated in the nucleolus also increased as the transfection time increased. At 4 h after LNA transfection, it was found that approximately 92% of the transfected cells had significant aggregation of P54nrb in the nucleolus. In the R-CN-LNA group, only 5% of cells after ASO transfection had P54nrb significantly aggregated in the nucleolus at 2 h, and only about 8% at 4 h. For the S-CN-LNA group, there was no significant aggregation of P54nrb, with a cell proportion of 0%. The above results showed that compared with LNA modification, CN-LNA modification could indeed reduce the impact of ASO on intracellular proteins, and the modification in S configuration had less impact than that in R configuration.

**Table 2 Percentage of cells in which LNA-ASO, R-CN-LNA-ASO, and S-CN-LNA-ASO significantly aggregated P54nrb to the nucleolus**

| **Sequence modifier** | **Transfection time** | **Cell percentage (%)** |
|---|---|---|
| LNA | 10 min | 0 |
| | 30 min | 4 |
| | 1 h | 13 |
| | 2 h | 43 |
| | 4 h | 92 |
| R-CN-LNA | 10 min | 0 |
| | 30 min | 0 |
| | 1 h | 0 |
| | 2 h | 5 |
| | 4 h | 8 |
| S-CN-LNA | 10 min | 0 |
| | 30 min | 0 |
| | 1 h | 0 |
| | 2h | 0 |
| | 4h | 0 |

Based on the above evaluation of the impact on subcellular distribution of intracellular proteins, the effect of CN-LNA modification on the toxicity of induced apoptosis was further investigated by detecting Caspase 3/7 activity on HeLa cells.

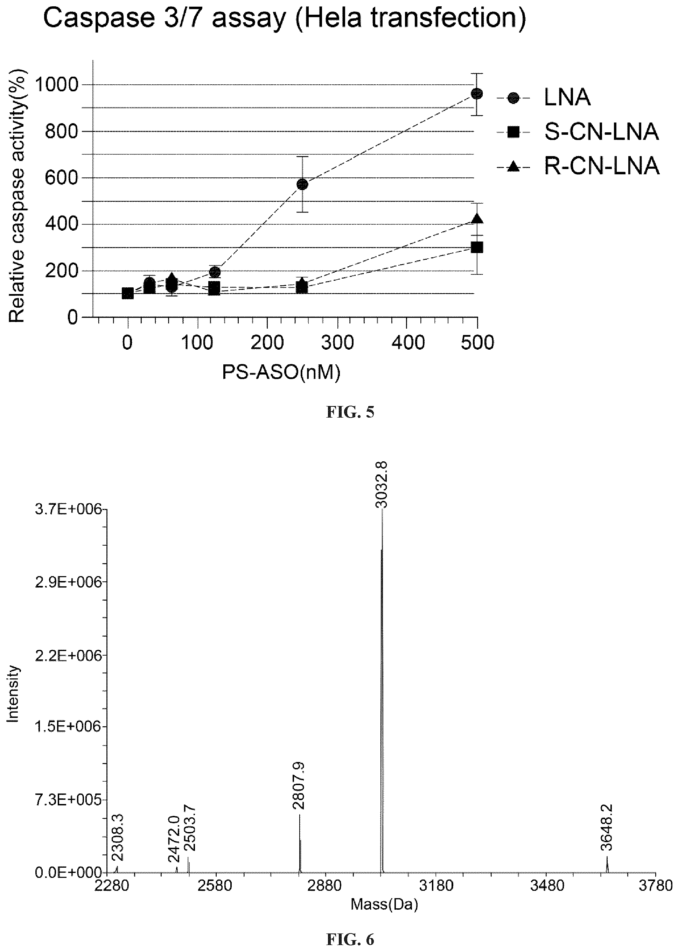

The cells were transfected with ON1, ON2, and ON3 at concentrations of 500 nM, 250 nM, 125 nM, 62.5 nM, 31.25 nM, and 0 nM for 4 h, respectively, incubated for 8 h, and the Caspase 3/7 activity was detected with a fluorescent microplate reader. As shown in FIG. 5, the experimental results showed that: (1) At low concentrations (0-62.5 nM), no obvious changes in caspase activity were seen in the three groups. (2) Starting from the concentration of 125 nM, the caspase activity level of the LNA modified group increased rapidly as the dosage concentration increased, while the two groups modified by CN-LNA only increased slowly starting from 250 nM, and the modified group in S configuration was lower than that in R configuration. (3) At high concentrations of 250 nM and 500 nM, compared to LNA modification, both R-CN-LNA and S-CN-LNA modifications could reduce the increase in caspase activity by not less than 2 times, and the modification inS configuration was better than that in R configuration. The above results showed that compared with LNA modification, R/S-CN-LNA modification could significantly reduce the apoptosis toxicity induced by PS ASO, which was consistent with the results of immunofluorescence assay.

In short, the introduction of CN groups into LNA could reduce the lipophilicity of LNA and improve the water solubility of the modified structure. CN-LNA modification could significantly reduce the effect of PS ASO on intracellular proteins, induce apoptosis toxicity, and improve the therapeutic effect of PS ASO. This exhibited a significant application value for nucleic acid drugs and could provide a new generation of chemical modification technology support for nucleic acid drugs.

### Experimental Example 2 Nuclease resistance test of S-CN-LNA-T

Stability to nucleases is one of the important parameters for chemical modification of nucleotides. In this experiment, snake venom phosphodiesterase (SVPDE) was used to investigate the resistance of S-CN-LNA-T-modified oligonucleotide (5'-TTTTTTTTTT-3' (SEQ ID NO: 2), T = S-6'-CN-LNA) ON4 on nuclease, and to compare with the sequences of R-CN-LNA (ON5), LNA (ON6), thio-modified (ON7), and native (ON8). The corresponding nucleic acid sequence (7 nM) was enzymatically digested using SVPDE (1.0 µg/mL) under physiological conditions of 37°C and a buffer system of 50 mM Tris-HCl, 10 mM MgCl₂, pH=8.0. The incubation solution was taken out at different time points (0, 2, 5, 10, 20, 30, 40 min) and quantified by HPLC to obtain the corresponding content-time curve. The nuclease resistance test results (referring to FIG. 7, T= S-CN-LNA (rectangle) modified ON4, R-CN-LNA (diamond) modified ON5, LNA (triangle) modified ON6, 3'-thio-T (Ts, cross) modified ON7, and natural T (Natural-T, star) modified ON8) showed that under the condition of 1.0 µg/mL SVPDE, the R-6'-CN-LNA modified sequence ON7 degraded slowly with more than 40% still not degraded after 40 min, more than 50% of the S-6'-CN-LNA modified sequence ON8 was not degraded, while less than 10% of the LNA modified sequence remained. This indicated that 6'-CN-LNA could greatly improve the nuclease resistance of oligonucleotides, which was significantly better than that of LNA, and its S configuration was better than that of R configuration.

Obviously, although the CN group is relatively small, it can still enhance the stability of LNA against nucleases, which is consistent with the results of other C6'-modified LNAs. It is worth noting that cEt-LNA in R and S configurations reported in the literature has similar nuclease resistance; but for CN-LNA, CN-LNA in S configuration, the S configuration has better nuclease resistant than the R configuration. Considering that the CN group on CN-LNA in S configuration faces the phosphate ester, this seems to indicate that CN shows a more complex effect on the phosphate ester. If so, this mechanism may have a greater impact on the interaction between CN-LNA-modified ASO and proteins, not just electrostatic interactions.

## Claims

1. A 6'-CN-modified locked nucleoside, wherein the 6'-CN-modified locked nucleoside is selected from the group consisting of a 6'-CN-modified locked nucleoside
in R configuration shown in 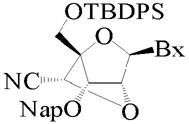 and a 6'-CN-modified locked nucleoside in S configuration shown in

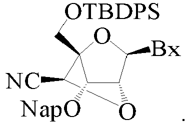

2. A synthesis method of the C6'-CN-modified nucleoside according to claim 1, wherein the 6'-(CN)-modified locked nucleoside is in R configuration or S configuration, and wherein the C6'-CN-modified nucleoside in R configuration is 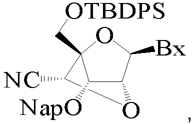 and a synthesis process thereof comprises: isomerizing a terminal olefin of 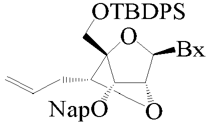 to obtain 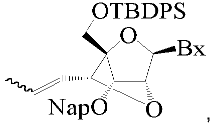 conducting dihydroxylation to obtain 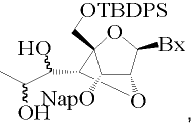 conducting oxidative cleavage to obtain an aldehyde compound 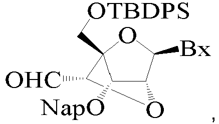 and then converting an aldehyde group of 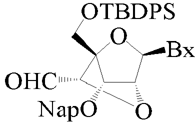 into a CN group to obtain the C6'-CN-modified nucleoside in R configuration shown in 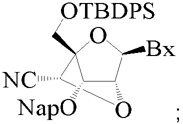 the C6'-CN-modified nucleoside in S configuration is 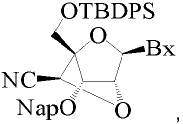 and a synthesis process thereof comprises: isomerizing a terminal olefin of 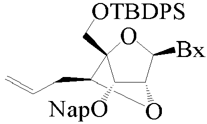 to obtain 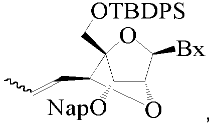 conducting dihydroxylation to obtain 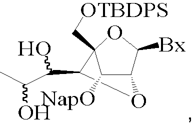 conducting oxidative cleavage to obtain an aldehyde compound 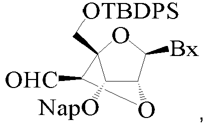 and then converting an aldehyde group of 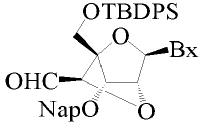 into a CN group to obtain the C6'-CN-modified nucleoside in S configuration shown in 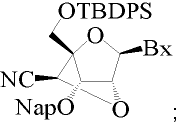 and
Bx is selected from the group consisting of substituted or unsubstituted adenine, guanine, thymine, cytosine, and uracil, and salts thereof.

3. The synthesis method according to claim 2, wherein a synthesis process of 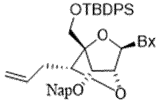 comprises: methylsulfonating 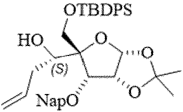 in S configuration, removing methylene of the methylsulfonated product, and acetylating to obtain 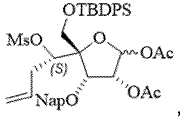 and then conducting glycosylation and nucleophilic substitution in sequence to obtain 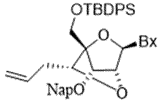 in R configuration;
a synthesis process of 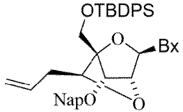 comprises: methylsulfonating 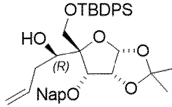 in R configuration, removing methylene of the methylsulfonated product, and acetylating in sequence to obtain 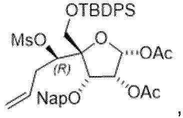 and then conducting glycosylation and nucleophilic substitution in sequence to obtain 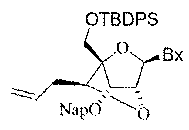 in S configuration; and
the glycosylation comprises: subjecting 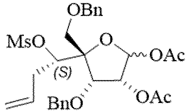 or 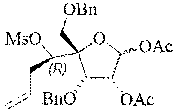 to a reaction with thymine, N6-benzoyladenine, or 6-chloroguanine at 50°C to 100°C in the presence of an activator; the activator comprises *N*,*O-*bis(trimethylsilyl)acetamide (BSA) and trimethylsilyl trifluoromethanesulfonate (TMSOTf), and a reaction medium is selected from the group consisting of acetonitrile, 1,2-dichloroethane, and toluene.

4. The synthesis method according to claim 3, wherein a synthesis process of the 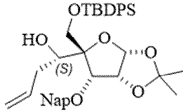 in S configuration comprises: oxidizing a primary alcohol group of 5-*O*-(tert-butyldiphenylsilyl)-4-C-hydroxymethyl-1,2-*O*-isopropylidene-3-*O*-(2-naphthylmethyl)-α-*D*-ribofuranose 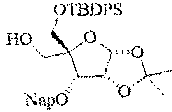 into an aldehyde group, and then allylating the aldehyde group to obtain 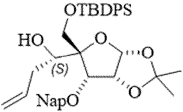 in S configuration; a synthesis process of 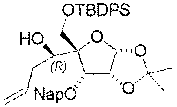 in R configuration comprises: oxidizing a secondary alcohol group of 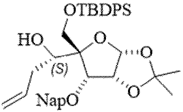 into a ketone group, and then reducing the ketone group into a secondary alcohol group to obtain 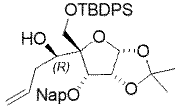 in R configuration; and
in the process of reducing the ketone group into the secondary alcohol group, a reducing agent used is at least one selected from the group consisting of lithium aluminum hydride, lithium borohydride, and a mixture of lithium chloride and sodium borohydride; a reaction medium is at least one selected from the group consisting of dichloromethane (DCM), tetrahydrofuran (THF), methanol, and ethanol, and the reducing is conducted at -78°C to 0°C.

5. The synthesis method according to claim 4, wherein a process of reducing the ketone group into the secondary alcohol group comprises: conducting the reducing at -40°C to 0°C using the mixture of the sodium borohydride and the lithium chloride as the reducing agent and using a mixture of the THF and the methanol as the reaction medium.

6. The synthesis method according to claim 2, wherein the catalyst for the isomerization of the terminal olefin is selected from the group consisting of a ruthenium catalyst, a palladium catalyst, a rhodium catalyst, and an iridium catalyst;
a reaction medium for the isomerization of the terminal olefin is selected from the group consisting of methanol, ethanol, n-butanol, and toluene;
the isomerization of the terminal olefin is conducted at 60°C to 100°C; and
the isomerization of the terminal olefin is conducted for 12 h to 72 h.

7. The synthesis method according to claim 2, wherein the catalyst for the isomerization of the terminal olefin is carbonylchlorohydridotris(triphenylphosphine)ruthenium (II); a reaction medium for the isomerization of the terminal olefin is ethanol; and the isomerization of the terminal olefin is conducted at 60°C to 80°C.

## Patentansprüche

1. Ein 6'-CN-modifiziertes geschlossenes Nukleosid, wobei das 6'-CN-modifizierte geschlossene Nukleosid ausgewählt ist aus der Gruppe bestehend aus einem 6'-CN-modifizierten geschlossenen Nukleosid in R-Konfiguration gezeigt in 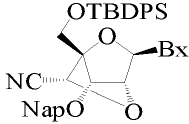 und einem 6'-CN-modifizierten geschlossenen Nukleosid in S-Konfiguration gezeigt in

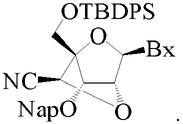

2. Ein Herstellungsverfahren des C6'-CN-modifizierten geschlossenen Nukleosids nach Anspruch 1,
wobei das 6'-(CN)-modifizierte geschlossene Nukleosid in R-Konfiguration oder in S-Konfiguration vorliegt,
und wobei das 6'-(CN)-modifizierte geschlossene Nukleosid in R-Konfiguration 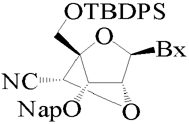 ist, und ein Herstellungsprozess davon umfasst: Isomerisieren eines terminalen Olefins gemäß 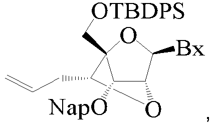 um 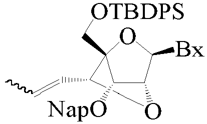 zu erhalten, Durchführen einer Dihydroxylierung, um 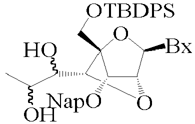 zu erhalten, Durchführen einer oxidativen Spaltung, um eine Aldehydverbindung gemäß 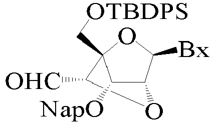 zu erhalten, und anschließend Umwandeln einer Aldehydgruppe gemäß 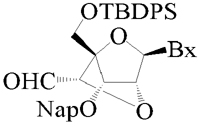 in eine CN-Gruppe, um das 6'-CN-modifizierte Nukleosid in R-Konfiguration gezeigt in 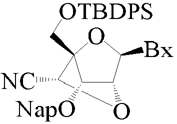 zu erhalten;
wobei das 6'-(CN)-modifizierte geschlossene Nukleosid in S-Konfiguration 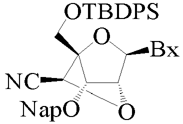 ist, und ein Herstellungsprozess davon umfasst: Isomerisieren eines terminalen Olefins gemäß 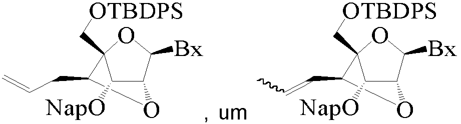 zu erhalten, Durchführen einer Dihydroxylierung, um 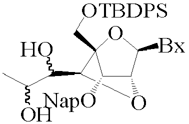 zu erhalten, Durchführen einer oxidativen Spaltung, um eine Aldehydverbindung gemäß 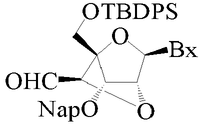 zu erhalten, und anschließend Umwandeln einer Aldehydgruppe gemäß 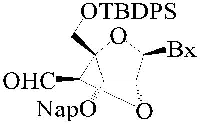 in eine CN-Gruppe, um das 6'-CN-modifizierte Nukleosid in S-Konfiguration gezeigt in 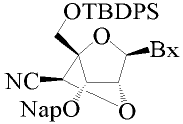 zu erhalten;
und wobei Bx ausgewählt ist aus der Gruppe bestehend aus substituiertem oder nicht-substituiertem Adenin, Guanin, Thymin, Cytosin, und Urazil, und Salzen davon.

3. Das Herstellungsverfahren nach Anspruch 2, wobei ein Herstellungsprozess von 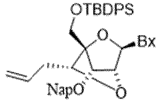 umfasst: Methylsulfonieren von 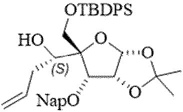 in S-Konfiguration, Entfernen von Methylen von dem methylsulfonierten Produkt und Azetylieren, um 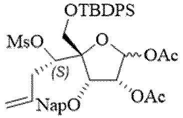 zu erhalten, und anschließend nacheinander Durchführen einer Glykosylierung und nukleophilen Substitution, um 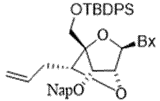 in R-Konfiguration zu erhalten; wobei ein Herstellungsprozess von 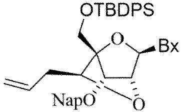 umfasst: Methylsulfonieren von 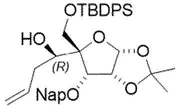 in R-Konfiguration; nacheinander entfernen von Methylen von dem methylsulfonierten Produkt und Azetylieren, um 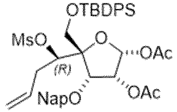 zu erhalten, und anschließend nacheinander Durchführen einer Glykosylierung und nukleophilen Substitution, um 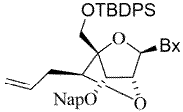 in S-Konfiguration zu erhalten; und wobei die Glykosylierung umfasst: Unterwerfen von 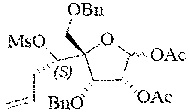 oder 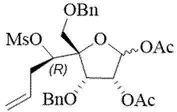 einer Reaktion mit Thymin, N6-Benzoyladenin, oder 6-Chloroguanin bei 50°C bis 100°C in Anwesenheit eines Aktivators; wobei der Aktivator *N*,*O-*bis(trimethylsilyl)Acetamid (BSA) und Trimethylsilyl-Trifluoromethansulfonat (TMSOTf) umfasst, und wobei ein Reaktionsmedium ausgewählt ist aus der Gruppe bestehend aus Azetonitril, 1,2-Dichlorethan und Toluol.

4. Das Herstellungsverfahren nach Anspruch 3, wobei ein Herstellungsprozess von 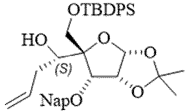 in S-Konfiguration umfasst: Oxidieren einer primären Alkoholgruppe von 5-*O*-(tert-butyldiphenylsilyl)-4-C-hydroxymethyl-1,2-*O*-isopropyliden-3-*O*-(2-naphthylmethyl)-α-*D*-ribofuranose 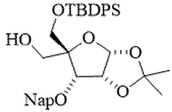 in eine Aldehydgruppe und anschließend Allylieren der Aldehydgruppe, um 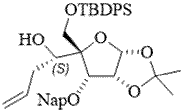 in S-Konfiguration zu erhalten; wobei ein Herstellungsprozess von 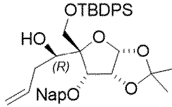 in R-Konfiguration umfasst: Oxidieren einer sekundären Alkoholgruppe von 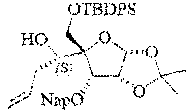 in eine Ketongruppe und anschließend Reduzieren der Ketongruppe in eine sekundäre Alkoholgruppe, um 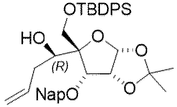 in R-Konfiguration zu erhalten;
und wobei in dem Prozess des Reduzierens der Ketongruppe zu einer sekundären Alkoholgruppe mindestens ein verwendetes Reduktionsmittel ausgewählt ist aus der Gruppe bestehend aus Lithium-Aluminium-Hydrid, Lithium-Borhydrid, und einer Mischung aus Lithium-Chlorid und Natrium-Borhydrid;
wobei ein Reaktionsmedium mindestens eines ausgewählt aus der Gruppe bestehend aus Dichlormethan (DCM), Tetrahydrofuran (THF), Methanol, und Ethanol ist,
und wobei die Reduktion bei -78°C bis 0°C durchgeführt wird.

5. Das Herstellungsverfahren nach Anspruch 4, wobei ein Prozess des Reduzierens der Ketongruppe zu einer sekundären Alkoholgruppe umfasst: Durchführen der Reduktion bei -40°C bis 0°C, wobei die Mischung aus Natrium-Borhydrid und Lithium-Chlorid als Reduktionsmittel verwendet wird und wobei eine Mischung aus THF und Methanol als Reaktionsmedium verwendet wird.

6. Das Herstellungsverfahren nach Anspruch 2, wobei der Katalysator für die Isomerisierung des terminalen Olefins ausgewählt ist aus der Gruppe bestehend aus einem Ruthenium-Katalysator, einem Palladium-Katalysator, einem Rhodium-Katalysator und einem Iridium-Katalysator;
wobei ein Reaktionsmedium für die Isomerisierung des terminalen Olefins ausgewählt ist aus der Gruppe bestehend aus Methanol, Ethanol, n-Butanol, und Toluol;
wobei die Isomerisierung des terminalen Olefins bei 60°C bis 100°C durchgeführt wird; und
wobei die Isomerisierung des terminalen Olefins für 12 h bis 72 h durchgeführt wird.

7. Das Herstellungsverfahren nach Anspruch 2, wobei der Katalysator für die Isomerisierung des terminalen Olefins Carbonylchlorohydridotris(triphenylphosphin)ruthenium (II) ist; wobei ein Reaktionsmedium für die Isomerisierung des terminalen Olefins Ethanol ist; und wobei die Isomerisierung des terminalen Olefins bei 60°C bis 80°C durchgeführt wird.

## Revendications

1. Nucléoside verrouillé à modification 6'-CN, le nucléoside verrouillé à modification 6'-CN étant choisi dans le groupe constitué par un nucléoside verrouillé à modification 6'-CN en configuration R représenté par 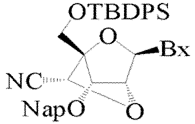 et un nucléoside verrouillé à modification 6'-CN en configuration S représenté par

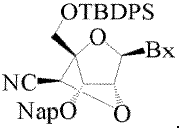

2. Procédé de synthèse du nucléoside à modification C6'-CN selon la revendication 1, le nucléoside verrouillé à modification 6'-(CN) étant en configuration R ou configuration S, et le nucléoside à modification C6'-CN en configuration R étant 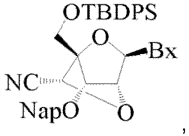 et un procédé de synthèse de ce dernier comprenant effectuer une isomérisation d'une oléfine terminale de 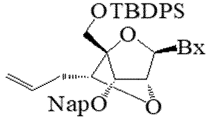 pour obtenir 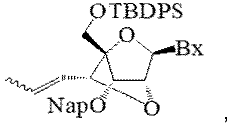 effectuer une dihydroxylation pour obtenir 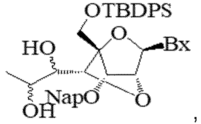 effectuer un clivage oxydatif pour obtenir un composé aldéhydique 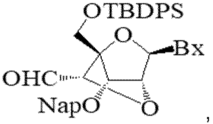 et ensuite convertir un groupe aldéhyde de 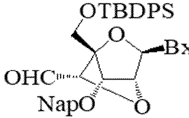 en un groupe CN pour obtenir le nucléoside à modification C6'-CN en configuration R représenté par 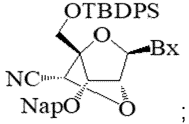 le nucléoside à modification C6'-CN en configuration S étant 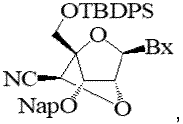 et un procédé de synthèse de ce dernier comprenant effectuer une isomérisation d'une oléfine terminale de 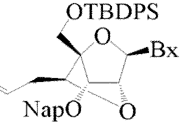 pour obtenir 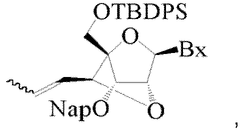 effectuer une dihydroxylation pour obtenir 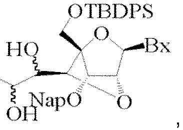 effectuer un clivage oxydatif pour obtenir un composé aldéhydique 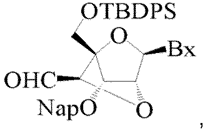 et ensuite convertir un groupe aldéhyde de 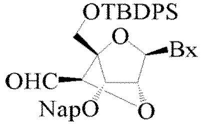 en un groupe CN pour obtenir le nucléoside à modification C6'-CN en configuration S représenté par 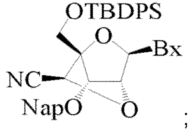 et
Bx étant choisi dans le groupe constitué par l'adénine, la guanine, la thymine, la cytosine et l'uracile, substitué(e)s ou non substitué(e)s, et leurs sels.

3. Procédé de synthèse selon la revendication 2, un procédé de synthèse de 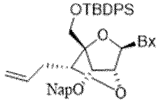 comprenant : soumettre à une méthylsulfonation 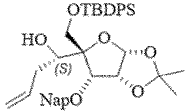 en configuration S, éliminer le méthylène du produit méthylsulfoné et effectuer une acétylation pour obtenir 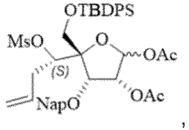 et ensuite effectuer une glycosylation et une substitution nucléophile, dans l'ordre, pour obtenir 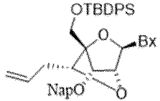 en configuration R ;
un procédé de synthèse de 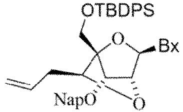 comprenant : soumettre à une méthylsulfonation 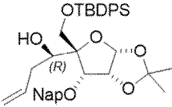 en configuration R, éliminer le méthylène du produit méthylsulfoné et effectuer une acétylation, dans l'ordre, pour obtenir 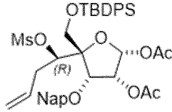 et ensuite effectuer une glycosylation et une substitution nucléophile, dans l'ordre, pour obtenir 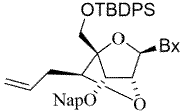 en configuration S ; et la glycosylation comprenant : soumettre 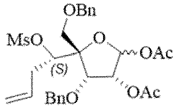 ou 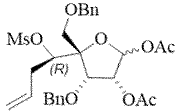 à une réaction avec la thymine, la N6-benzoyladénine, ou la 6-chloroguanine à 50 °C à 100 °C en présence d'un activateur ; l'activateur comprenant le *N,O*-bis(triméthylsilyl)acétamide (BSA) et le trifluorométhanesulfonate de triméthylsilyle (TMSOTf), et un milieu réactionnel étant choisi dans le groupe constitué par l'acétonitrile, le 1,2-dichloroéthane, et le toluène.

4. Procédé de synthèse selon la revendication 3, un procédé de synthèse de 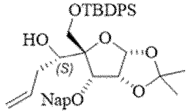 en configuration S comprenant : effectuer l'oxydation d'un groupe alcool primaire de*5*-*O*-(tert-butyldiphénylsilyl)-4-C-hydroxyméthyl-1,2-O-isopropylidène-3-O-(2-naphtylméthyl)-α-D-ribofuranose 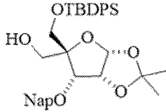 en un groupe aldéhyde, et ensuite effectuer une allylation du groupe aldéhyde pour obtenir 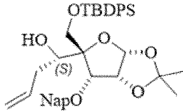 en configuration S ;
un procédé de synthèse de 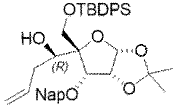 en configuration R comprenant : effectuer l'oxydation d'un groupe alcool secondaire de 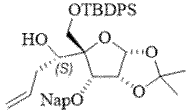 en un groupe cétone, et ensuite réduire le groupe cétone en un groupe alcool secondaire pour obtenir 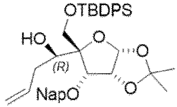 en configuration R ; et
dans le processus de réduction du groupe cétone en le groupe alcool secondaire, un agent réducteur utilisé étant au moins un choisi dans le groupe constitué par l'hydrure de lithium et d'aluminium, le borohydrure de lithium, et un mélange de chlorure de lithium et de borohydrure de sodium ; un milieu réactionnel étant au moins un choisi dans le groupe constitué par le dichlorométhane (DCM), le tétrahydrofurane (THF), le méthanol et l'éthanol, et la réduction étant effectuée à une température de -78 °C à 0 °C.

5. Procédé de synthèse selon la revendication 4, un processus de réduction du groupe cétone en le groupe alcool secondaire comprenant : effectuer la réduction à une température de -40°C à 0°C en utilisant le mélange de borohydrure de sodium et de chlorure de lithium en tant que l'agent réducteur et en utilisant un mélange du THF et du méthanol en tant que le milieu de réaction.

6. Procédé de synthèse selon la revendication 2, le catalyseur pour l'isomérisation de l'oléfine terminale étant choisi dans le groupe constitué par un catalyseur au ruthénium, un catalyseur au palladium, un catalyseur au rhodium et un catalyseur à l'iridium ;
un milieu réactionnel pour l'isomérisation de l'oléfine terminale étant choisi dans le groupe constitué par le méthanol, l'éthanol, le n-butanol et le toluène ;
l'isomérisation de l'oléfine terminale étant effectuée à une température de 60 °C à 100 °C ; et
l'isomérisation de l'oléfine terminale étant effectuée pendant 12 h à 72 h.

7. Procédé de synthèse selon la revendication 2, le catalyseur pour l'isomérisation de l'oléfine terminale étant le carbonylchlorohydridotris-(triphénylphosphine)ruthénium (II) ; le milieu réactionnel pour l'isomérisation de l'oléfine terminale étant l'éthanol ; et l'isomérisation de l'oléfine terminale étant effectuée à une température de 60 °C à 80 °C.
